(19) Europäisches Patentamt
European Patent Office
Office européen des brevets

(11) EP 3 040 890 B1

(12) **EUROPEAN PATENT SPECIFICATION**

(45) Date of publication and mention of the grant of the patent:
**28.07.2021 Bulletin 2021/30**

(51) Int Cl.:
***G16C 60/00*** *(2019.01)* *G16C 20/30* *(2019.01)*
*G16C 10/00* *(2019.01)*

(21) Application number: **14461609.1**

(22) Date of filing: **31.12.2014**

# (54) System for finding materials having defined properties and computer-implemented method for finding materials having defined properties

System zum Auffinden von Materialien mit definierten Eigenschaften und computerimplementiertes Verfahren zum Auffinden von Materialien mit definierten Eigenschaften

Système pour trouver des matériaux ayant des propriétés définies et procédé mis en oeuvre par ordinateur pour trouver des matériaux ayant des propriétés définies

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.07.2016 Bulletin 2016/27**

(73) Proprietor: **IGLOO Spolka z ograniczona odpowiedzialnoscia**
**32-720 Nowy Wisnicz (PL)**

(72) Inventor: **Michalski, Rafal**
**31-462 Krakow (PL)**

(74) Representative: **Hudy, Ludwik**
**Kancelaria Patentowa Patelha**
**Czernichow 4**
**32-070 Czernichow, Krakow (PL)**

(56) References cited:

- **VON RANKE P. J. ET AL: "Influence of the crystalline electrical field on the magnetocaloric effect of DyAl 2 , ErAl 2 , and DyNi 2", PHYSICAL REVIEW B, vol. 58, no. 18, 1 November 1998 (1998-11-01), pages 12110-12116, XP055198729, DOI: http://dx.doi.org/10.1103/PhysRevB.58.1211 0**
- **ABADÍA C. ET AL: "Study of the crystal electric field interaction in RFe 11 Ti single crystals", J. PHYS.: CONDENS. MATTER, vol. 10, no. 2, 19 January 1998 (1998-01-19), pages 349-361, XP055199131,**
- **WANG Y. J. ET AL: "Perpendicular anisotropy in magnetic multilayer films", JOURNAL OF MAGNETISM AND MAGNETIC MATERIALS, ELSEVIER SCIENCE PUBLISHERS, AMSTERDAM, NL, vol. 115, no. 1, 1 September 1992 (1992-09-01), pages 9-19, XP024463406, ISSN: 0304-8853, DOI: 10.1016/0304-8853(92)90179-R [retrieved on 1992-09-01]**
- **HALL G. G. ET AL: "Point charge models for molecular properties", CHEMICAL PHYSICS LETTERS, ELSEVIER BV, NL, vol. 20, no. 6, 15 July 1973 (1973-07-15), pages 501-503, XP026491585, ISSN: 0009-2614, DOI: 10.1016/0009-2614(73)80484-1 [retrieved on 1973-07-15]**
- **None**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to a system for finding materials having defined properties and a computer implemented method for finding materials having defined properties, particularly materials containing elements having one ion with an unclosed electron shell.

The search for materials with defined properties is a long and expensive process, even in the era of advanced material technology and with modern research techniques, using devices controlled by computers with high computing power. Currently, the properties of materials, such as mechanical, thermal or magnetic properties, are defined analysing samples made of a defined material. When it turns out, during the analysis, that the material has properties fulfilling the criteria defined by the designers, the step of finding materials with properties defined in the project ends. Examples of projects requiring materials with given properties may include a suspension bridge, a vehicle or even a semiconductor.

From publication No. CA2863843 A1 of the Patent Application titled "Apparatus and method for measuring properties of ferromagnetic material", a device for measuring properties of an object made of ferromagnetic material is known.

From publication No. WO2014134655 A1 of the Patent Application titled "Estimating material properties", an update of data on the concentration of iron in a bulk mine sample is known. The definition of properties of material is based on values of one or more parameters of the sample. After obtaining measurements concerning properties of a material, the processor of the processing unit decides on an update of values concerning the properties of the material, based on the measurements.

From publication No. WO2014150916 A1 of the Patent Application entitled "Systems and methods for improving direct numerical simulation of material properties from rock samples and determining uncertainty in the material properties", a method for determination of Representative Elementary Volume based on one or more properties of material is known. The method consists in defining multiple test volumes, determining the value of the difference between neighbouring pairs of sample volumes and adopting the Representative Elementary Volume based on the value of the difference for every multiplicity of the tested volumes.

From publication of P.J. von Ranke et al. titled "Influence of the crystalline electrical field on the magnetocaloric effect of DyAl2, ErAl2 and DyNi2", PHYSICAL REVIEW B, vol. 58, no. 18, pages 12110-12116, 1998, are known calculations of magnetic entropy change $\Delta S_{mag}$ and the magnetocaloric effect $\Delta T_{ad}$ in $DyAl_2$, $ErAl_2$ and $DyNi_2$ using a Hamiltonian that takes into account the effects of crystalline electrical field.

In turn, from publication of C. Abadia et al. titled "Study of the crystal electric field interaction in RFe11Ti single crystals", J.PHYS.: CONDENS. MATTER, vol. 10, no. 2, pages 349-361, 1998, are known magnetization measurements along the main symmetry directions of the tetragonal structure that have been performed on $RFe_{11}Ti$ single crystals at applying high magnetic fields up to 12 Tesla and temperatures ranging from 4.2 to 300 K.

Furthermore, from publication of Y.J. Wang et al. titled "Perpendicular anisotropy in magnetic multilayer films", Journal of Magnetism and Magnetic Materials, vol. 115, no. 1, pages 9-19, 1992, is known a single-ion model approximated by point charge interaction.

From publication of G.G. Hall et al. titled "Point charge models for molecular properties", CHEMICAL PHYSICS LETTERS, vol. 20, no. 6, pages 501-503, 1973, is known a point charge model for the electron density of a molecule that preserves the correct total charge and dipole moment.

The aim of the present invention is to create a system for finding materials having defined properties and to create a computer implemented method for finding materials having defined properties, based particularly on the knowledge of Crystal Electric Field (CEF).

In the case of the system, the aim was achieved by features of claim 14, while in the case of the method according to the invention, the aim was achieved by features of claim 1.

According to the invention, a method for finding materials having defined properties, during which a material containing ions of at least one Mendeléev Table component element with unclosed electron shells is selected based on information available in the state of art, the method comprising the steps defining at least one kind of ions being the basis for calculations and material simulations by choosing at least one component element of a chosen material directly from the Mendeléev Table,

determining an oxidation state of atoms of the component element being selected in the chosen material define their electron configuration,

for selected at least one kind of ions of the component element, after determining values of quantum numbers of an orbital magnetic moment L, answering a question whether Crystal Electric Field (CEF) coefficients are known and, in case when Crystal Electric Field (CEF) coefficients are known, inputting Crystal Electric Field (CEF) coefficients defined by Stevens coefficients having a form of $B^m_n$ or, in case when Crystal Electric Field (CEF) coefficients are not known, after selecting a method for finding values of Crystal Electric Field (CEF) coefficients, carrying out calculations of Stevens coefficients with the form $B^m_n$ and inputting Crystal Electric Field (CEF) coefficients defined by Stevens coefficients having a form of $B^m_n$,

starting calculations with predefinated parameters or, after selecting calculation method, carrying out calculations of a spin and orbital magnetic moment L, S when an $|L,S,L_z,S_z>$ basis is chosen according to chosen calculation space or a total magnetic moment J corresponding to a ground state of the selected ions when an $|L,S,J,J_z>$ basis is chosen for finding a complete set of Crystal Electric Field (CEF) coefficients, defined by Stevens coefficients having a form of $B^m{}_n$, expressed in energy units and defining an immediate charge environment of the selected ions in a crystal lattice by calculation of Stevens coefficients having the form of $B^m{}_n$,
based on the Stevens coefficients having the form of $B^m{}_n$, generating a total energy operator, called a Hamiltonian matrix containing matrix components being elements of Stevens operators multiplied by the defined Stevens coefficients $B^m{}_n$ ($H_{CEF}=\Sigma B^m{}_n O^m{}_n$), (x, y, z) or (x, z) components of operators of magnetic field potential, projection operators of orbital magnetic moment, spin magnetic moment and total magnetic moment, and components of operators of the spin-orbit coupling,
after carrying out operations on the matrix, creating a model of an ideal material containing the selected ions, the selected ions being spatially oriented identically and not interacting with each other, but interacting with external magnetic and electric fields, with a calculated structure of energy states together with their spectral properties, and being subjected to classical Boltzmann statistics, and having the directional (x, y, z) or (x, z) components of magnetic properties calculated,
based on the model of the ideal material, defining calorimetric, electron and magnetic properties in a form of temperature dependencies of material containing ions in the defined environment of the Crystal Electric Field (CEF), and
verifying the properties of the ideal material with properties of a real material, whether the properties of the material obtained from calculations correspond to the properties of the material being searched for.

The values of quantum numbers of orbital magnetic moment L, spin magnetic moment S and optionally total magnetic moment J corresponding to the ground state of electron configuration of a selected ion may be determined based on Hund's rules.

Calculation of Stevens coefficients having the form of $B^m{}_n$ may be carried out after choosing one of the calculation methods and defining the computation space, selecting a basis for calculations and determining the values of constants.

In a preferred embodiment of the invention, calculations of the Stevens coefficients having the form of $B^m{}_n$ are carried out using Point Charge Model Approximation (PCM) or using an interactive three-dimensional (3D) visualisation of component multi-poles of the electric field and their superpositions defined as crystal field (CEF) or by a conversion of crystal field (CEF) coefficients ($A^m{}_n$ -> $B^m{}_n$) from known results of other calculations for systems isostructural with the one being calculated, but containing other ions.

The results of calculations of the Stevens coefficients having the of form $B^m{}_n$ may be harmonised by comparing the obtained results using Point Charge Model Approximation (PCM) or an interactive visualisation of the crystal field (CEF) in 3D, or by a conversion of crystal field (CEF) coefficients ($A^m{}_n$ -> $B^m{}_n$) from results of other calculations for systems isostructural with the one being calculated, but containing other ions.

All operations leading to the calculation of the structure of states of the selected ions in the defined environment in the crystal lattice may be carried out after choosing the computation space from a vector space spanned across a body of real numbers and space spanned across a body of complex numbers and choosing a basis for construction of the Hamiltonian matrix, or the total energy operator.

It may be anticipated that after choosing the space of real numbers and carrying out the calculations in the $|L,S,J,J_Z>$ basis, while generating the matrix containing the products of the matrix elements of Stevens operators and defined Stevens coefficients ($B^m{}_n$, $O^m{}_n$), and operators of directional components (x, z) of the magnetic field, at first, an empty matrix is created with rows and columns numbered with values of $|J_z>$, the matrix being filled with products of the matrix elements of Stevens operators and defined Stevens coefficients ($B^m{}_n$, $O^m{}_n$), and component operators (x, z) of the magnetic field, and after choosing the space of real numbers and carrying out the calculations with the $|L, S, L_z, S_z>$ basis, while generating the matrix containing the products of the matrix elements of Stevens operators and defined Stevens coefficients ($B^m{}_n$, $O^m{}_n$), and component operators (x, z) of the magnetic field, at first, an empty matrix is created with rows and columns numbered with $|L_z, S_z>$ combinations, which, as an initially prepared Hamiltonian matrix, is filled with components of Stevens operators ($B^m{}_n$, $O^m{}_n$), component operators (x, z) of the magnetic field and components of the spin-orbit coupling operator.

Alternatively, after choosing the space of real numbers and complex numbers, and carrying out the calculations in the $|L,S,J,J_Z>$ basis, while generating the matrix containing fillings with the products of the matrix elements of Stevens operators and defined Stevens coefficients ($B^m{}_n$, $O^m{}_n$), and component operators (x, y, z) of the magnetic field, at first, an empty matrix is created with rows and columns numbered with values of $|J_z>$, the matrix being filled with products of the matrix elements of Stevens operators and defined Stevens coefficients ($B^m{}_n$, $O^m{}_n$), total moment projection operators and component operators (x, y, z) of the magnetic field, and after choosing the space of complex numbers and carrying out the calculations with the $|L, S, L_z, S_z>$ basis, while generating the matrix containing the products of the matrix elements of Stevens operators and defined Stevens coefficients ($B^m{}_n$, $O^m{}_n$), and component operators (x, y, z) of the magnetic field, at first, an empty matrix is created with rows and columns numbered with $|L_z, S_z, >$ combinations, which, as a Hamiltonian matrix, is filled with components of Stevens operators ($B^m{}_n$, $O^m{}_n$), projection operators of total spin and orbital magnetic moments, component operators (x, y, z) of the magnetic field and components of the spin-orbit coupling operator.

Preferably, after filling with products of the matrix elements of Stevens operators and defined Stevens coefficients ($B^m{}_n$, $O^m{}_n$), projection operators of total spin and orbital magnetic moments, component operators (x, z) or (x, y, z) of magnetic the field and optionally with components of the spin-orbit coupling operator, diagonalisation of the Hamiltonian matrix is carried out, and after the diagonalisation of the Hamiltonian matrix, n-complete sets of pairs of energy eigenvalues E' (i=1..n) and eigenfunctions being linear combinations of basis vectors are calculated, and next, based on their form, the expected values of the directional components (x,z) or (x,y,z) of magnetic moments of the individual n-eigenstates of energy E' are calculated.

N-eigenstates of energy E' may be sorted with their expected values of directional components of magnetic moments $<m^i{}_j>$ (i=1..n, j=x,z or j=x,y,z) of the individual states, and next, the sum of states Z(T) and population $N^i(T)$ of every energy state of the obtained structure are calculated in defined temperature increments according to Boltzmann statistics, based on which courses of temperature dependencies of free energy, internal energy, entropy, magnetic susceptibility, calculated for a field applied along x and z or x, y and z directions, and Schottky specific heat are calculated in order to determine the calorimetric, electron and magnetic properties of a material containing ions in the defined environment of the Crystal Electric Field (CEF).

Preferably, a new complete set of result data is created, containing the calorimetric, electron and magnetic properties of a material containing ions in the defined environment of the Crystal Electric Field (CEF) together with an interactive visualisation of this environment and calculation parameters, and the new complete set of result data is presented in the form of an independent complete set of data available directly and in parallel with other result data, enabling direct comparisons of the obtained results.

Various separate complete sets of the result data may be archived in a single merged numerical form together with the data pertaining to calculations, simulations and visualisations of every separate complete set of the result data, and the numerical form of the result data enables access to a chosen property or a course of the temperature dependency of the chosen property from different complete sets of the result data simultaneously.

Preferably, the form of the result data enables implementation of the saved result data and comparison with adequate current calculations.

According to another idea of the invention, in a system for finding materials having defined properties that has a computing unit with a processor, a device for presentation of data and calculation results and with access to data on materials, and a testing unit adapted to carry tests on real materials and communicating with the computing unit, the processor comprises

a module for finding and selecting elements of the chosen material, enabling determination of their electron configuration based on values of quantum numbers of orbital magnetic moment L, spin magnetic moment S when an $|L,S,L_z,S_z>$ basis is chosen or based on values of total magnetic moment J when an $|L,S,J,J_z>$ basis is chosen,

a module for finding a complete set of Crystal Electric Field (CEF) coefficients, defined by Stevens coefficients with the form of $B^m{}_n$,

a module for preparation of data for the calculations with a module for selection of computational technique and a module for determination of input data, space and basis of the calculations, the module communicating with the module,

a module for construction of a model of an ideal material containing defined ions and adapted to communicating with the module for finding a complete set of Crystal Electric Field (CEF) coefficients, the ions being spatially oriented identically and not interacting with each other, but interacting with external fields, with a calculated structure of energy states together with their spectral properties, and being subjected to classical Boltzmann statistics, and having directional (x, y, z) or (x, z) components of magnetic properties calculated, the module for construction of a model of the ideal material being connected with the testing unit in order to verify the model of the ideal material with a real material,

a module for comparison of the ideal material with the real material in order to verify the model of the ideal material with the real material, when the properties of the material obtained from calculations correspond to the properties of the material being searched for.

The module for construction of the model of the ideal material may contain a module for calculation of complete sets of pairs of the energy eigenvalues E' (i=1..n) and eigenfunctions being linear combinations of basis vectors, and a module for calculation of courses of temperature dependencies of free energy, internal energy, entropy, magnetic susceptibility, calculated for a field applied along x and z or x, y and z directions, and Schottky specific heat in order to determine the calorimetric, electron and magnetic properties of a material containing ions in the defined environment of the Crystal Electric Field (CEF).

The subject of the invention will be illustrated as an embodiment in the drawing, where Fig. 1 shows a system for finding materials having defined properties and a method for finding materials having defined properties, Fig. 2 shows a block diagram of a computing unit, Fig. 3 shows a block diagram of a model of an ideal material, Figs. 4A - 4D show a block diagram of a method for searching for materials having defined properties, Figs. 5A - 5E show a block diagram of calculations of energy eigenvalues together with the wave functions corresponding to them, Fig. 6 shows a three-dimensional visualisation of CEF for praseodymium ions in a $PrCl_3$ crystal, Fig. 7 shows a matrix for praseodymium ions in a $PrCl_3$ crystal, Fig. 8 shows a diagram of energy states, Fig. 9 shows entropy vs. temperature, Fig. 10 shows the temperature dependency of the specific heat component originating from praseodymium ions, Fig. 11 shows a visualisation of magnetic susceptibility in an inverse form, Fig. 12 shows a fragment of the Hamiltonian matrix, constructed in the space spanned across a body of complex numbers, Fig. 13 shows a structure of states with the ground state and a group of states marked, Fig. 14 shows a visualisation of the course of magnetic susceptibility vs. temperature, for all spatial components, Fig. 15 shows a three-dimensional visualisation of CEF for nickel ions in nickel(II) oxide NiO, Fig. 16 shows a matrix of nickel ions in nickel(II) oxide NiO, Fig. 17 shows a diagram of energy states with the ground state and a group of next states marked, Fig. 18 shows a diagram of magnetic susceptibility measured for a field applied in parallel to the x and z axes, Fig. 19 shows a diagram of the splitting of a ground triplet into singlet components, Fig. 20 shows a diagram of the specific heat c(T) course, entropy course, and Fig. 21 shows a diagram of magnetic susceptibility for all spatial components.

A system 1 for finding materials having defined properties, shown schematically in Fig. 1, contains a computing unit able to create a model of an ideal material, which has properties, if not the same, then close to those of the material being searched for. Before construction of the model of the ideal material, a material is chosen, hereinafter called the chosen material, containing atoms of elements with given properties. Most often, the properties of such chosen material are not known, but based on the state of knowledge available via a server 11 in a base 13 of the state of art, via Internet 12 or in a base 14 of the state of art in paper form, it may be expected that the chosen material will have properties at least close to the properties of the material being searched for. A computing unit 10 has a possibility to communicate with the mentioned bases 13, 14. Moreover, the computing unit has a possibility to communicate with a station 15, in which tests of a tested material 16 imitating the model of the ideal material are carried out.

Fig. 2 schematically shows the computing unit 10, containing a processor 20 and other subassemblies 90 such as ROM 92, RAM 91, mass storage 94, a display module and a display monitor 95 and a user interface 93 enabling communication with the system. The computing unit contains a module 30 for selection of the element and its oxidation state, a module 40 for finding values of coefficients in the form $B^m_n$ with a module 41 of the Point Charge Model (PCM), a module 43 for interactive visualisation of CEF and a module 42 for conversion of CEF coefficients. Moreover, the processor contains a module 50 for preparation of data for the calculations with a module 51 for selection of computational technique and a module 52 for determination of input data, space and basis of the calculations, as well as a module 60 for construction of a model of the ideal material, a module 70 for visualisation and archiving and a module 80 for comparison of the ideal material with a real material.

Next, Fig. 3 schematically shows the module 60 for construction of the model of the ideal material of the computing unit 10. The module 60 contains a module 61 for collection of the input data and calculation parameters, a module 62 for selection of the calculation space, a module 63 for construction of the matrix and its transformation, a module 64 for solving of eigenproblems, a module 65 for calculation of the expected values of magnetic moments and their verification, a module 66 for statistical calculations, a module 67 for calculation of directional components and a module 68 for calculation of courses of temperature dependencies and their interpretation.

As results from a block diagram of a computer implemented method for searching for materials having defined properties shown in Fig. 4A - 4D, after a start in step 100, an ion being the basis for calculations and material simulations is defined by choosing an element directly from the Mendeléev Table or the Periodic Table in step 200. The individual steps define the subject of finding material so as to realise the possibilities of the given naturally occurring material by default and according to the current state of art, as well as to enable studies in areas unknown or inconsistent with the canon. The properties of the defined material, in case of ionic complexes or coordination compounds, will eventually be determined by the unclosed electron shell; thus, within the framework of the adopted technique, the oxidation state of atoms of the chosen element is determined in step 300. According to the current valid state of knowledge, it unequivocally defines the electron configuration being the basis for calculations. The determined electron configuration, defined based on rules of quantum mechanics and the chemistry of atoms, automatically determines the values of total spin quantum numbers: L, defining the orbital moment, S, defining the spin moment, and optionally J, defining the total moment, the quantum numbers corresponding to the ground state on Hund's rules, from which the first Hund's rule states that the ground state of a multiplet structure has a maximum value of S allowed by the Pauli exclusion principle, the second Hund's rule states that the ground state has a maximum allowed L value, with maximum S, and the third Hund's rule states that the primary multiplet has a corresponding $J=|L-S|$ when the shell is less than half full, and $J=L+S$, where the fill is greater.

The values of quantum numbers L, S and optionally J, determined based on the above rules, construct a space for calculations constituting the essence of finding material. In step 400, the fact whether the ion has an unclosed electron shell is checked, and the procedure is continued, depending on the fact whether the so-defined ion has an unclosed electron shell or not. After choosing the ion, its immediate charge environment in the crystal lattice is determined.

The procedure of finding material is continued by checking in step 500 whether the crystal field coefficients $B^m_n$ are known, and if they are known, the coefficients $B^m_n$ are introduced in step 560. This means that knowledge of a complete set of the crystal field coefficients developed according to the adopted convention allows for them to be passed on directly to the calculations.

The charge environment is parameterised within the framework of the procedure principles by its expansion into a series of multipoles with various spatial configurations, in which a first multipole is a dipole, the next multipole is a quadrupole and so on, and in which the weight of the contribution of the determined electrical multipoles is defined by $A^m_n$ coefficients, and the form of functions determining the spatial distribution of the potential of the individual, more significant multipoles in Cartesian coordinates is presented by the following formulas:

$$V_2^0 = 3z^2 - r^2$$

$$V_2^2 = x^2 - y^2$$

$$V_4^0 = 35z^4 - 30r^2z^2 + 3r^4$$

$$V_4^2 = (7z^2 - r^2)(x^2 - y^2)$$

$$V_4^3 = z\ (x^3 - 3x\ y^2)$$

$$V_4^4 = (x^4 - 6x^2y^2 + y^4)$$

$$V_6^0 = 231z^6 - 315r^2z^4 + 105r^4z^2 + 5r^6$$

$$V_6^2 = [16z^6 - 16(x^2 + y^2)z^2 + (x^2 + y^2)^2](x^2 - y^2)$$

$$V_6^3 = (11z^3 - 3z\,r^2)(x^3 - 3x\ y^2)$$

$$V_6^4 = (11z^3 - r^2)(x^4 - 6x^2y^2 + y^4)$$

$$V_6^6 = x^6 - 15x^4y^2 + 15x^2y^4 - y^6$$

where: $r^2 = x^2 + y^2 + z^2$

According to the current state of knowledge, there are operators operating in quantum spaces with symmetry properties identical to the potentials defined above in the real space. These operators are Stevens operators $O^m_n$ operating in spaces defined by spin quantum numbers. Thus, a classical Hamiltonian of the crystal field for the potential of the series of multipoles may be defined:

$$H_{CEF} = \sum_{m,n} A_n^m\ V_n^m(x, y, z)$$

based on the current state of knowledge, an analogous CEF Hamiltonian is defined in the space of spin quantum numbers, with the form:

$$H_{CEF} = \sum\sum B_n^m\ \hat{\mathbf{O}}_n^m(L, L_z)$$

In the above analogous CEF Hamiltonian, the contribution of multipoles is defined by CEF coefficients, called Stevens coefficients with the form $B^m_n$. The method for conversion of coefficients $A^m_n$ -> $B^m_n$ between the classical and quantum spaces, based on the rules of quantum mechanics, by dynamically calculated Clebsch and Gordon coefficients based on defining expressions, constitutes the foundation of visualisation possibilities of CEFs within the framework of the optimisation being carried out and conversion of values of the coefficients between isostructural compounds with various ionic ligands after transition from step 580 to step 540.

In case the crystal field coefficients $B^m_n$ are not known, selection of the method for finding the values of crystal field coefficients $B^m_n$ is carried out in step 510. It should be noted that in the majority of cases, determination of Stevens coefficients together with their all related parameters is the biggest difficulty when using methods originating from atomic physics. Depending on the selection of the method in step 520, the calculations of Stevens coefficients with the form $B^m_n$ are carried out using the Point Charge Model (PCM); in step 530, the calculations are carried out using an interactive three-dimensional (3D) visualisation of the crystal field (CEF) components; and in step 540, the calculations are carried out by a conversion of crystal field (CEF) coefficients $A^m_n$ -> $B^m_n$ from known results of other calculations for systems isostructural with the one being calculated, but containing other ions. After completion of the calculations, in step 550, the values of Stevens coefficients are harmonised. The process is based on an analysis of the spread of the coefficient values obtained by various methods, then, on a verification of the result values in comparison with the known experimental data or theoretical relations connecting the values of parameters in case of specific symmetries of the crystal lattice by proportions. While determining the complete set of the crystal field coefficients corresponding to the defined spatial distribution of the electrical charge, it may be additionally interactively visualised and compared with the point charge model PCM in step 530. Also, in step 570, the values of coefficients may be compared and converted by extraction of values of universal parameters characterising the crystal lattice $A^m_n$ from the form $B^m_n$ of Stevens coefficients useful for the calculations, after transition to step 540.

Ultimately, input of the start data occurs in steps 600, while in steps 610, 620, 630, 640, 650, and 660, selection of the method is carried out. These steps allows for collecting of all the necessary data in order to correctly define the input data for the calculations. In a case where calculations with predefined calculations are chosen, the calculations will be carried out in a full $|L,S,L_z,S_z>$ basis, with the value of the spin-orbit coupling constant $\lambda_{s\text{-}o}$ assumed as a free ion, in the space of complex numbers which allows for obtaining of results with all three directional components in space. In a case where a vector space spanned across a body of real numbers and the $|L,S,J,J_z>$ basis are chosen, complete results are not obtained in step 640; however, considering the significantly faster calculations, they are useful in cyclic calculations, for instance in order to search for a proper complete set of CEF parameters in connection with the result properties.

After carrying out the calculations in step 700, a complete set of the result data is presented in step 800.

Figs. 5A - 5E show a block diagram of calculations of energy eigenvalues together with the wave functions corresponding to them. The first five steps 1100, 1200, 1300, 1400, 1500 illustrate the method for setting up the calculation procedure in response to the entered parameters concerning the technique of calculations. In this moment, the ion is already determined, and the values of magnetic quantum numbers L and S corresponding to the ground state according to the fundamentals of the atom physics are known, e.g. in case of $Cr^{3+}$ -> ion L=3, S=3/2. Depending on the adopted input setting, step 1410 or 1430 or 1510 or 1530 is carried out by generating an empty matrix with a dimension dependent on quantum number L of the total orbital magnetic moment of the whole unclosed shell, and quantum number S of the total spin moment of the whole unclosed shell, or on quantum number J of the total moment of the whole unclosed shell corresponding to a combination of basic values of L and S, if J is a good quantum number in the specific case. This name has no precise definition - it results from the practice of theoretical calculations, when it is sometimes possible to use it in a theoretical description (4f, 5f configurations), when the assumption that the states of the ground multiplet are so distant from the states of the first excited multiplet that their influence may be omitted, is correct. Sometimes, unfortunately, it is impossible (3d, 4d configuration), when the states interpenetrate, and number J is unjustified - and the spin-orbital coupling should be included into the calculations with a finite value of the coupling constant, obtaining a structure of states of the whole term. The created matrix has rows and columns numbered with the available values of a combination of quantum numbers of $z^{th}$ component of the total orbital magnetic moment, spin magnetic moment $L_z$, $S_z$ or optionally with values of $z^{th}$ component or element of total moment $J_z$ with z indices, because z coordinate axis is the main axis of the Hamiltonian's quantisation. According to the fundamental rules of quantum mechanics, $L_z$ assumes values of the range from -L to L, with the increment equal to 1, $S_z$ analogically assumes values of the range from -S to S, and similarly, $J_z$ assumes values of the range from -J to J. In the case of the $Cr^{3+}$ example, it means that $L_z$ will assume values $L_z$ = -3,-2,-1,0,1,2,3, and $S_z$ = -3/2,-1/2,1/2,3/2; therefore, n = (2S+1)(2L+1) possible various combinations, for instance in case of $Cr^{3+}$ n = 28. Unique $L_z$, $S_z$ pairs are the discriminant of a row or column in the so-created matrix. The above assumption of values pertains to the $|L,S,L_z,S_z>$ basis in steps 1410 and 1430.

In case of calculations in a constrained basis $|L,S,J,J_z>$, the calculations are carried out only for the ground multiplet, or degenerated state of the electron structure of a free ion, for which L, S and J numbers are determined, where CEF and magnetic interactions lift the multiplet degeneration, leading to formation of a structure of separated energy states, and not - as in the above case - for the whole structure of the ground term, or degenerated state of the electron structure of a free ion, for which L, S numbers are determined, where CEF and magnetic interactions lift the degeneration of the whole term, together with possible multiplets, leading to formation of a structure of separated energy states. Therefore, in this case (|L,S,J,$J_z$>), no constant of spin-orbital coupling $\lambda_{s\text{-}o}$ is defined, and its value is assumed conceptionally as infinite. In spite of the clearly simplified character of such calculations, they are considered correct, e.g. for rare earth metal ions $Nd^{3+}$ -> L=6, S=3/2, J=9/2, the size of the created matrix is n=(2J+1)=10; thus columns and rows of the matrix will by numbered with values of $J_z$=-9/2,-7/2,-5/2,-3/2-1/2,1/2,3/2,5/2,7/2,9/2.

The method of filling of the created matrices, carried out in steps 1420, 1440, 1520, 1540, is based on the applied total energy operator, the so-called Hamiltonian, of the crystal field (CEF), and magnetic interactions in the form of internal spin-orbital coupling, and interaction with external magnetic field $B_{ext}$ defined by the user. Depending on the selected basis, i.e. |L,S,$L_z$,$S_z$>, the Hamiltonian has the following form:

$$\mathrm{H}_{LS} = \sum\sum \mathrm{B}_\mathrm{n}^\mathrm{m} \hat{\mathbf{O}}_\mathrm{n}^\mathrm{m}(\mathrm{L},\mathrm{L}_z) + \lambda \mathbf{L}\cdot\mathbf{S} + \mu_B(\mathbf{L} + g_e\mathbf{S})\cdot\mathbf{B}_{ext}$$

It should be noted that in the |L,S,J,$J_z$> basis, the form of the Hamiltonian does not contain the spin-orbit interaction, because in this model, an infinitely strong L and S coupling is assumed, resulting in a so-called good quantum number J, with values running through the J=L+S...|L-S| range, and the third Hund's rule determines its value for the ground multiplet. In such a case, the Hamiltonian assumes the following form:

$$\mathrm{H}_J = \sum\sum \mathrm{B}_\mathrm{n}^\mathrm{m} \hat{\mathbf{O}}_n^m(\mathrm{J},\mathrm{J}_z) + g_L \mu_B \mathbf{J}\cdot\mathbf{B}_{ext}$$

Filling of the Hamiltonian matrix with elements or components in the |L,S,$L_z$,$S_z$> basis is defined by the rules of construction of a matrix of angular momentum operators, and the construction itself is explained below. All Stevens operators $\mathbf{O}^m{}_n$ and operators of interaction with an external field are generated from fundamental components or elements in the following way:

$$< \mathrm{L},\mathrm{L}_z \,|\, \mathbf{L}_z \,|\, \mathrm{L},\mathrm{L}_z > = \mathrm{L}_z$$

$$< \mathrm{S},\mathrm{S}_z \,|\, \mathbf{S}_z \,|\, \mathrm{S},\mathrm{S}_z > = \mathrm{S}_z$$

$$< \mathrm{L},\mathrm{L}_z \pm 1 \,|\, \mathbf{L_x} \,|\, \mathrm{L},\mathrm{L}_z > = \frac{1}{2}\sqrt{(\mathrm{L} \mp \mathrm{L}_z)(\mathrm{L} \pm \mathrm{L}_z + 1)}$$

$$< \mathrm{S},\mathrm{S}_z \pm 1 \,|\, \mathbf{S_x} \,|\, \mathrm{S},\mathrm{S}_z > = \frac{1}{2}\sqrt{(\mathrm{S} \mp \mathrm{S}_z)(\mathrm{S} \pm \mathrm{S}_z + 1)}$$

Vertical dashes | separate the individual row elements, and a given row should be read as follows: < row | **matrix element of the operator** | column > = value of this element.

Therefore, the elements of the component spin-orbital matrix should be inserted into positions of the matrix according to the following recipe:

$$< \mathrm{L},\mathrm{S},\mathrm{L}_z,\mathrm{S}_z \,|\, \lambda\, \mathbf{L}\cdot\mathbf{S} \,|\, \mathrm{L},\mathrm{S},\mathrm{L}_z,\mathrm{S}_z > = \lambda\, \mathrm{L}_z\mathrm{S}_z$$

$$< \mathrm{L},\mathrm{S},\mathrm{L}_z,\mathrm{S}_z \,|\, \lambda\, \mathbf{L}\cdot\mathbf{S} \,|\, \mathrm{L},\mathrm{S},\mathrm{L}_z \pm 1,\mathrm{S}_z \mp 1 > = \\ = \frac{1}{2}\lambda\,(\sqrt{\mathrm{L}(\mathrm{L}+1) - \mathrm{L}_z(\mathrm{L}_z \pm 1)}\sqrt{\mathrm{S}(\mathrm{S}+1) - \mathrm{S}_z(\mathrm{S}_z \mp 1)})$$

Now, it is noteworthy that important commutation relations between the spin operators exist, allowing for constructing operators of directional components of **L+=** $\mathbf{L}_x$ + i $\mathbf{L}_y$ and L_= $\mathbf{L}_x$ - i $\mathbf{L}_y$ moments. This concerns any spin operators of one kind, thus:

$$\mathbf{J}_+ = \mathbf{J}_x + i\,\mathbf{J}_y \text{ and } \mathbf{J}_ = \mathbf{J}_x - i\,\mathbf{J}_y$$

where "i" is an imaginary number i= $(-1)^{1/2}$, and the notation of numbers of such a type requires using special, double matrices, which is carried out in steps 1410 and 1430.

Analogically, in the case of the $|L,S,J,J_z>$ basis, the elementary operators are generated as follows:

$$<J,J_z|\mathbf{J_z}|J,J_z>=J_z$$

$$<J,J_z+1|\mathbf{J}_+|J,J_z>=<J,J_z|\mathbf{J}_-|J,J_z+1>=\frac{1}{\sqrt{2}}\sqrt{(J-J_z)(J+J_z+1)}$$

$$\hat{O}_0^0=1$$

$$\hat{O}_1^0=\mathbf{J_z}$$

$$\hat{O}_1^1=\frac{1}{2}(\mathbf{J}_++\mathbf{J}_-)$$

$$\hat{O}_2^0=3\mathbf{J}_\mathbf{z}^2-J(J+1)$$

$$\hat{O}_2^1=\frac{1}{4}[(\mathbf{J_z}\mathbf{J}_++\mathbf{J}_+\mathbf{J_z})+(\mathbf{J_z}\mathbf{J}_-+\mathbf{J}_-\mathbf{J_z})]$$

$$\hat{O}_2^2=\frac{1}{2}(\mathbf{J}_+^2+\mathbf{J}_-^2)$$

$$\hat{O}_4^0=35\mathbf{J}_\mathbf{z}^4-[30J(J+1)-25]\mathbf{J}_\mathbf{z}^2+3J^2(J+1)^2-6J(J+1)$$

$$\hat{O}_4^2=[7\mathbf{J}_\mathbf{z}^4-J(J+1)-5](\mathbf{J}_+^2+\mathbf{J}_-^2)+(\mathbf{J}_+^2+\mathbf{J}_-^2)[7\mathbf{J}_\mathbf{z}^4-J(J+1)-5]$$

$$\hat{O}_4^3=\frac{1}{4}[\mathbf{J_z}(\mathbf{J}_{|}^3+\mathbf{J}^3)+(\mathbf{J}_{|}^3+\mathbf{J}^3)\mathbf{J_z}]$$

$$\hat{O}_4^4=\frac{1}{2}(\mathbf{J}_+^4+\mathbf{J}_-^4)$$

$$\hat{O}_6^0=231\mathbf{J}_\mathbf{z}^6-[315J(J+1)-736]\mathbf{J}_\mathbf{z}^4+[105J^2(J+1)^2-525J(J+1)+294]\mathbf{J}_\mathbf{z}^2+ \\ -5J^3(J+1)^3+40J^2(J+1)^2-60J(J+1)$$

$$\hat{O}_6^2=\frac{1}{4}\{\{337\mathbf{J}_\mathbf{z}^4-[18J(J+1)+123]\mathbf{J}_\mathbf{z}^2+J^2(J+1)^2+10J(J+1)+102\}(\mathbf{J}_+^2+\mathbf{J}_-^2)+ \\ +(\mathbf{J}_+^2+\mathbf{J}_-^2)\{337\mathbf{J}_\mathbf{z}^4-[18J(J+1)+123]\mathbf{J}_\mathbf{z}^2+J^2(J+1)^2+10J(J+1)+102\}$$

$$\hat{O}_6^3 = \frac{1}{4}\{[11\mathbf{J_z}^3 - 3J(J+1)\mathbf{J_z} - 59\mathbf{J_z}](\mathbf{J}_+^3 + \mathbf{J}_-^3) + (\mathbf{J}_+^3 + \mathbf{J}_-^3)[11\mathbf{J_z}^3 - 3J(J+1)\mathbf{J_z} - 59\mathbf{J_z}]\}$$

$$\hat{O}_6^4 = \frac{1}{4}\{[11\mathbf{J_z}^2 - J(J+1) - 38\mathbf{J_z}](\mathbf{J}_+^4 + \mathbf{J}_-^4) + (\mathbf{J}_+^4 + \mathbf{J}_-^4)[11\mathbf{J_z}^2 - J(J+1) - 38\mathbf{J_z}]\}$$

$$\hat{O}_6^6 = \frac{1}{2}(\mathbf{J}_+^6 + \mathbf{J}_-^6)$$

Thus, generation of the CEF Hamiltonian matrix in the |L,S,J,$J_z$> basis, carried out in steps 1440 and 1540, resolves itself into insertion of the determined values of products of the $B^m_n$ coefficients with definition constants into the coordinates defining the row <$J_z$ | and the column |$J'_z$ > of operators obtained in the result of substitution of relations for **J+, J_** and **J**$_z$ into the above definitions, into the created matrix.

In the case of generation of the Hamiltonian CEF matrix in the |L,S,$L_z$,$S_z$> basis, the component relations of Stevens operators $\mathbf{O}^m_n$ are identical as above, but they describe the operators of the orbital magnetic moment, because CEF does not interact directly onto spin S, or:

$$\hat{O}_0^0 = 1$$

$$\hat{O}_1^0 = \mathbf{L_z}$$

$$\hat{O}_1^1 = \frac{1}{2}(\mathbf{L}_+ + \mathbf{L}_-)$$

$$\hat{O}_2^0 = 3\mathbf{L_z}^2 - L(L+1)\ldots$$

$$\hat{O}_2^1 = \frac{1}{4}[(\mathbf{L_z}\mathbf{L}_+ + \mathbf{L}_+\mathbf{L_z}) + (\mathbf{L_z}\mathbf{L}_- + \mathbf{L}_-\mathbf{L_z})]$$

$$\hat{O}_2^2 = \frac{1}{2}(\mathbf{L}_+^2 + \mathbf{L}_-^2)$$

$$\hat{O}_4^0 = 35\mathbf{L_z}^4 - [30L(L+1) - 25]\mathbf{L_z}^2 + \ldots$$

However, generation of the Hamiltonian CEF matrix in the |L,S,$L_z$,$L_z$> basis, carried out in steps 1420 and 1520, resolves itself into insertion of the determined values of products of the $B^m_n$ coefficients with definition constants into the coordinates defining the row <$L_z$ | and column |$L'_z$> of operators obtained in the result of substitution of relations for **L$_+$, L_** and **L**$_z$ into the above definitions, into the created matrix.

According to the presented relations, Hamiltonian matrices are created automatically in order to bring them, in the next step 1550, to a diagonal form in step 1200, in order to solve the so-called eigenequation of the operator or to obtain pairs of vectors and eigenvalues of the operator, in this case - total energy operator, the so-called Hamiltonian.

In case of a matrix with complex elements, the created complex matrix is distributed in step 2210 into a system of real matrices with terms containing only real components and only imaginary component values, and then, according to the rules of matrix analysis, they are concatenated into a matrix with a doubled dimension n'=2n. Such an operation, based on the theorems on complex matrices, allows for carrying out of further calculations of complex matrices analogously as for real matrices. In particular, the diagonalisation procedure carried out in step 2100, or numerical bringing the matrix by arbitrary addition, subtraction and multiplication by the numbers of rows and columns in order to obtain the state in which non-zero elements are located only in the cells of the matrix having the same row number and column number, is universal for all situations. Diagonalisation of the matrix is carried out according to the Jacobi method, based on numerical recurrent techniques. The diagonalisation procedure of the created matrices requires an assumption of a proper diagonalisation precision parameter, influencing the duration of calculations to a very high extent. Considering the recurrent character of the diagonalisation process, an ideal diagonalisation never ends; therefore, the moment of stopping the calculations depends on the accuracy parameter - its value is configurable, but low enough (of the order of $10^{-12}$-$10^{-14}$) to be numerically treated as zero. The constants numerically defining the moment of stopping the diagonalisation procedure, by determination of the maximum number of iteration and minimum inaccuracy value, are defined in step 1200 each time the calculations start during the diagonalisation procedure.

The obtained matrix in the diagonal form constitutes a base for obtaining a solution, the so-called Hamiltonian eigenproblem, in step 2200 - or obtaining the energy eigenvalues, the so-called permissible energy levels together with wave functions corresponding to them and being linear combinations of basis vectors, resulting from the adopted basis and method. According to the foundations of quantum mechanics, normalised wave functions allow for calculating the expected values of observables, the so-called physical quantities connected with an operator creating them, in individual states. Considering the fact that the basis for calculations is in every case based on the so-called magnetic quantum numbers L, S or J, the expected values of states calculated in steps 2210 and 2220 directly from their wave functions are directional components of the total magnetic moments of these states.

In other words, as results from the Hamiltonian defined in the $|L,S,J,J_Z\rangle$ basis, with every $i^{th}$ state of the fine structure, its magnetic moment may be related:

$$<m_i^x> = <\Gamma_i|g_L\mu_B\mathbf{J_x}|\Gamma_i>, <m_i^y> = <\Gamma_i|g_L\mu_B\mathbf{J_y}|\Gamma_i> \text{ or } <m_i^z> = <\Gamma_i|g_L\mu_B\mathbf{J_z}|\Gamma_i>$$

Analogically, in the case of the $|L,S,L_Z,L_Z\rangle$ basis:

$$<m_i^x> = <\Gamma_i|\mu_B(\mathbf{L_x}+g_e\mathbf{S_x})|\Gamma_i>, <m_i^y> = <\Gamma_i|\mu_B(\mathbf{L_y}+g_e\mathbf{S_y})|\Gamma_i> \text{ or } <m_i^z> = <\Gamma_i|\mu_B(\mathbf{L_z}+g_e\mathbf{S_z})|\Gamma_i>$$

where: $g_L$ - constant called Landé g factor, defined with the following expression:

$$g_L = 1 + (1.002324)\frac{J(J+1)+S(S+1)-L(L+1)}{2J(J+1)}$$

where: $g_e$ = 2.002324
$\mu_B$ - Bohr magneton, equal to $\mu_B = 9.27 \cdot 10^{-24}$ J/T, J/T≡A·m²

Considering the fact that the matrix elements or components of operators of the y component of the magnetic moment $\mathbf{L}_y$, $\mathbf{S}_y$ and $\mathbf{J}_y$ have imaginary components and generate complex matrix elements, in the case when the calculations are limited to real matrices, this component of the individual states will not be possible to obtain in step 2220. Only z and x components of the magnetic moment of the individual states will be obtained then. In case of calculations in complex matrices, full information on the expected values of all Cartesian directional components x, y, z of the magnetic moment of the individual states, corresponding to specific energy levels calculated in step 2210, are obtained.

In step 2300, sorting and normalisation of energy eigenvalues with wave functions is carried out, being a universal procedure providing the first data for result visualisation and archiving. The energy eigenstates sorted ascending show in steps 2700 and 2800 the energy structure obtained as a result of an interaction of the defined ion with the defined charge environment of the crystal lattice. The structure of the eigenstates, together with wave functions corresponding to them and the expected values of directional components of the total magnetic moment in the individual states obtained in step 2300, calculated based on them, constitutes the basis for further calculations of material properties. Till now, precise quantum mechanic calculations of the energy structure of a single ion have been carried out. Passing to step 2400, it is assumed that a material containing the so-defined ions in its crystal structure is modelled. Thus, a model of an ideal material containing the defined ions with a calculated structure of energy states is created, the ions being spatially oriented identically, forming a perfect crystal, and not interacting with each other, but interacting with external fields, being subject to classical Boltzmann statistics. According to this statistics, at T = 0 K, only the ground state is occupied. In such a case, the magnetic moment of the ion is exactly equal to the ground state moment, or its calculated expected value. While simulating an increase in temperature, the probability of occupation of higher states increases according to Boltzmann statistics. According to these statistics, the number of ions in the state with energy of E*i* (population of the $i^{th}$ state) $N_i$ (T) at a non-zero temperature T is equal to: $N_i(T) = N_0 \frac{\exp\left(-\frac{E_i}{k_B T}\right)}{Z(T)}$ where Z(T) is the sum of states calculated using the universal relation:

$$Z(T) = Tr\left(\exp\left(-\frac{\hat{\mathbf{H}}_{CEF}}{k_B T}\right)\right) = \sum_i \exp\left(-\frac{E_i}{k_B T}\right)$$

$k_B$ is Boltzmann constant $k_B$ = 1.380488 $10^{-23}$ J/K, while $N_o$ is the initial number of ions, replaced in the case of the presented calculations with Avogadro's number $N_A$=6.02214129$\times10^{23}$ mole$^{-1}$, yielding results converted per one mole of the defined ions of a material. While numerically calculating the sums of states of the group of defined ions in step 2400, computation parameters in the form of the temperature range $\Delta$T and computation step $\sigma$T loaded in step 1200 are adopted. Thus, the set of population values for the individual states at specific non-zero temperatures $N_i$(T) is calculated cyclically based on Z(T) for every $k^{th}$ temperature step $T_k = T_{k-1} + \sigma T$.

Knowing the sum of states, free energy F(T) is calculated in step 2500, using the universal relation:

$$F(T) = -k_B T \ln Z(T)$$

which further allows for calculating the internal energy of the system of ions U(T):

$$U(T) = -k_B T \frac{\partial}{\partial T}\left(\frac{F(T)}{k_B T}\right) = F(T) - T\left(\frac{\partial F(T)}{\partial T}\right)$$

Based on the knowledge of thermodynamic functions of state, the number of system properties based on them may be determined. The internal energy of a system of discrete states at T $\neq$ 0, occupied according to classical Boltzmann statistics, converted per one mole of non-interacting paramagnetic ions, is a sum of the total energies of the individual ions.

$$U(T) = N_A \sum_{i=1} E_i p_i = N_A \sum_{i=1} \frac{E_i n_i}{Z} = N_A \sum_{i=1} \frac{E_i \exp\left(-\frac{E_i}{k_B T}\right)}{\left(1 + \sum_{j=1}^{N} \exp\left(-\frac{E_i}{k_B T}\right)\right)}$$

where $N_A$ is Avogadro's number.

Specific heat, converted per one mole of ions, is defined as a temperature derivative of internal energy:

$$c_{mol} = \left(\frac{\partial U(T)}{\partial T}\right)_{E_i} = N_A \cdot k_B \sum_{i=1} \left(\frac{E_i \exp\left(-\frac{E_i}{k_B T}\right)\left[E_i + E_i\left(\sum_{j=1} \exp\left(-\frac{E_j}{k_B T}\right)\right) - \left(\sum_{j=1} E_j \exp\left(-\frac{E_j}{k_B T}\right)\right)\right]}{T^2\left(1 + \sum_{j=1} \exp\left(-\frac{E_j}{k_B T}\right)\right)^2}\right)$$

where the product of the Avogadro constant and Boltzmann constant is the universal gas constant $N_A \cdot k_B = R$ = 8.31 J/K·mole.

The so-calculated electron heat is called Schottky heat or Schottky anomaly, and it constitutes a canon of thermodynamic calculations of localised electron systems.

The $c_{mol}$(T) curve has such a property that the temperature entropy change $\Delta$S(T), calculated based on it, determines the number of unoccupied states at a temperature of T. Within the framework of the method, thermodynamic entropy is calculated by numerical integration of the obtained $c_{mol}$(T) curve in the temperature range defined in step

2500 as:

$$S(T) = S(0) + \int_0^T \frac{c_{mol}(T)}{T} dT$$

The last step 2600 allows for obtaining information on the course of magnetic susceptibility of a material, based on the obtained electron structure of the defined ions with the expected values of directional components of the moments in step 2300 in the defined environment and thermodynamics of a statistical system of the above ions, calculated in the given temperature steps defined in step 2400.

Magnetic susceptibility for a paramagnetic state is calculated according to its definition as a ratio of the induced magnetisation, understood as a sum of the magnetic moments at a given temperature to the magnetic field applied. Investing the relations between the thermodynamic functions presented in the beginning of the section, one may relatively simply calculate the directional components of temperature dependency of the magnetic susceptibility of the $4f^n$ system in the crystal structure. In the limit of low external fields, the susceptibility is defined as a derivative:

$$\chi_f^j = \frac{\partial M_j(T)}{\partial B_j} = \frac{k_B T}{B_j}\left(\frac{\partial \ln Z(T)}{\partial B_j}\right)$$

where j (j=x, y, z) is a direction in a local coordination system connected with the axes of quantisation of an unclosed shell in CEF with a defined symmetry.

Considering the relations between the thermodynamic functions calculated in step 2500, the following expression is obtained:

$$\chi_j = \frac{N_A g_L^2 \mu_B^2}{Z}\left[\frac{\sum_{l=k}\left|\langle\Gamma_k \,|\, \mathbf{J_j} \,|\, \Gamma_l\rangle\right|^2}{k_B T}\exp\left(-\frac{E_l}{k_B T}\right) + 2\sum_{l\neq k}\frac{\left|\langle\Gamma_k \,|\, \mathbf{J_j} \,|\, \Gamma_l\rangle\right|^2}{(E_k - E_l)}\exp\left(\frac{-E_l}{k_B T}\right) - \right.$$
$$\left. - \frac{1}{k_B T}\left(\sum_l \left|\langle\Gamma l \,|\, \mathbf{J_j} \,|\, \Gamma_l\rangle\right|^2 \exp\left(-\frac{E_j}{k_l T}\right)\right)^2\right]$$

where l, k number the eigenstates of the Hamiltonian used.

For high temperatures and for very weak crystal fields, the above expression reduces itself to Curie-type susceptibility, and thus, among others, a tool in the form of processor-assigned software allows for comparing the results with a curve representing this law for a defined $m_{eff}$ parameter.

The presented method allows for clear determining of components of the total magnetic moments of ions with an unclosed shell forming a statistical system, simulating in this way the properties of a material with a crystal structure. Knowing that the total magnetic moment of an ion consists of an orbital part and a spin part, the moment is defined by the following equality $\mathbf{m_c = m_L + m_S}$. Knowledge of the expected values of components of the angular momentum vector of the individual states allows for determining the contributions of the spin and orbital parts of the magnetic moment.

Remembering that:

$$J_Z = L_Z + S_Z$$

and taking into account that $g_l$ = 1 and $g_s$= 2.002324:

$$m_c^z = m_L^z + m_S^z \quad \Rightarrow \quad g_L \mu_B J_Z \cong 1\mu_B L_Z + 2\mu_B S_Z$$

useful dependencies are obtained:

$$L_Z = (2 - g_L) J_Z, \; S_Z = (g_L - 1) J_Z \text{ or } m_S \cong 2 \mu_B (g_L - 1) J_Z, \; m_L = \mu_B (2 - g_L) J_Z$$

From the above relations, it results that ratios of the individual components of the magnetic moment to the total moment for calculations carried out in the $|L,S,J,J_z\rangle$ basis are constant for a given ion and temperature invariant. Thus:

$$\frac{m_S}{m_c} = \frac{2(2 - g_L)}{(g_L - 1)},$$

$$\frac{m_S}{m_c} = \frac{2(g_L - 1)}{g_L}$$

In case of calculations carried out in the full $|L,S,L_z,S_z\rangle$ basis, taking the coupling between states of various multiplets into account, the values of orbital and spin components are obtained directly, because of the specifics of precise calculations.

The presented optimisation enables determination of a full three- or two-dimensional distribution of magnetic properties, namely magnetic susceptibility, effective moment, spin and orbital components, expected values of magnetic moments in states and their components, spectral properties, namely the system of states, eigenfunctions, transfer matrix of transition probability and so on, and calorimetric properties, particularly Schottky specific heat and entropy. All the above properties are simulated in the form of temperature dependencies in the range defined by the user. Information on properties at a temperature of T = 0 K, where only the lowest level of the structure of states is occupied, is also available. The total magnetic moment of the system at T = 0 K is equal to the moment for the ground state. Population of states in non-zero temperatures determines the atomic magnetic properties of ions, directly affecting the simulated macroscopic magnetic properties of the compound.

On the basis of the structure of multi-electron states obtained as a consequence of the above calculations in the adopted basis $|L,S,J,J_z\rangle$ or $|L,S, L_z,S_z\rangle$ with the selection suggested for the type of ion, the following properties are predictable - as temperature dependencies obtained at a temperature range defined by the user, such as:

- electron entropy $S_e$ (T) connected with temperature filling of multi-electron states of the calculated electron structure of an ion or atom;
- electron component of specific heat $c_{mol}$(T) connected with temperature filling of multi-electron states of the calculated electron structure of the ion or atom;
- effective magnetic moment and its directional components $m_i$(B, T) in a defined coordination system;
- magnetic susceptibility and its directional components $\chi i(T)$;
- spectroscopic observability of inter-state transitions $\langle\Gamma i|J_\Gamma j\rangle$, $\langle\Gamma i|J+|\Gamma j\rangle$, $\langle\Gamma i|J_Z|\Gamma j\rangle$;
- spin and orbital component of the total magnetic moment $\langle\Gamma i|L|\Gamma i\rangle$, $\langle\Gamma i|S|\Gamma i\rangle$.

The optimisation presented above, in a theoretical way, will now be depicted in connection with exemplary elements.

Thus, in one of the examples, discussed in connection with Fig. 6 - 14, the chosen material is a material containing praseodymium ions $Pr^{3+}$ in a $PrCl_3$ crystal, having a hexagonal structure, with the electron structure corresponding to a $[Xe]4f^2$ structure.

Because of scientific consistence concerning the correctness of use of the J number as a "good quantum number" of ions with an unclosed shell 4f and 5f, or ions of lanthanides and actinides, calculations in the $|L,S,J,J_z\rangle$ basis are assumed correct in their case. On the basis of fundamental laws of atomic physics, including rules for the construction of electron shells and subshells of an atom, and the Pauli exclusion principle, and in consequence the Hund's rules, the basic values of quantum numbers of the ground term amount to: L=5, S=1, J=4. These values are commonly acknowledged and available as a canon in tables and handbooks on atomic physics and chemistry. The values of these numbers are a starting point for the method of calculations of the electron structure states. Because of relations between quantum numbers, values of the $J_z$ number are available, creating the basis or numbering the rows and columns of the CEF Hamiltonian matrix. For the discussed praseodymium ion $Pr^{3+}$, they amount to: one $L_z$= $|-4.0\rangle,|-3.0\rangle,|-2.0\rangle,|-1.0\rangle,|0.0\rangle,|1.0\rangle,|2.0\rangle,|3.0\rangle,|4.0\rangle$. Therefore, the order of the matrix to be created in the $|L,S,J,J_z\rangle$ basis amounts to: n=(2J+1)=9.

After obtaining a complete set of CEF parameters, defining the hexagonal field of the praseodymium ion environment in the $PrCl_3$ crystal, adopting values $B^0_2$=-1.43K, $B^0_4$=+50mK, $B^0_6$=-3.5mK, $B^6_6$=+35mK, an interactive three-dimensional visualisation of such a field 3100 in Cartesian coordinates correlated with quantisation axes of operators of Hamiltonian components is obtained, presented in Fig. 6. In Fig. 6, various values of CEF are evident, starting from low values 3120 to maximum values 3110.

Starting the calculations of the influence of the so-defined CEF on the $Pr^{3+}$ ion, the Hamiltonian matrix is generated on the basis of the defined Stevens operators. Numerical values of the filled matrix for the described praseodymium ion are shown in Fig. 7.

The Jacobi diagonalisation procedure, according to step 2100 from Fig. 5D, brings the matrix 3200 to the form with the diagonal 3210 and values 3220, allowing for solving its eigenequation and obtaining the complete set of eigenvalues and eigenvectors, according to step 2200 from Fig. 5D.

The eigenvalues of the total energy operator, the so-called Hamiltonian, 'EIGENVALUE' and eigenfunctions 'eigenfunction' corresponding to them and obtained from the solution of the Hamiltonian in the $|L,S,J,J_z>$ basis are presented below, while the elements of the wave function basis are shown as abbreviated, omitting the constants. For $Pr^{3+}$ in with L,S,J number, in general $|L,S,J,J_z>$=for $Pr^{3+}|5,1,4,J_z>= |J_z>$), the 'EIGENVALUE' values and eigenfunctions 'eigenfunction' corresponding to them, amount to:

| | |
|---|---|
| eigenfunction[1] = | +0.8941\|-4.0> +0.4478\|+2.0> |
| EIGENVALUE:E[1] = | 18.7767 |
| eigenfunction[2] = | +0.7071\|-3.0> +0.7071\|+3.0> |
| EIGENVALUE:E[2] = | 98.1186 |
| eigenfunction[3] = | +0.4478\|-2.0> +0.8941\|+4.0> |
| EIGENVALUE:E[3] = | 18.7767 |
| eigenfunction[4] = | +1\|-1.0> |
| EIGENVALUE:E[4] = | 41.2310 |
| eigenfunction[5] = | +1\|0.0> |
| EIGENVALUE:E[5] = | 161.0622 |
| eigenfunction[6] = | +1\|+1.0> |
| EIGENVALUE:E[6] = | 41.2310 |
| eigenfunction[7] = | -0.4478\|-4.0> +0.8941\|+2.0> |
| EIGENVALUE:E[7] = | -149.5155 |
| eigenfunction[8] = | -0.7071\|-3.0> +0.7071\|+3.0> |
| EIGENVALUE:E[8] = | -80.1652 |
| eigenfunction[9] = | +0.8941\|-2.0> -0.4478\|+4.0> |
| EIGENVALUE:E[9] = | -149. |

Calculation of the expected values according to step 2200 from Fig. 5D allows for obtaining a complete set of information useful for simulation of material properties on the basis on knowledge of the structure of electron energy states. As a consequence of selection of real computational space, knowledge on the expected values of directional operators of components is obtained for two directional components x and z.

Sorting of normalised wave functions and calculation of the expected values in step 2300 from Fig. 5E lead to obtaining a complete structure of the Hamiltonian eigenstates:

| Energy level \| | En-Eo \| | Jx \| | Sx \| | Lx \| | m(x) \| |
|---|---|---|---|---|---|
| -149.517953 | 0.000000 | -0.030607 | -0.03673 | 0.00612 | -0.02449 |
| -149.516098 | 0.001856 | -0.007440 | -0.00893 | 0.00149 | -0.00595 |
| -80.163279 | 69.354675 | 0.023975 | 0.02877 | -0.00480 | 0.01918 |
| 18.774289 | 168.292242 | -0.030718 | -0.03686 | 0.00614 | -0.02457 |
| 18.775766 | 168.293719 | -0.012284 | -0.01474 | 0.00246 | -0.00983 |
| 41.230363 | 190.748316 | -0.007819 | -0.00938 | 0.00156 | -0.00626 |
| 41.232504 | 190.750457 | 0.018916 | 0.02270 | -0.00378 | 0.01513 |
| 98.120109 | 247.638063 | 0.019243 | 0.02309 | -0.00385 | 0.01539 |
| 161.064298 | 310.582251 | 0.026735 | 0.03208 | -0.00535 | 0.02139 |
| Energy level \| | En-Eo \| | Jz \| | Sz \| | Lz \| | m(z) \| |
| -149.515502 | 0.000000 | -0.796762 | -0.95611 | 0.15935 | -0.63741 |
| -149.515502 | 0.000000 | 0.796762 | 0.95611 | -0.15935 | 0.63741 |
| -80.165199 | 69.350303 | 0.000000 | 0.00000 | -0.00000 | 0.00000 |

(continued)

| Energy level \| | En-Eo \| | Jz \| | Sz \| | Lz \| | m(z) \| |
|---|---|---|---|---|---|
| 18.776750 | 168.292251 | 2.796762 | 3.35611 | -0.55935 | 2.23741 |
| 18.776750 | 168.292251 | -2.796762 | -3.35611 | 0.55935 | -2.23741 |
| 41.230989 | 190.746491 | 1.000000 | 1.20000 | -0.20000 | 0.80000 |
| 41.230989 | 190.746491 | -1.000000 | -1.20000 | 0.20000 | -0.80000 |
| 98.118568 | 247.634070 | -0.000000 | -0.00000 | 0.00000 | -0.00000 |
| 161.062157 | 310.577658 | 0.000000 | 0.00000 | 0.00000 | 0.00000 |

The calculated values are presented in Fig. 8 in the diagram 3300 of energy states, in which the ground state 3310 and the next state 3320 are marked.

To define the influence of temperature on the properties of the ion, numerical summations are carried out in order to determine the sum of states Z(T), and calculations of filling of the individual states (populations) vs. temperature according to step 2400 from Fig. 5E for four parallel states are carried out. On the basis of calculations based on such statistics, results pertaining to the statistical behaviour of a large number of such ions in identical thermodynamic conditions were obtained. The operations were carried out according to formulas presented for step 2500 from Fig. 5E, by numerical integrations and differentiations directly during the visualisation or saving the results to a file according to steps 2700 and 2800 from Fig. 5E. Fig. 6 and 7 present the results of such calculations for the calculated structure of electron states of the $Pr^{3+}$ ion under the influence of a hexagonal CEF shown in Fig. 6. The temperature dependency 3510 of the specific heat component originating from the $Pr^{3+}$ ions is shown in Fig. 10 in plot 3500, while the dependency of entropy in two areas 3410 and 3420 vs. temperature is presented in Fig. 9 in plot 3400. In Fig. 9, horizontal lines 3430 are plotted indicating the relation of the calculated thermodynamic entropy originating from 4f electrons with the statistical entropy defined provisionally as a product of universal gas constant R (R=8.31 J/mole) and natural logarithm of the number of filled states.

The calculated expected values of operators: $\mathbf{J_x}$, $\mathbf{S_x}$, $L_x$ and $\mathbf{J_z}$, $\mathbf{S_z}$, $\mathbf{L_z}$ in eigenstates, allow for calculating the expected directional components of the expected values 3610, 3620 of magnetic moments of the individual $i^{th}$ states of electron structure < $m^i_x$> and < $m^i_z$>. This fact, in connection with knowledge on population of states vs. temperature, according to Boltzmann statistics, allows for calculating the directional magnetic susceptibility for possible simulation directions x and z. For convenience, in order to determine the so-called effective moment easily, the procedure of optimisation enables a visualisation of magnetic susceptibility in the inverse form, which is presented in Fig. 11 in the diagram 3600.

Lack of possibility to obtain information on y components during calculations in matrices based on real elements prevents construction of complete data on the spatial magnetic properties of a material. Realisation of calculations based on calculus of complex matrices allows for solving this problem. Moreover, it is more preferable to carry out the calculations in the full $|L,S,L_z,S_z>$ basis, in spite of the theoretical permissibility to use the $|L,S,J,J_z>$ basis for ions with an unclosed 4f and 5f shell, due to a more complete picture of the structure of states.

In case of the $|L,S,L_z,S_z>$ basis, the calculations are more time-consuming, by engaging larger hardware resources of the computing unit, but, from the theoretical point of view, they always ensure better results. Adoption of the $|L,S,L_z,S_z>$ basis provides an exemption from the necessity to adopt the assumption of preservation of the quantum number J. Therefore, as a consequence of the first two Hund's rules, the basic values of quantum numbers amount to L=5, S=1, respectively. The values of these numbers are a starting point for the method of calculations of the electron structure states. Because of the relations between quantum numbers, now their pairs $|L_z,S_z>$, and not $|J_z>$ as before, are creating the bases, or numbering rows and columns of the CEF Hamiltonian matrix. In case of the $Pr^{3+}$ ion being described, the states creating the basis for the calculations carried out are:

$$|L,S,L_z,S_z>=|\ L_z,S_z>:|+5,+1>,|+5,0>,|+5,-1>,|+4,+1>,|+4,0>,$$
$$|+4,-1>,|+3,+1>,|+3,0>,|+3,-1>,|+2,+1>,|+2,0>,|+2,-1>,|+1,+1>,|+1,0>,$$
$$|+1,-1>,|+0,+1>,|+0,0>,|+0,-1>,|-1,+1>,|-1,0>,|-1,-1>,|-2,+1>,|-2,0>,$$
$$|-2,-1>,|-3,+1>,|-3,0>,|-3,-1>,|-4,+1>,|-4,0>,|-4,-1>,|-5,+1>,|-5,0>,|-5,-1>.$$

Therefore, the order of the matrix to be created in the $|L,S,L_z,S_z>$ basis amounts to: n=(2L+1) (2S+1)=33.

After conversion of the field coefficients between the bases on the grounds of a database of transfer coefficients implemented in the tool, according to the relation between steps 800 and 540, a defined hexagonal CEF of the environment of the praseodymium ion in the $PrCl_3$ crystal is obtained with coefficients additionally containing information on the spin-orbit coupling:

$B^0_2$ = -1.43K, $B^0_4$=+50mK, $B^0_6$ =-3.5mK, $B^6_6$ =+35mK, $\lambda_{s-o}$=-650K

A fragment of the Hamiltonian matrix 3700 constructed in the space spanned across a body of complex numbers is shown in Fig. 12. A consequence of solving the Hamiltonian eigenequation described by the matrix from Fig. 12 is a structure 3800 of states shown in Fig. 13 with marked ground state 3810 and group 3820 of states. Analogically as above, directional magnetic susceptibility for possible simulation of directions z, x and y is calculated. A visualisation 3900 of the course of magnetic susceptibility vs. temperature, for all spatial components 3910, 3920, 3930, is presented in Fig. 14. One may see there a dramatically different course of the magnetic susceptibility curve in the field applied along the y axis. Such calculations enable construction of complete data on the spatial magnetic properties of a material.

In another example, discussed in connection with Fig. 15-24, the chosen material is a material containing an $Ni^{2+}$ ion in an oxide complex NiO, having a regular structure of the NaCl type, a coordination octahedron and an electron structure corresponding to the $[Ar]3d^8$ structure.

On the basis of fundamental laws of atomic physics and in consequence of the Hund's rules, the basic values of quantum numbers of the ground term amount to: L=3, S=1. The values of these numbers are a starting point for the method of calculations of the electron structure states. Because of the relations between quantum numbers, the available values of $L_z$ and $S_z$ numbers, creating the basis for the calculations, are:

$$|L,S,L_z,S_z\rangle=|L_z,S_z\rangle: |+3,+1\rangle,|+3,0\rangle,|+3,-1\rangle,|+2,+1\rangle,|+2,0\rangle, |+2,-1\rangle,|+1,+1\rangle,|+1,0\rangle,|+1,-1\rangle,|+0,+1\rangle,|+0,0\rangle,|+0,-1\rangle,|-1,+1\rangle, |-1,0\rangle,|-1,-1\rangle,|-2,+1\rangle,|-2,0\rangle,|-2,-1\rangle,|-3,+1\rangle,|-3,0\rangle,|-3,-1\rangle.$$

Automatically, together with selection of the basis according to step 500, a constant of the spin-orbit coupling is introduced as for a free ion: $\lambda_{s-o}$=41 meV, and its value is left unchanged for further calculations carried out according to step 660.

After obtaining CEF parameters, evaluated as: $B^0_4$=2 meV, $B^4_4$=10 meV, an interactive, three-dimensional visualisation of such a field 4100 is obtained in Cartesian coordinates correlated with the quantisation axes of operators of Hamiltonian components presented in Fig. 12 with dark areas 4110 and bright areas 4120.

The next step consists in generation of the Hamiltonian matrix, on the basis of the defined Stevens operators, while the apparent form of the matrix elements $d^8$ is the same as in the matrix $d^2$, and only the signs at the values of $\lambda_{s-o}$ are changed to opposites (+/-). The numerical values of a sector of the filled matrix 4200 are presented in Fig. 16. For promptitude of the demonstrative calculations, matrices with real elements are being selected, posing no large limitation in case of such a highly symmetrical system.

The Jacobi diagonalisation procedure according to step 1550 brings the matrix to a form allowing for solving its eigenequation and obtaining the complete set of eigenvalues and eigenvectors according to step 2200.

The calculated values of energy 'EIGENVALUE', eigenfunctions 'eigenfunction' $|L,S,L_z,S_z\rangle$ abbreviated as $|L_z,S_z\rangle$ have the following form:

EIGENVALUE:E[8] = 7641.1939

eigenfunction[9] = | 0.5192 | |3, |1.0>-0.48 | |1, -1.0>|0.48|-1, |1.0>-0.5192|-3,-1.0>

EIGENVALUE:E[9] = 7641.1939

eigenfunction[10]= +0.5637|+2,-1.0>-0.5198|+1,0.0>-0.0569|0,+1.0>+0.4285|-2,-1.0>+0.4746| 3,0.0>

EIGENVALUE:E[10] = -2464.5964

eigenfunction[11]=+0.4564|+3,+1.0>+0.3536|+1, -1.0>+0.5774|0,0.0>+0.3536|-1,+1.0>+0.4564|-3,-1.0>

EIGENVALUE:E[11] = 6840.0000

eigenfunction[12] =+0.4746|+3,0.0>+0.4285|+2,+1.0>-0.0569|0,-1.0>-0.5198|-1,0.0>+0.5637| 2,+1.0>

EIGENVALUE:E[12] = -2464.5964

eigenfunction[13] = +0.48|+3,+1.0>+0.5192|+1,-1.0>-0.5192|-1,+1.0>-0.48|-3,-1.0>

EIGENVALUE:E[13] = -3081.1939

eigenfunction[14]= -0.3827|+3,0.0>+0.4981|+2,+1.0>+0.0615|0,-1.0>+0.5946|-1,0.0>+0.4981| 2,+1.0>

EIGENVALUE:E[14]= -3081.1939

eigenfunction[15]=-0.3847|+3,-1.0>+0.0676|+2,0.0>-0.5895|+1,+1.0>+0.5895|-1,-1.0>-0.0676|-2,0.0> +0.3847|-3,+1.0>

EIGENVALUE:E[15] = -2464.5964

(continued)

eigenfunction[16]= +0.0433|+3,0.0>+0.7517|+2,+1.0>-0.0024|0,-1.0-0.0519|-1,0.0-0.656| 2,+1.0>
EIGENVALUE:E[16]= -16690.3836
eigenfunction[17]=-0.1748|+3,-1.0>+0.5724|+2,0.0>+0.3766|+1,+1.0>+0.3766|-1,-1.0>+0.5724|-2,0.0> -0.1748|-3,+1.0>
EIGENVALUE:E[17]= -3212.2460
eigenfunction[18] =+0.4981|+2,-1.0>+0.5946|+1,0.0>+0.0615|0,+1.0>+0.4981|-2,-1.0>-0.3827| 3,0.0>
EIGENVALUE:E[18]= -3081.1939
eigenfunction[19]=+0.0395|+3,-1.0>+0.7039|+2,0.0>+0.0549|+1,+1.0>-0.0549|-1,-1.0>-0.7039|-2,0.0> -0.0395|-3,+1.0
EIGENVALUE:E[19] =-16690.3836
eigenfunction[20]=-0.6561|+2,-1.0>-0.0519|+1,0.0>-0.0024|0,+1.0>+0.7517|-2,-1.0>+0.0433|-3,0.0>
EIGENVALUE:E[20]=-16690.3836
eigenfunction[21]=-0.4715|+3,+1.0>+0.5215|+1,-1.0>+0.1067|0,0.0>+0.5215|-1,+1.0>-0.4715|-3,-1.0>
EIGENVALUE:E[21]=-3212.2460

Calculation of the expected values according to step 2200 from Fig. 5D allows for obtaining a complete set of information useful for simulation of material properties on the basis on the structure of states. As a consequence of selection of real computational space, knowledge on the expected values of directional operators of moment components will be obtained for two directional components x and z.

Sorting of normalised wave functions and calculation of the expected values in step 2300 from Fig. 5E lead to obtaining a complete structure of the Hamiltonian eigenstates:

| Energy level | En-Eo | Jx | Sx | Lx | m(x) |
|---|---|---|---|---|---|
| -16690.601145 | 0.000000 | -1.267532 | -0.99541 | -0.27212 | -2.26526 |
| -16690.383555 | 0.217590 | -0.000039 | -0.00000 | -0.00004 | -0.00004 |
| -16690.165965 | 0.435180 | 1.267455 | 0.99541 | 0.27204 | 2.26518 |
| -3212.246464 | 13478.354681 | -0.007063 | -0.00385 | -0.00321 | -0.01092 |
| -3212.245986 | 13478.355159 | -0.000118 | -0.00017 | 0.00005 | -0.00029 |
| -3081.298277 | 13609.302868 | -0.578373 | -0.49255 | -0.08582 | -1.07207 |
| -3081.193407 | 13609.407738 | 0.006961 | 0.00385 | 0.00311 | 0.01082 |
| -3081.089535 | 13609.511609 | 0.578308 | 0.49231 | 0.08600 | 1.07176 |
| -2464.705362 | 14225.895783 | -0.635400 | -0.49797 | -0.13743 | -1.13453 |
| -2464.596520 | 14226.004624 | -0.000956 | -0.00116 | 0.00020 | -0.00212 |
| -2464.487443 | 14226.113702 | 0.635449 | 0.49822 | 0.13723 | 1.13482 |
| -2279.999877 | 14410.601268 | 0.001035 | 0.00133 | -0.00029 | 0.00236 |
| 6839.999932 | 23530.601077 | -0.001070 | -0.00048 | -0.00059 | -0.00155 |
| 7641.156682 | 24331.757827 | 0.421426 | -0.49253 | 0.91396 | -0.07225 |
| 7641.193953 | 24331.795098 | 0.000952 | 0.00037 | 0.00059 | 0.00132 |
| 7641.231082 | 24331.832227 | -0.421892 | 0.49232 | -0.91422 | 0.07158 |
| 9074.920835 | 25765.521980 | 0.097361 | -0.49340 | 0.59076 | -0.39719 |
| 9074.979921 | 25765.581065 | 0.000951 | -0.00115 | 0.00210 | -0.00020 |
| 9075.039127 | 25765.640272 | -0.096803 | 0.49361 | -0.59042 | 0.39796 |
| 9212.245985 | 25902.847130 | 0.000220 | 0.00011 | 0.00011 | 0.00033 |
| 9212.246018 | 25902.847162 | -0.000874 | 0.00115 | -0.00202 | 0.00027 |

| Energy level | En-Eo | Jz | Sz | Lz | m(z) |
|---|---|---|---|---|---|
| -16690.383554 | 0.000000 | 1.267493 | 0.99541 | 0.27208 | 2.26522 |
| -16690.383554 | 0.000000 | 0.000000 | 0.00000 | 0.00000 | 0.00000 |
| -16690.383554 | 0.000000 | -1.267493 | -0.99541 | -0.27208 | -2.26522 |
| -3212.245971 | 13478.137583 | -0.000000 | -0.00000 | -0.00000 | -0.00000 |

(continued)

| Energy level | En-Eo | Jz | Sz | Lz | m(z) |
|---|---|---|---|---|---|
| -3212.245971 | 13478.137583 | 0.000000 | 0.00000 | 0.00000 | 0.00000 |
| -3081.193897 | 13609.189657 | 0.578341 | 0.49243 | 0.08591 | 1.07191 |
| -3081.193897 | 13609.189657 | 0.000000 | -0.00000 | 0.00000 | 0.00000 |
| -3081.193897 | 13609.189657 | -0.578341 | -0.49243 | -0.08591 | -1.07191 |
| -2464.596411 | 14225.787142 | 0.635425 | 0.49810 | 0.13733 | 1.13468 |
| -2464.596411 | 14225.787142 | -0.635425 | -0.49810 | -0.13733 | -1.13468 |
| -2464.596411 | 14225.787142 | -0.000000 | 0.00000 | -0.00000 | -0.00000 |
| -2280.000000 | 14410.383554 | -0.000000 | -0.00000 | 0.00000 | -0.00000 |
| 6840.000000 | 23530.383554 | -0.000000 | -0.00000 | -0.00000 | -0.00000 |
| 7641.193897 | 24331.577450 | -0.421659 | 0.49243 | -0.91409 | 0.07191 |
| 7641.193897 | 24331.577450 | 0.000000 | 0.00000 | 0.00000 | 0.00000 |
| 7641.193897 | 24331.577450 | 0.421659 | -0.49243 | 0.91409 | -0.07191 |
| 9074.979965 | 25765.363518 | -0.097082 | 0.49351 | -0.59059 | 0.39757 |
| 9074.979965 | 25765.363518 | 0.000000 | 0.00000 | 0.00000 | 0.00000 |
| 9074.979965 | 25765.363518 | 0.097082 | -0.49351 | 0.59059 | -0.39757 |
| 9212.245971 | 25902.629524 | -0.000000 | 0.00000 | -0.00000 | -0.00000 |
| 9212.245971 | 25902.629524 | 0.000000 | -0.00000 | 0.00000 | 0.00000 |

The calculated values may be visualised directly in the diagram 4300 of energy states presented in Fig. 17 with marked ground state 4310 and group 4320 of the next states.

The next step consists in calculation of the sum of states and populations of the individual states vs. temperature according to step 2500 from Fig. 5E. After the calculations, results pertaining to the statistical behaviour of a large number of such ions in identical thermodynamic conditions are obtained. The operations are carried out according to formulas presented earlier for step 2200 from Fig. 5D, by numerical integrations and differentiations directly during the visualisation or saving the results to a file in steps 2700 and 2800 from Fig. 5E.

In the result of statistical calculations, it is evident that only a minimal contribution to specific heat originating from d electrons exists, because of a triplet ground state highly separated energetically. Knowledge on thermodynamic functions allows for simulating the directional components of magnetic susceptibility vs. temperature.

In this case, both calculated directions are equivalent, as one may expect introducing an octahedral field with a regular symmetry. The result of calculations of magnetic susceptibility 4410 measured along the field parallel to the x and z axes is presented in Fig. 18 in the diagram 4400. The simulation procedure allows for comparing the obtained result with curve 4420 representing the Curie law with a defined value of the effective moment. In this case, the value of the effective number of Bohr magnetons was assumed as 2.5.

A crystal field with a lower symmetry is able to split the directional components, determining an easy magnetisation axis. The calculations using the defined tools allow for simulating the effect of deformation of the oxygen octahedron coordinating the $Ni^{+2}$ ion.

In fact, such deformations may be of a structural origin or they may result from the influence of external forces. They may have a natural origin as a consequence of Jahn-Teller effect, as well as a consequence of induced external conditions. Such a simulation leads to results with properties directionally diffused, which may be seen in full calculations in a complex space. The simulation of deformation of the coordination octahedron of the $Ni^{2+}$ ion is realised by the introduction of additional components of the quadrupole moment described with the $O^2_2$ operator. The result of exemplary calculations leads to a splitting of the ground triplet into singlet components 4510, 4520 presented in Fig. 19 in the diagram 4500. Such a splitting of the state 4620, 4630 is revealed as a maximum 4610 in the specific heat c(T) course shown in Fig 20 in the diagram 4600 and the course 4710 of entropy with a straight line 4720 marked in the diagram 4700, and magnetic susceptibility for all spatial components 4810, 4820, 4830 presented in Fig. 21 in the diagram 4800, as well as magnetic susceptibility 4900 in the inverse form 4910, 4920, 4930, and it allows for modelling, anticipating and describing the properties of the material in the function of achievable structural modifications.

## Claims

1. A computer implemented method for finding materials having defined properties, during which a material containing ions of at least one Mendeléev Table component element with unclosed electron shells is selected based on information available in the state of art, the method comprising the steps
defining at least one kind of ions being the basis for calculations and material simulations by choosing (200) at least one component element of a chosen material directly from the Mendeléev Table,
determining (300) an oxidation state of atoms of the component element being selected in the chosen material to define their electron configuration,
for selected at least one kind of ions of the component element, after determining values of quantum numbers of an orbital magnetic moment L, answering (500) a question whether Crystal Electric Field (CEF) coefficients are known and, in case when Crystal Electric Field (CEF) coefficients are known, inputting (560) Crystal Electric Field (CEF) coefficients defined by Stevens coefficients having a form of $B^m_n$ or, in case when Crystal Electric Field (CEF) coefficients are not known,
after selecting (510) a method for finding values of Crystal Electric Field (CEF) coefficients, carrying out (520, 530, 540) calculations of Stevens coefficients with the form $B^m_n$ and inputting (560) Crystal Electric Field (CEF) coefficients defined by Stevens coefficients having the form of $B^m_n$,
starting (600) calculations with predefined parameters or, after selecting (610) a calculation method, carrying out calculations of a spin magnetic moment S and orbital magnetic moment L when an $|L,S,L_z,S_z>$ basis is chosen according to a chosen calculation space (640) or a total magnetic moment J corresponding to a ground state of the selected ions when an $|L,S,J,J_z>$ basis is chosen for finding a complete set of Crystal Electric Field (CEF) coefficients, defined by Stevens coefficients having the form of $B^m_n$, expressed in energy units and defining an immediate charge environment of the selected ions in a crystal lattice by calculation of Stevens coefficients having the form of $B^m_n$,
based on the Stevens coefficients having the form of $B^m_n$, generating a total energy operator (1410, 1420, 1430, 1440, 1510, 1520, 1530, 1540), called a Hamiltonian matrix containing matrix components being elements of Stevens operators multiplied by the defined Stevens coefficients $B^m_n$ ($H_{CEF}=\sum B^m_n O^m_n$), (x, y, z) or (x, z) components of operators of magnetic field potential, projection operators of orbital magnetic moment, spin magnetic moment and total magnetic moment, and components of operators of the spin-orbit coupling,
after carrying out operations on the matrix, creating a model of an ideal material containing the selected ions, the selected ions being spatially oriented identically and not interacting with each other, but interacting with external magnetic and electric fields, with a calculated structure of energy states together with their spectral properties, and being subjected to classical Boltzmann statistics, and having the directional (x, y, z) or (x, z) components of magnetic properties calculated,
based on the model of the ideal material, defining calorimetric, electron and magnetic properties in a form of temperature dependencies of material containing ions in the defined environment of the Crystal Electric Field (CEF), and verifying the properties of the ideal material with properties of a real material, whether the properties of the material obtained from calculations correspond to the properties of the material being searched for.

**2.** The method according to claim 1, wherein the value of the quantum numbers of the orbital magnetic moment L, the spin magnetic moment S and optionally the total magnetic moment J corresponding to the ground state of electron configuration of the selected ions is determined based on Hund's rules.

**3.** The method according to claim 1, wherein calculation of Stevens coefficients with the form of $B^m_n$ is carried out after choosing (610) one of calculation methods and determining (630) a computation space, choosing (640) a basis for calculations and determining (650) values of constants.

**4.** The method according to claim 3, wherein calculations of Stevens coefficients with the form of $B^m_n$ are carried out using a Point Charge Model (520) (PCM) or using an interactive three-dimensional (3D) visualisation (530) of component multipoles of the electric field and their superpositions defined as crystal field (CEF) or by a conversion (540) of CEF coefficients ($A^m n$ -> $B^m_n$) from known results of other calculations for systems isostructural with the one being calculated, but containing other ions.

**5.** The method according to claim 4, wherein results of calculations of the Stevens coefficients with the form of $B^m_n$ are harmonised by comparing obtained results using the Point Charge Model Approximation (PCM) (520) or the interactive visualisation (530) of the crystal field (CEF) in 3D, or by the conversion (540) of crystal field (CEF) coefficients ($A^m n$ -> $B^m_n$) from results of other calculations for systems isostructural with the one being calculated, but containing other ions.

**6.** The method according to claim 1, wherein all operations leading to calculation of the structure of states of the selected ions in the defined environment in the crystal lattice are carried out after choosing (630) the computation space from the vector space spanned across a body of real numbers and space spanned across a body of complex numbers, and choosing a basis for construction of the Hamiltonian matrix or the total energy operator.

**7.** The method according to claim 6, wherein after choosing (630) the space of real numbers and carrying out the calculations in $|L,S,J,J_Z>$ basis, while generating the matrix containing the products of the matrix elements of Stevens operators and defined Stevens coefficients ($B^m{}_n$, $\mathbf{O}^m{}_n$**)** and operators of directional components (x, z) of the magnetic field, at first, an empty matrix (1430, 1530) is created with rows and columns numbered with values of $|J_z>$, the matrix being filled (1440, 1540) with products of the matrix elements of Stevens operators and defined Stevens coefficients ($B^m{}_n$, $\mathbf{O}^m{}_n$**)** and component operators (x, z) of the magnetic field, and after choosing the space of real numbers and carrying out the calculations with $|L, S, L_z, S_z>$ basis, while generating the matrix containing the products of the matrix elements of Stevens operators and defined Stevens coefficients ($B^m{}_n$, $O^m{}_n$) and component operators (x, z) of the magnetic field, at first, an empty matrix is created (1410, 1510) with rows and columns numbered with $|L_z, S_z>$ combinations, which, as an initially prepared Hamiltonian matrix, is filled (1420, 1520) with components of Stevens operators ($B^m{}_n$, $O^m{}_n$), component operators (x, z) of the magnetic field and components of the spin-orbit coupling operator.

**8.** The method according to claim 6, wherein after choosing the space of real numbers and complex numbers, and carrying out the calculations in the $|L,S,J,J_Z>$ basis, while generating the matrix (1440, 1540) containing fillings with the products of the matrix elements of Stevens operators and defined Stevens coefficients ($B^m{}_n$, $\mathbf{O}^m{}_n$**),** and component operators (x, y, z) of the magnetic field, at first, an empty matrix (1430, 1530) is created with rows and columns numbered with values of $|J_z>$, the matrix being filled (1440, 1540) with products of the matrix elements of Stevens operators and defined Stevens coefficients ($B^m{}_n$, $\mathbf{O}^m{}_n$**),** total moment projection operators and component operators (x, y, z) of the magnetic field, and after choosing the space of complex numbers and carrying out the calculations with the $|L, S, L_z, S_z>$ basis, while generating the matrix containing the products of the matrix elements of Stevens operators and defined Stevens coefficients ($B^m{}_n$, $\mathbf{O}^m{}_n$**)** and component operators (x, y, z) of the magnetic field, at first, an empty matrix is created (1410, 1510) with rows and columns numbered with $|L_z, S_z,>$ combinations, which, as a Hamiltonian matrix, is filled (1420, 1520) with components of Stevens operators ($B^m{}_n$, $\mathbf{O}^m{}_n$**),** projection operators of total spin and orbital magnetic moments, component operators (x, y, z) of the magnetic field and components of the spin-orbit coupling operators.

**9.** The method according to claim 7 or 8, wherein after filling (1420, 1440, 1520, 1540) with the products of the matrix elements of Stevens operators and defined Stevens coefficients ($B^m{}_n$, $\mathbf{O}^m{}_n$**),** projection operators of total spin and orbital magnetic moments, component operators (x, z) or (x, y, z) of the magnetic field and optionally with components of the spin-orbit coupling operator, diagonalisation of the Hamiltonian matrix is carried out (1550), and after the diagonalisation (2100) of the Hamiltonian matrix, n-complete sets of pairs of energy eigenvalues E' (i=1..n) and eigenfunctions being linear combinations of basis vectors are calculated (2200), and next, based on their form, the expected values of the directional components (x,z) or (x,y,z) of magnetic moments of the individual n-eigenstates of energy E' are calculated (2210, 2220).

**10.** The method according to claim 9, wherein n-eigenstates of energy E' are sorted (2300) with their expected values of directional components of magnetic moments $<m^i_j>$ (i=1..n, j=x,z or j=x,y,z) of the individual states, and next, the sum of states Z(T) and population $N^i(T)$ of every energy state of the obtained structure are calculated (2400) in defined temperature increments according to Boltzmann statistics, based on which the courses of temperature dependencies of free energy, internal energy, entropy, magnetic susceptibility, calculated for a field applied along (x and z) or (x, y and z) directions, and Schottky specific heat in order to determine the calorimetric, electron and magnetic properties of a material containing ions in the defined environment of the crystal field (CEF) are calculated (2500).

**11.** The method according to claim 10, wherein a new complete set of result data is created (2700), comprising the calorimetric, electron and magnetic properties of a material containing ions in the defined environment of the crystal field (CEF) together with an interactive visualisation of this environment and calculation parameters, and the new complete set of result data is presented in the form of an independent set of data available directly and in parallel with other result data, enabling direct comparisons of the obtained results.

**12.** The method according to claim 11, wherein various separate complete sets of the result data are archived (2800) in a single merged numerical form together with the data pertaining to calculations, simulations and visualisations of every separate set of the result data, and the numerical form of the result data enables access to a chosen property or a course of the temperature dependency of a chosen property from different complete sets of the result data simultaneously.

**13.** The method according to claim 12, wherein the form of the result data enables implementation of the saved result data and comparison with adequate current calculations.

**14.** A system for finding materials having defined properties, the system adapted to carry out any of the method of claims 1 to 13, the system comprising a computing unit (10) with a processor (20),
a device (95) for presentation of data and calculation results and with access to data on materials,
a testing unit (15) adapted to carry out tests on real materials and communicating with the computing unit (10),
**characterised in that** the processor (20) comprises
a module (30) for finding and selecting elements of the chosen material, enabling determination of their electron configuration based on values of quantum numbers of orbital magnetic moment L, spin magnetic moment S when an $|L,S,L_z,S_z>$ basis is chosen or based on values of total magnetic moment J when an $|L,S,J,J_z>$ basis is chosen,
a module (40) for finding a complete set of Crystal Electric Field (CEF) coefficients, defined by Stevens coefficients with the form of $B^m_n$,
a module (50) for preparation of data for the calculations with a module (51) for selection of computational technique and a module (52) for determination of input data, space and basis of the calculations, the module (50) communicating with the module (40),
a module (60) for construction of a model of an ideal material containing defined ions and adapted to communicating with the module (40) for finding a complete set of Crystal Electric Field (CEF) coefficients, the ions being spatially oriented identically and not interacting with each other, but interacting with external fields, with a calculated structure of energy states together with their spectral properties, and being subjected to classical Boltzmann statistics, and having directional (x, y, z) or (x, z) components of magnetic properties calculated, the module (60) for construction of a model of the ideal material being connected with the testing unit (15) in order to verify the model of the ideal material with a real material,
a module (80) for comparison of the ideal material with the real material in order to verify the model of the ideal material with the real material, when the properties of the material obtained from calculations correspond to the properties of the material being searched for.

**15.** The system according to claim 14, wherein the module (60) for construction of the model of the ideal material comprises a module (64) for calculation of complete sets of pairs of energy eigenvalues E' (i=1..n) and eigenfunctions being linear combinations of basis vectors, and a module (68) for calculation of courses of temperature dependencies of free energy, internal energy, entropy, magnetic susceptibility, calculated for a field applied along (x and z) or (x, y and z) directions, and Schottky specific heat in order to determine calorimetric, electron and magnetic properties of a material containing ions in defined environment of the crystal field (CEF).

## Patentansprüche

**1.** Ein computerimplementiertes Verfahren zum Auffinden von Materialien, die definierte Eigenschaften aufweisen, bei dem ein Material, das Ionen mindestens eines Elements der Mendelejewschen Tabelle mit ungeschlossenen Elektronenhüllen enthält, basierend auf Informationen, die im Stand der Technik verfügbar sind, das Verfahren umfassend die folgenden Schritte
Definieren von mindestens einer Art von Ionen als Grundlage für Berechnungen und Materialsimulationen durch Auswählen (200) von mindestens einem Komponentenelement eines direkt aus der Mendelejewschen Tabelle ausgewählten Materials,
Bestimmen (300) eines Oxidationszustands von Atomen des ausgewählten Komponentenelements in dem gewählten Material, um ihre Elektronenkonfiguration zu definieren,
für mindestens eine ausgewählte Art von Ionen des Komponentenelements nach Bestimmen von Werten von Quantenzahlen eines orbitalen magnetischen Moments L, Beantworten (500) einer Frage, ob Koeffizienten des elektrischen Kristallfelds (Crystal Electric Field, CEF) bekannt sind, und, wenn Koeffizienten des elektrischen Kristallfelds (CEF) bekannt sind, Eingeben (560) von Koeffizienten des elektrischen Kristallfelds (CEF), die durch Stevens-Koeffizienten definiert sind, die eine Form von $B^m_n$ aufweisen, oder, wenn Koeffizienten des elektrischen Kristallfelds (CEF) nicht bekannt sind,
nach Auswählen (510) eines Verfahrens zum Finden von Werten von Koeffizienten des elektrischen Kristallfelds (CEF), Ausführen (520, 530, 540) von Berechnungen von Stevens-Koeffizienten mit der Form $B^m_n$ und Eingeben (560) von Koeffizienten des elektrischen Kristallfelds (CEF), die durch Stevens-Koeffizienten definiert sind, die die Form $B^m_n$ aufweisen,
Beginnen (600) von Berechnungen mit vordefinierten Parametern oder, nach Auswählen (610) eines Berechnungsverfahrens, Durchführen von Berechnungen eines magnetischen Spinmoments S und eines magnetischen Orbitalmoments L, wenn eine Basis $|L,S,L_Z,S_Z>$ gemäß einem gewählten Berechnungsraum (640) oder eines magnetischen Gesamtmoments J, das einem Grundzustand der ausgewählten Ionen entspricht, wenn eine Basis $|L,S,J,J_Z>$ gewählt wird, um einen vollständigen Satz von Koeffizienten des elektrischen Kristallfelds (CEF) zu finden, die durch Stevens-Koeffizienten definiert sind, die die Form von $B^m_n$ aufweisen, ausgedrückt in Energieeinheiten, und Definieren einer unmittelbaren Ladungsumgebung der ausgewählten Ionen in einem Kristallgitter durch Berechnung von Stevens-Koeffizienten, die die Form von $B^m_n$ aufweisen,
basierend auf den Stevens-Koeffizienten, die die Form von $B^m_n$ aufweisen, Erzeugen eines Gesamtenergie-Operators (1410, 1420, 1430, 1440, 1510, 1520, 1530, 1540), der als Hamilton-Matrix bezeichnet wird, die Matrixkomponenten enthält, die Elemente von Stevens-Operatoren sind, die mit Komponenten von definierten Stevens-Koeffizienten $B^m_n$ ($H_{CEF}=\sum B^m_n O^m_n$), (x, y, z) oder (x, z) Operatoren des magnetischen Feldpotentials, Projektionsoperatoren des magnetischen Orbitalmoments, des magnetischen Spinmoments und des magnetischen Gesamtmoments, und Komponenten von Operatoren der Spin-Orbit-Kopplung multipliziert werden,
nach Durchführen von Operationen über die Matrix, Erstellen eines Modells eines idealen Materials, das die ausgewählten Ionen enthält, wobei die ausgewählten Ionen räumlich identisch ausgerichtet sind und nicht miteinander interagieren, sondern mit äußeren magnetischen und elektrischen Feldern interagieren, mit einer berechneten Struktur von Energiezuständen zusammen mit ihren spektralen Eigenschaften, und die einer klassischen Boltzmann-Statistik unterzogen werden, und die Richtungskomponenten (x, y, z) oder (x, z) mit berechneten magnetischen Eigenschaften aufweisen,
basierend auf dem Modell des idealen Materials, Definieren der kalorimetrischen, elektronischen und magnetischen Eigenschaften in Form von Temperaturabhängigkeiten des Materials mit Ionen in der definierten Umgebung des elektrischen Kristallfelds (CEF), und
Überprüfen der Eigenschaften des idealen Materials mit Eigenschaften eines reellen Materials, ob die aus den Berechnungen erlangten Eigenschaften des Materials mit den Eigenschaften des gesuchten Materials übereinstimmen.

2. Das Verfahren nach Anspruch 1, **dadurch gekennzeichnet, dass** der Wert der Quantenzahlen des magnetischen Orbitalmoments L, des magnetischen Spinmoments S und optional des magnetischen Gesamtmoments J, die dem Grundzustand der Elektronenkonfiguration der ausgewählten Ionen entsprechen, basierend auf Hundschen Regeln bestimmt wird.

3. Das Verfahren nach Anspruch 1, **dadurch gekennzeichnet, dass** die Berechnung der Stevens-Koeffizienten mit der Form $B^m_n$ nach Auswählen (610) eines von Berechnungsverfahren und Bestimmen (630) eines Berechnungsraums, Auswählen (640) einer Basis für Berechnungen und Bestimmen (650) von Werten von Konstanten durchgeführt wird.

4. Das Verfahren nach Anspruch 3, **dadurch gekennzeichnet, dass** Berechnungen von Stevens-Koeffizienten mit der Form $B^m_n$ unter Verwendung eines Punktladungsmodells (520) (Point Charge Model, PCM) oder unter Verwendung einer interaktiven dreidimensionalen (3D) Visualisierung (530) von Komponentenmultipolen des elektrischen Felds und deren als Kristallfeld (CEF) definierten Überlagerungen oder durch eine Umrechnung (540) von CEF-Koeffizienten ($A^m n$ -> $B^m_n$) aus bekannten Ergebnissen anderer Berechnungen für Systeme, die mit dem zu berechnenden isostrukturell sind, aber andere Ionen enthalten, durchgeführt werden.

5. Das Verfahren nach Anspruch 4, **dadurch gekennzeichnet, dass** Ergebnisse von Berechnungen der Stevens-Koeffizienten mit der Form $B^m_n$ harmonisiert werden, indem erlangte Ergebnisse unter Verwendung der Punktladungsmodell-Annäherung (PCM) (520) oder der interaktiven Visualisierung (530) des Kristallfelds (CEF) in 3D verglichen werden, oder durch die Umrechnung (540) von Kristallfeld-(CEF)-Koeffizienten ($A^m n$ -> $B^m_n$) aus Ergebnissen anderer Berechnungen für Systeme, die mit dem zu berechnenden isostrukturell sind, aber andere Ionen enthalten.

6. Das Verfahren nach Anspruch 1, **dadurch gekennzeichnet, dass** alle Operationen, die zum Berechnen der Struktur der Zustände der ausgewählten Ionen in der definierten Umgebung in dem Kristallgitter führen, nach Auswählen (630) des Berechnungsraums aus dem Vektorraum, der über einen Körper reeller Zahlen aufgespannt ist, und dem Raum, der über einen Körper komplexer Zahlen aufgespannt ist, und Auswählen einer Basis zur Konstruktion der Hamilton-Matrix oder des Gesamtenergieoperators durchgeführt werden.

7. Das Verfahren nach Anspruch 6, **dadurch gekennzeichnet, dass** nach Auswählen (630) des Raums reeller Zahlen und Durchführen der Berechnungen in der Basis $|L,S,J,J_z>$, während einer Erzeugung der Matrix, die die Produkte der Matrixelemente der Stevens-Operatoren und der definierten Stevens-Koeffizienten ($B^m_n$, $O^m_n$) und der Operatoren der Richtungskomponenten (x, z) des Magnetfelds, zunächst eine leere Matrix (1430, 1530) erzeugt wird, deren Zeilen und Spalten mit Werten von $|J_Z>$ nummeriert sind, wobei die Matrix mit Produkten der Matrixelemente von Stevens-Operatoren und definierten Stevens-Koeffizienten ($B^m_n$, $O^m_n$) und Komponentenoperatoren (x, z) des Magnetfeldes gefüllt wird (1440, 1540), und nach Auswählen des Raums der reellen Zahlen und Durchführen der Berechnungen mit der Basis $|L, S, L_z, S_z>$, während der Erzeugung der Matrix, die die Produkte der Matrixelemente von Stevens-Operatoren und definierten Stevens-Koeffizienten ($B^m_n$, $O^m_n$) und Komponentenoperatoren (x, z) des Magnetfelds enthält, zunächst eine leere Matrix erzeugt (1410, 1510) wird, deren Zeilen und Spalten mit $|L_Z, S_z>$-Kombinationen nummeriert sind, die als anfänglich vorbereitete Hamilton-Matrix mit Komponenten von Stevens-Operatoren ($B^m_n$, $O^m_n$), Komponentenoperatoren (x, z) des Magnetfeldes und Komponenten des Spin-Orbit-Kopplungsoperators gefüllt (1420, 1520) wird.

**8.** Das Verfahren nach Anspruch 6, **dadurch gekennzeichnet, dass** nach Auswählen des Raums reeller Zahlen und komplexer Zahlen und Durchführen der Berechnungen in der Basis $|L,S,J,J_z>$, während einer Erzeugung der Matrix (1440, 1540), die Füllungen mit den Produkten der Matrixelemente der Stevens-Operatoren und der definierten Stevens-Koeffizienten ($B^m_n$, $O^m_n$) und Komponentenoperatoren (x, y, z) des Magnetfelds, zunächst eine leere Matrix (1430, 1530) erzeugt wird, deren Zeilen und Spalten mit Werten von $|J_Z>$ nummeriert sind, wobei die Matrix mit Produkten der Matrixelemente von Stevens-Operatoren und definierten Stevens-Koeffizienten ($B^m_n$, $O^m_n$), Gesamtmoment-Projektionsoperatoren und Komponentenoperatoren (x, y, z) des Magnetfeldes gefüllt wird (1440, 1540), und nach Auswählen des Raums der komplexen Zahlen und Durchführen der Berechnungen mit der Basis $|L, S, L_z, S_z>$, während der Erzeugung der Matrix, die die Produkte der Matrixelemente von Stevens-Operatoren und definierten Stevens-Koeffizienten ($B^m_n$, $O^m_n$) und Komponentenoperatoren (x, y, z) des Magnetfelds enthält, zunächst eine leere Matrix erzeugt (1410, 1510) wird, deren Zeilen und Spalten mit $|L_Z, S_z>$-Kombinationen nummeriert sind, die als Hamilton-Matrix mit Komponenten von Stevens-Operatoren ($B^m_n$, $O^m_n$), Projektionsoperatoren von magnetischen Spin- und Orbit-Gesamtmomentoperatoren, Komponentenoperatoren (x, y, z) des Magnetfeldes und Komponenten des Spin-Orbit-Kopplungsoperators gefüllt (1420, 1520) wird.

**9.** Das Verfahren nach Anspruch 7 oder 8, **dadurch gekennzeichnet, dass** nach Füllen (1420, 1440, 1520, 1540) mit den Produkten aus den Matrixelementen von Stevens-Operatoren und definierten Stevens-Koeffizienten ($B^m_n$, $O^m_n$), Projektionsoperatoren von magnetischen Spin- und Orbit-Gesamtmomentoperatoren, Komponentenoperatoren (x, z) oder (x, y, z) des Magnetfelds und gegebenenfalls mit Komponenten des Spin-Orbit-Kopplungsoperators eine Diagonalisierung der Hamilton-Matrix durchgeführt (1550) wird, und nach der Diagonalisierung (2100) der Hamilton-Matrix n-komplette Sätze von Paaren von Energie-Eigenwerten E' (i=1..n) und Eigenfunktionen, die lineare Kombinationen von Basisvektoren sind, berechnet (2200) werden, und als nächstes, basierend auf ihrer Form, die erwarteten Werte der Richtungskomponenten (x, z) oder (x, y, z) von magnetischen Momenten der einzelnen n Energie-Eigenzustände E' berechnet (2210, 2220) werden.

**10.** Das Verfahren nach Anspruch 9, **dadurch gekennzeichnet, dass** n Energie-Eigenzustände E' mit ihren erwarteten Werten der Richtungskomponenten von magnetischen Momenten $<m^i_j>$ (i=1..n, j=x,z oder j=x,y,z) der einzelnen Zustände sortiert werden (2300) und anschließend die Summe der Zustände Z(T) und die Population $N^i(T)$ von jedem Energiezustand der erlangten Struktur in definierten Temperaturinkrementen nach der Boltzmann-Statistik berechnet werden (2400), auf deren Basis die Verläufe der Temperaturabhängigkeiten der freien Energie, der inneren Energie, der Entropie, der magnetischen Suszeptibilität, berechnet für ein entlang der (x und z)- oder (x, y und z)-Richtung angelegtes Feld, und der spezifischen Schottky-Wärme zum Bestimmen der kalorimetrischen, elektronischen und magnetischen Eigenschaften eines ionenhaltigen Materials in der definierten Umgebung des Kristallfelds (CEF) berechnet (2500) werden.

**11.** Das Verfahren nach Anspruch 10, **dadurch gekennzeichnet, dass** ein neuer vollständiger Ergebnisdatensatz erzeugt (2700) wird, der die kalorimetrischen, elektronischen und magnetischen Eigenschaften eines ionenhaltigen Materials in der definierten Umgebung des Kristallfelds (CEF) zusammen mit einer interaktiven Visualisierung dieser Umgebung und der Berechnungsparameter umfasst, und der neue vollständige Ergebnisdatensatz in Form eines unabhängigen Datensatzes präsentiert wird, der direkt und parallel zu anderen Ergebnisdaten verfügbar ist, wodurch direkte Vergleiche der erlangten Ergebnisse ermöglicht werden.

**12.** Das Verfahren nach Anspruch 11, **dadurch gekennzeichnet, dass** verschiedene separate vollständige Sätze der Ergebnisdaten in einer einzigen zusammengeführten numerischen Form zusammen mit den Daten archiviert (2800) werden, die zu Berechnungen, Simulationen und Visualisierungen von jedem separaten Satz der Ergebnisdaten gehören, und die numerische Form der Ergebnisdaten den Zugriff auf eine ausgewählte Eigenschaft oder einen Verlauf der Temperaturabhängigkeit einer ausgewählten Eigenschaft aus verschiedenen vollständigen Sätzen der Ergebnisdaten gleichzeitig ermöglicht.

**13.** Das Verfahren nach Anspruch 12, **dadurch gekennzeichnet, dass** die Form der Ergebnisdaten eine Implementierung der gespeicherten Ergebnisdaten und einen Vergleich mit adäquaten aktuellen Berechnungen ermöglicht.

**14.** Ein System zum Auffinden von Materialien, die definierte Eigenschaften aufweisen, wobei das System geeignet ist, eines der Verfahren nach einem der Ansprüche 1 bis 13 auszuführen, das System umfassend
eine Recheneinheit (10) mit einem Prozessor (20),
eine Vorrichtung (95) zur Darstellung von Daten und Berechnungsergebnissen und mit Zugang zu Daten über Material,
eine Testeinheit (15), die angepasst ist, um Tests über reelle Materialien durchzuführen und mit der Recheneinheit (10) zu kommunizieren,
**dadurch gekennzeichnet, dass** der Prozessor (20) Folgendes umfasst
ein Modul (30) zum Auffinden und Auswählen von Elementen des gewählten Materials, das eine Bestimmung ihrer Elektronenkonfiguration basierend aus Werten der Quantenzahlen magnetischen Orbitalmoments L, des magnetischen Spinmoments S, wenn eine Basis $|L,S,L_Z,S_Z>$ gewählt wird, oder basierend auf Werten des magnetischen Gesamtmoments J, wenn eine Basis $|L,S,J,J_Z>$ gewählt wird, ermöglicht,
ein Modul (40) zum Auffinden eines vollständigen Satzes von Koeffizienten des elektrischen Kristallfelds (CEF), definiert durch Stevens-Koeffizienten mit der Form von $B^m{}_n$,
ein Modul (50) zum Vorbereiten von Daten für die Berechnungen mit einem Modul (51) zum Auswählen der Rechentechnik und einem Modul (52) zum Bestimmen der Eingangsdaten, des Raums und der Basis der Berechnungen, wobei das Modul (50) mit dem Modul (40) kommuniziert,
ein Modul (60) zum Aufbau eines Modells eines idealen Materials, das definierte Ionen enthält und dazu geeignet ist, mit dem Modul (40) zu kommunizieren, um einen vollständigen Satz von Koeffizienten des elektrischen Kristallfelds (CEF) zu finden, wobei die Ionen räumlich identisch ausgerichtet sind und nicht miteinander interagieren, sondern mit äußeren Feldern interagieren, mit einer berechneten Struktur von Energiezuständen zusammen mit ihren spektralen Eigenschaften, und einer klassischen Boltzmann-Statistik unterzogen wurden, und ausgerichtete (x, y, z) oder (x, z) Komponenten von magnetischen Eigenschaften berechnet werden, wobei das Modul (60) zum Aufbau eines Modells des idealen Materials mit der Testeinheit (15) verbunden ist, um das Modell des idealen Materials mit einem reellen Material zu überprüfen,
ein Modul (80) zum Vergleich des idealen Materials mit dem reellen Material, um das Modell des idealen Materials mit dem reellen Material zu überprüfen, wenn die aus den Berechnungen erlangten Eigenschaften des Materials den Eigenschaften des gesuchten Materials entsprechen.

**15.** Das System nach Anspruch 14, **dadurch gekennzeichnet, dass** das Modul (60) zum Aufbau des Modells des idealen Materials ein Modul (64) zur Berechnung vollständiger Sätze von Paaren von Energieeigenwerten E' (i=1..n) und Eigenfunktionen, die lineare Kombinationen von Basisvektoren sind, und ein Modul (68) zur Berechnung von Verläufen von Temperaturabhängigkeiten der freien Energie, der inneren Energie, der Entropie, der magnetischen Suszeptibilität, berechnet für ein entlang der Richtungen (x und z) oder (x, y und z) angelegtes Feld, und der spezifischen Schottky-Wärme umfasst, um kalorimetrische, elektronische und magnetische Eigenschaften eines ionenhaltigen Materials in definierter Umgebung des Kristallfelds (CEF) zu bestimmen.

## Revendications

**1.** Un procédé mis en œuvre par ordinateur destiné à trouver des matériaux ayant des propriétés définies, pendant lequel un matériau contenant des ions d'au moins un élément composant du Tableau de Mendeleïev avec des couches électroniques non fermées est choisi sur la base d'informations disponibles dans l'état de la technique, le procédé comprenant les étapes suivantes:

la définition d'au moins un type d'ions étant la base de calculs et de simulations de matériaux en choisissant (200) au moins un élément composant d'un matériau choisi directement à partir du Tableau de Mendeleïev,
la détermination (300) d'un état d'oxydation des atomes de l'élément composant étant sélectionné dans le matériau choisi pour définir leur configuration électronique,
pour l'au moins un type d'ions sélectionné de l'élément composant, après la détermination de valeurs de nombres quantiques d'un moment magnétique orbital L, la réponse (500) à une question pour déterminer si des coefficients de champ électrique cristallin (CEC) sont connus et, dans le cas où des coefficients de champ électrique cristallin (CEC) sont connus, l'entrée (560) des coefficients de champ électrique cristallin (CEC) définis par des coefficients de Stevens ayant une forme de $B^m{}_n$, ou dans le cas où des coefficients de champ électrique cristallin (CEC) ne sont pas connus,

après la sélection (510) d'un procédé pour trouver des valeurs de coefficients de champ électrique cristallin (CEC), la réalisation (520, 530, 540) de calculs de coefficients de Stevens avec la forme $B^m_n$ et l'entrée (560) de coefficients de champ électrique cristallin (CEC) définis par des coefficients de Stevens ayant la forme de $B^m_n$, le commencement (600) de calculs avec des paramètres prédéfinis ou, après la sélection (610) d'un procédé de calcul, la réalisation de calculs d'un moment magnétique de spin S et d'un moment magnétique orbital L quand une base |L, S, $L_z$, $S_z$ > est choisie selon un espace de calcul choisi (640) ou d'un moment magnétique total J correspondant à un état fondamental des ions sélectionnés quand une base |L, S, J, $J_z$ > est choisie pour trouver un ensemble complet de coefficients de champ électrique cristallin (CEC), définis par des coefficients de Stevens ayant la forme de $B^m_n$, exprimés en unités d'énergie et définissant un environnement de charge immédiat des ions sélectionnés dans un réseau cristallin par le calcul de coefficients de Stevens ayant la forme de $B^m_n$,

sur la base des coefficients de Stevens ayant la forme de $B^m_n$, la génération d'un opérateur d'énergie totale (1410, 1420, 1430, 1440, 1510, 1520, 1530, 1540), dénommé matrice hamiltonienne contenant des composantes de matrice étant des éléments d'opérateurs de Stevens multipliés par les coefficients de Stevens définis $B^m_n$ ($H_{CEC}=\sum B^m_n O^m_n$), (x, y, z) ou (x, z) composantes d'opérateurs de potentiel de champ magnétique, des opérateurs de projection de moment magnétique orbital, de moment magnétique de spin et de moment magnétique total, et des composantes d'opérateurs du couplage spin-orbite,

après la réalisation d'opérations sur la matrice, la création d'un modèle d'un matériau idéal contenant les ions sélectionnés, les ions sélectionnés étant orientés dans l'espace de manière identique et n'interagissant pas les uns avec les autres, mais interagissant avec des champs magnétiques et électriques externes, avec une structure calculée d'états d'énergie conjointement avec leur propriétés spectrales, et étant soumis à la statistique de Boltzmann classique, et ayant les composantes directionnelles (x, y, z) ou (x, z) de propriétés magnétiques calculées,

sur la base du modèle du matériau idéal, la définition de propriétés calorimétriques, électroniques et magnétiques sous la forme de dépendances à la température d'un matériau contenant des ions dans l'environnement défini du champ électrique cristallin (CEC), et

la vérification des propriétés du matériau idéal avec les propriétés d'un matériau réel, pour déterminer si les propriétés du matériau obtenu à partir des calculs correspondent aux propriétés du matériau recherché.

**2.** Le procédé selon la revendication 1, dans lequel la valeur des nombres quantiques du moment magnétique orbital L, du moment magnétique de spin S et facultativement du moment magnétique total J correspondant à l'état fondamental de la configuration électronique des ions sélectionnés est déterminée sur la base des règles de Hund.

**3.** Le procédé selon la revendication 1, dans lequel le calcul de coefficients de Stevens avec la forme de $B^m_n$ est réalisé après avoir choisi (610) l'un des procédés de calcul et déterminé (630) un espace de calcul, choisi (640) une base pour les calculs et déterminé (650) des valeurs de constantes.

**4.** Le procédé selon la revendication 3, dans lequel les calculs de coefficients de Stevens avec la forme de $B^m_n$ sont réalisés en utilisant un modèle de charge ponctuelle (520) (MCP) ou en utilisant une visualisation tridimensionnelle interactive (3D) (530) de multipôles de composante du champ électrique et de leurs superpositions définies comme un champ cristallin (CEC) ou par une conversion (540) de coefficients de CEC ($A^m$n -> $B^m_n$) à partir de résultats connus d'autres calculs pour des systèmes isostructuraux avec celui calculé, mais contenant d'autres ions.

**5.** Le procédé selon la revendication 4, dans lequel les résultats des calculs des coefficients de Stevens avec la forme de $B^m_n$ sont harmonisés en comparant les résultats obtenus en utilisant l'approximation de modèle de charge ponctuelle (MCP) (520) ou la visualisation interactive (530) du champ cristallin (CEC) en 3D, ou par la conversion (540) des coefficients de champ cristallin (CEC) ($A^m$n -> $B^m_n$) à partir des résultats d'autres calculs pour des systèmes isostructuraux avec celui calculé, mais contenant d'autres ions.

**6.** Le procédé selon la revendication 1, dans lequel toutes les opérations menant au calcul de la structure des états des ions sélectionnés dans l'environnement défini dans le réseau cristallin sont réalisées après avoir choisi (630) l'espace de calcul à partir de l'espace vectoriel étendu sur un corps de nombres réels et un espace étendu sur un corps de nombres complexes, et choisi une base pour la construction de la matrice hamiltonienne ou de l'opérateur d'énergie totale.

**7.** Le procédé selon la revendication 6, dans lequel après avoir choisi (630) l'espace de nombres réels et réalisé les calculs dans une base |L, S, J, $J_z$ >, tout en générant la matrice contenant les produits des éléments de matrice d'opérateurs de Stevens et de coefficients de Stevens définis ($B^m_n$, $O^m_n$) et des opérateurs de composantes directionnelles (x, z) du champ magnétique, en premier lieu, une matrice vide (1430, 1530) est créée avec des rangs et des colonnes numérotés avec des valeurs de $|J_z>$, la matrice étant remplie (1440, 1540) de produits des éléments de matrice d'opérateurs de Stevens et de coefficients de Stevens définis ($B^m_n$, $O^m_n$) et des opérateurs de composantes (x, z) du champ magnétique, et après avoir choisi l'espace de nombres réels et réalisé les calculs avec une base $|L, S, L_z, S_z>$, tout en générant la matrice contenant les produits des éléments de matrice d'opérateurs de Stevens et de coefficients de Stevens définis ($B^m_n$, $O^m_n$) et des opérateurs de composante (x, z) du champ magnétique, en premier lieu, une matrice vide est créée (1410, 1510) avec des rangs et des colonnes numérotés avec des combinaisons $|L_z, S_z>$, qui, comme une matrice hamiltonienne préparée initialement, est remplie (1420, 1520) avec des composantes d'opérateurs de Stevens ($B^m_n$, $O^m_n$), des opérateurs de composante (x, z) du champ magnétique et des composantes de l'opérateur de couplage spin-orbite.

**8.** Le procédé selon la revendication 6, dans lequel après avoir choisi l'espace de nombres réels et de nombres complexes, et réalisé les calculs dans la base $|L, S, J, J_z>$, tout en générant la matrice (1440, 1540) contenant des remplissages avec les produits des éléments de matrice d'opérateurs de Stevens et de coefficients de Stevens définis ($B^m_n$, $O^m_n$), et des opérateurs de composante (x, y, z) du champ magnétique, en premier lieu, une matrice vide (1430, 1530) est créée avec des rangs et des colonnes numérotés avec des valeurs de $|J_z>$, la matrice étant remplie (1440, 1540) avec des produits des éléments de matrice d'opérateurs de Stevens et de coefficients de Stevens définis ($B^m_n$, $O^m_n$), des opérateurs de projection de moment total et des opérateurs de composante (x, y, z) du champ magnétique, et après avoir choisi l'espace de nombres complexes et réalisé les calculs avec la base $|L, S, L_z, S_z>$, tout en générant la matrice contenant les produits des éléments de matrice d'opérateurs de Stevens et de coefficients de Stevens ($B^m_n$, $O^m_n$) et des opérateurs de composante (x, y, z) définis du champ magnétique, en premier lieu, une matrice vide est créée (1410, 1510) avec des rangs et des colonnes numérotés avec des combinaisons $|L_z, S_z,>$, qui, comme une matrice hamiltonienne, est remplie (1420, 1520) avec des composantes d'opérateurs de Stevens ($B^m_n$, $O^m_n$), des opérateurs de projection de moments magnétiques de spin et d'orbite totaux, des opérateurs de composantes (x, y, z) du champ magnétique et des composantes des opérateurs de couplage spin-orbite.

**9.** Le procédé selon la revendication 7 ou 8, dans lequel après le remplissage (1420, 1440, 1520, 1540) avec les produits des éléments de matrice d'opérateurs de Stevens et de coefficients de Stevens définis ($B^m_n$, $O^m_n$), des opérateurs de projection de moments magnétiques de spin et d'orbite totaux, des opérateurs de composante (x, z) ou (x, y, z) du champ magnétique et facultativement avec des composantes de l'opérateur de couplage spin-orbite, la diagonalisation de la matrice hamiltonienne est réalisée (1550), et après la diagonalisation (2100) de la matrice hamiltonienne, n ensembles complets de paires de valeurs propres d'énergie E' (i=1..n) et de fonctions propres étant des combinaisons linéaires de vecteurs de base sont calculés (2200), et puis, sur la base de leur forme, les valeurs attendues des composantes directionnelles (x, z) ou (x, y, z) de moments magnétiques des n états propres d'énergie E' individuels sont calculées (2210, 2220).

**10.** Le procédé selon la revendication 9, dans lequel les n états propres d'énergie E' sont triées (2300) avec leurs valeurs attendues de composantes directionnelles de moments magnétiques $< m^i_j>$ (i = 1..n, j = x, z ou j = x, y, z) des états individuels, et puis, les sommes des états Z(T) et de la population $N^i(T)$ de chaque état d'énergie de la structure obtenue sont calculées (2400) dans des paliers de température définis selon la statistique de Boltzmann, sur la base de quoi les cours des dépendances à la température de l'énergie libre, de l'énergie interne, de l'entropie, de la susceptibilité magnétique, calculés pour un champ appliqué le long des directions (x et z) ou (x, y et z), et la chaleur spécifique de Schottky afin de déterminer les propriétés calorimétriques, électroniques et magnétiques d'un matériau contenant des ions dans l'environnement défini du champ cristallin (CEC) sont calculés (2500).

**11.** Le procédé selon la revendication 10, dans lequel un nouvel ensemble complet de données de résultat est créé (2700), comprenant les propriétés calorimétriques, électroniques et magnétiques d'un matériau contenant des ions dans l'environnement défini du champ cristallin (CEC) conjointement avec une visualisation interactive de cet environnement et des paramètres de calcul, et le nouvel ensemble complet de données de résultats est présenté sous la forme d'un ensemble indépendant de données disponibles directement et en parallèle avec d'autres données de résultat, permettant des comparaisons directes des résultats obtenus.

**12.** Le procédé selon la revendication 11, dans lequel différents ensembles complets distincts des données de résultats sont archivés (2800) sous une forme numérique fusionnée unique conjointement avec les données se rapportant aux calculs, simulations et visualisations de chaque ensemble distinct des données de résultat, et la forme numérique des données de résultat permet l'accès à une propriété choisie ou à un cours de la dépendance à la température d'une propriété choisie à partir d'ensembles complets différents des données de résultat simultanément.

**13.** Le procédé selon la revendication 12, dans lequel la forme des données de résultat permet la mise en œuvre des données de résultat sauvegardées et la comparaison avec des calculs courants adéquats.

**14.** Un système pour trouver des matériaux ayant des propriétés définies, le système conçu pour réaliser l'un quelconque des procédés des revendications 1 à 13, le système comprenant
une unité de calcul (10) avec un processeur (20),
un dispositif (95) pour la présentation de données et de résultats de calcul et avec l'accès à des données sur des matériaux,
une unité d'analyse (15) conçue pour réaliser des analyses sur des matériaux réels et communiquant avec l'unité de calcul (10),
**caractérisé en ce que** le processeur (20) comprend
un module (30) pour trouver et sélectionner des éléments du matériau choisi, permettre la détermination de leur configuration électronique sur la base de valeurs de nombres quantiques de moment magnétique orbital L, de moment magnétique de spin S quand une base $|L, S, L_z, S_z >$ est choisie ou sur la base de valeurs de moment magnétique total J quand une base $|L, S, J, J_z >$ est choisie,
un module (40) pour trouver un ensemble complet de coefficients de champ électrique cristallin (CEC), défini par des coefficients de Stevens avec la forme de $B^m{}_n$,
un module (50) pour la préparation de données pour les calculs avec un module (51) pour la sélection d'une technique de calcul et un module (52) pour la détermination de données d'entrée, de l'espace et de la base des calculs, le module (50) communiquant avec le module (40),
un module (60) pour la construction d'un modèle d'un matériau idéal contenant des ions définis et conçu pour communiquer avec le module (40) pour trouver un ensemble complet de coefficients de champ électrique cristallin (CEC), les ions étant orientés dans l'espace de manière identique et n'interagissant pas les uns avec les autres, mais interagissant avec des champs externes, avec une structure calculée d'états d'énergie conjointement avec leurs propriétés spectrales, et étant soumis à la statistique de Boltzmann classique, et ayant des composantes directionnelles (x, y, z) ou (x, z) de propriétés magnétiques calculées, le module (60) pour la construction d'un modèle du matériau idéal étant connecté avec l'unité d'analyse (15) afin de vérifier le modèle du matériau idéal avec un matériau réel,
un module (80) pour la comparaison du matériau idéal avec le matériau réel afin de vérifier le modèle du matériau idéal avec le matériau réel, quand les propriétés du matériau obtenu d'après les calculs correspondent aux propriétés du matériau recherché.

**15.** Le système selon la revendication 14, dans lequel le module (60) pour la construction du modèle du matériau idéal comprend un module (64) pour le calcul d'ensembles complets de paires de valeurs propres d'énergie E' (i=1..n) et de fonctions propres étant des combinaisons linéaires de vecteurs de base, et un module (68) pour le calcul de cours de dépendances à la température d'énergie libre, d'énergie interne, d'entropie, de susceptibilité magnétique, calculés pour un champ appliqué le long de directions (x et z) ou (x, y et z), et de la chaleur spécifique de Schottky afin de déterminer les propriétés calorimétriques, électroniques et magnétiques d'un matériau contenant des ions dans un environnement défini du champ cristallin (CEC).

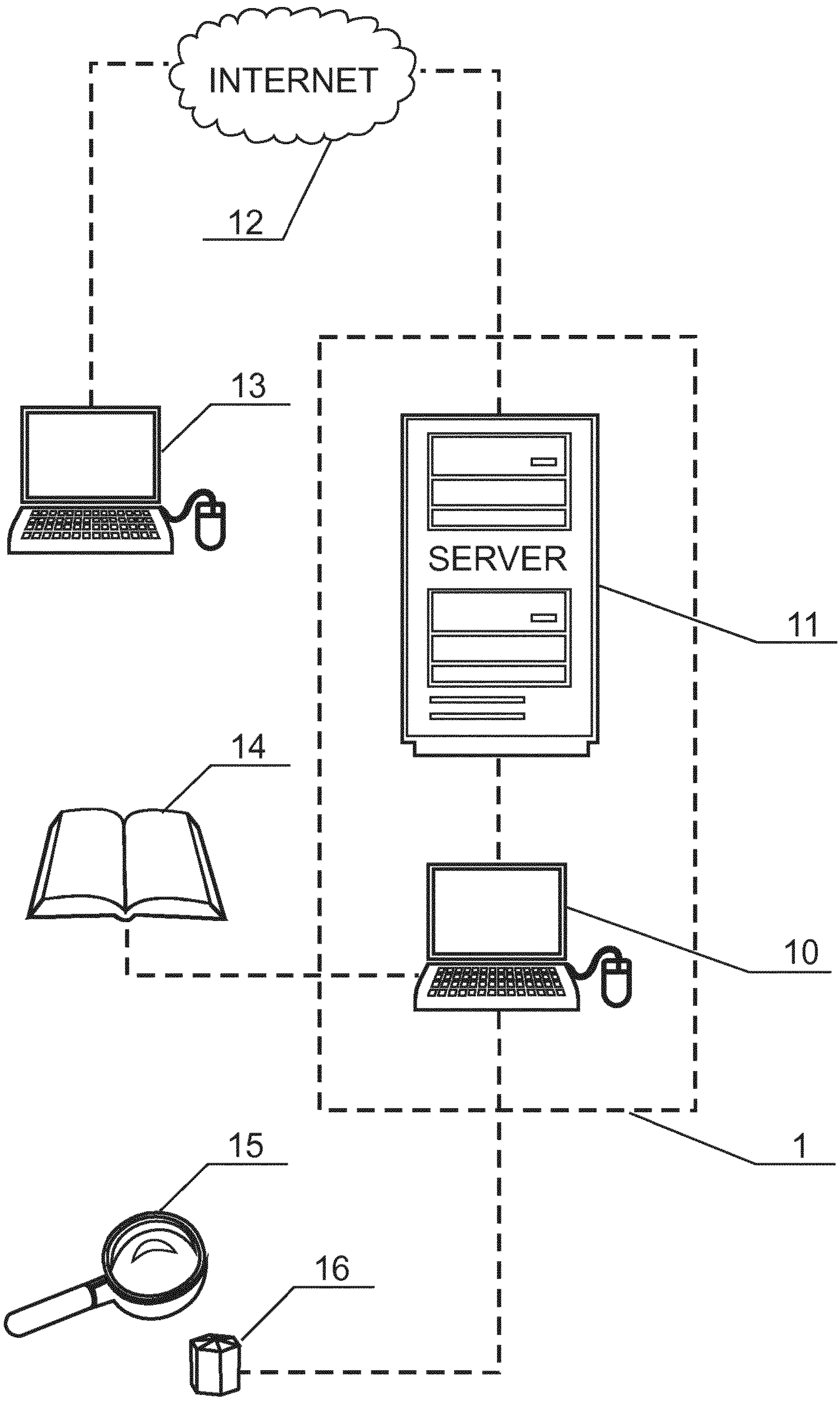


Fig. 1

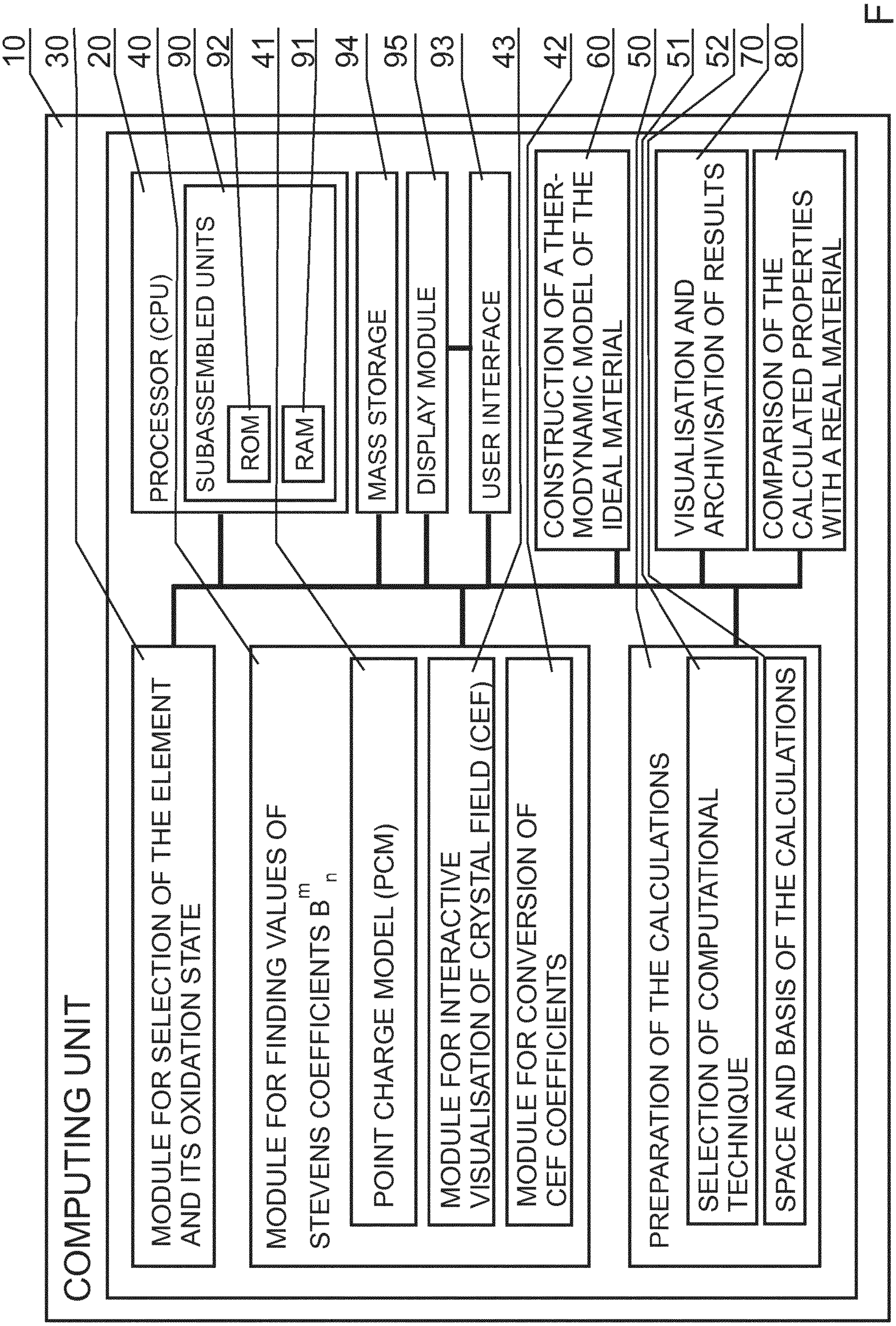
COMPUTING UNIT
MODULE FOR SELECTION OF THE ELEMENT AND ITS OXIDATION STATE
MODULE FOR FINDING VALUES OF STEVENS COEFFICIENTS $B_n^m$
POINT CHARGE MODEL (PCM)
MODULE FOR INTERACTIVE VISUALISATION OF CRYSTAL FIELD (CEF)
MODULE FOR CONVERSION OF CEF COEFFICIENTS
PREPARATION OF THE CALCULATIONS
SELECTION OF COMPUTATIONAL TECHNIQUE
SPACE AND BASIS OF THE CALCULATIONS
PROCESSOR (CPU)
SUBASSEMBLED UNITS
ROM
RAM
MASS STORAGE
DISPLAY MODULE
USER INTERFACE
CONSTRUCTION OF A THERMODYNAMIC MODEL OF THE IDEAL MATERIAL
VISUALISATION AND ARCHIVISATION OF RESULTS
COMPARISON OF THE CALCULATED PROPERTIES WITH A REAL MATERIAL
10
30
20
40
90
92
41
91
94
95
93
43
42
60
50
51
52
70
80

Fig. 2

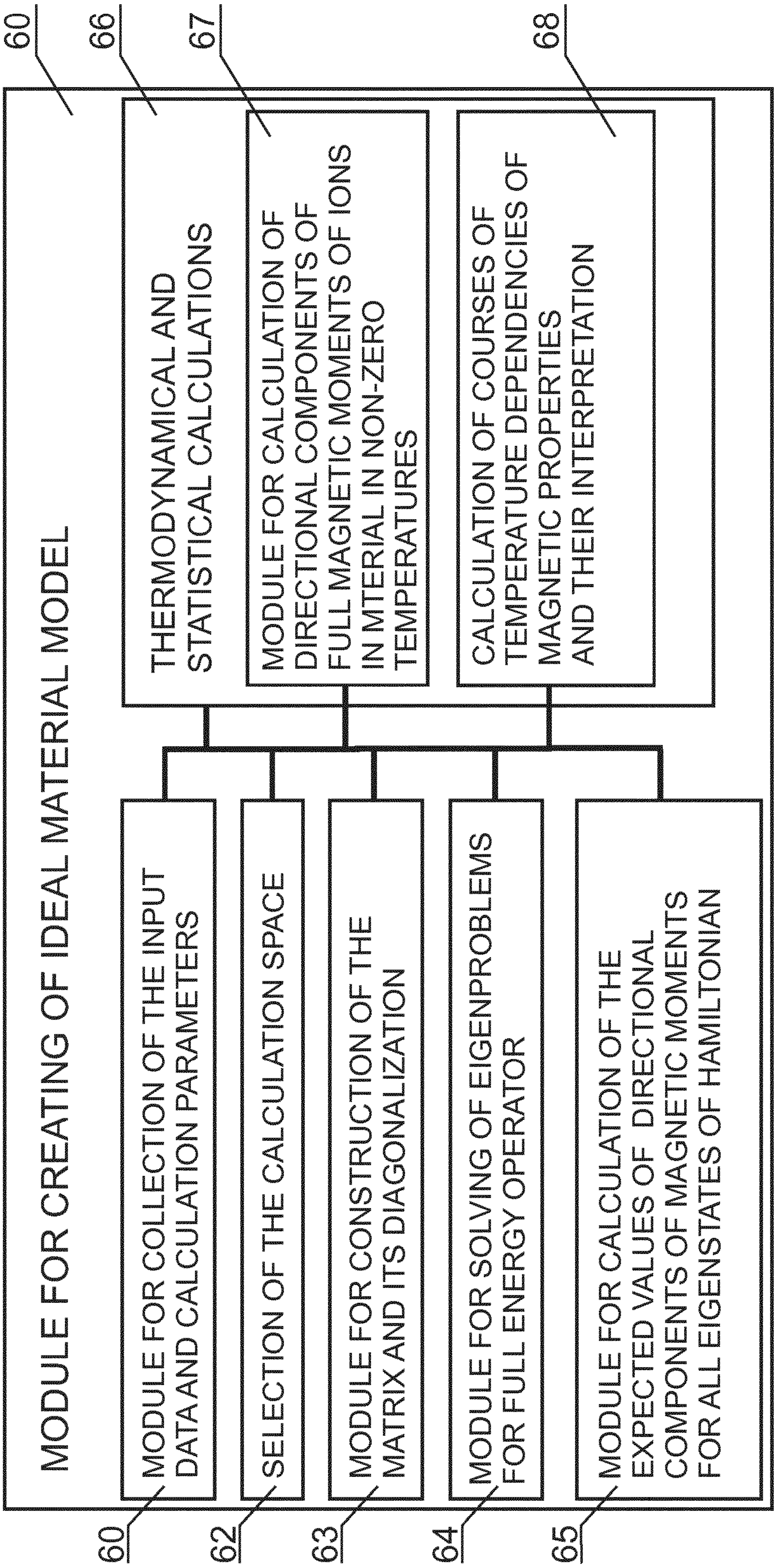
MODULE FOR CREATING OF IDEAL MATERIAL MODEL
MODULE FOR COLLECTION OF THE INPUT DATA AND CALCULATION PARAMETERS
SELECTION OF THE CALCULATION SPACE
MODULE FOR CONSTRUCTION OF THE MATRIX AND ITS DIAGONALIZATION
MODULE FOR SOLVING OF EIGENPROBLEMS FOR FULL ENERGY OPERATOR
MODULE FOR CALCULATION OF THE EXPECTED VALUES OF DIRECTIONAL COMPONENTS OF MAGNETIC MOMENTS FOR ALL EIGENSTATES OF HAMILTONIAN
THERMODYNAMICAL AND STATISTICAL CALCULATIONS
MODULE FOR CALCULATION OF DIRECTIONAL COMPONENTS OF FULL MAGNETIC MOMENTS OF IONS IN MTERIAL IN NON-ZERO TEMPERATURES
CALCULATION OF COURSES OF TEMPERATURE DEPENDENCIES OF MAGNETIC PROPERTIES AND THEIR INTERPRETATION
60
66
67
68
60
62
63
64
65

Fig. 3

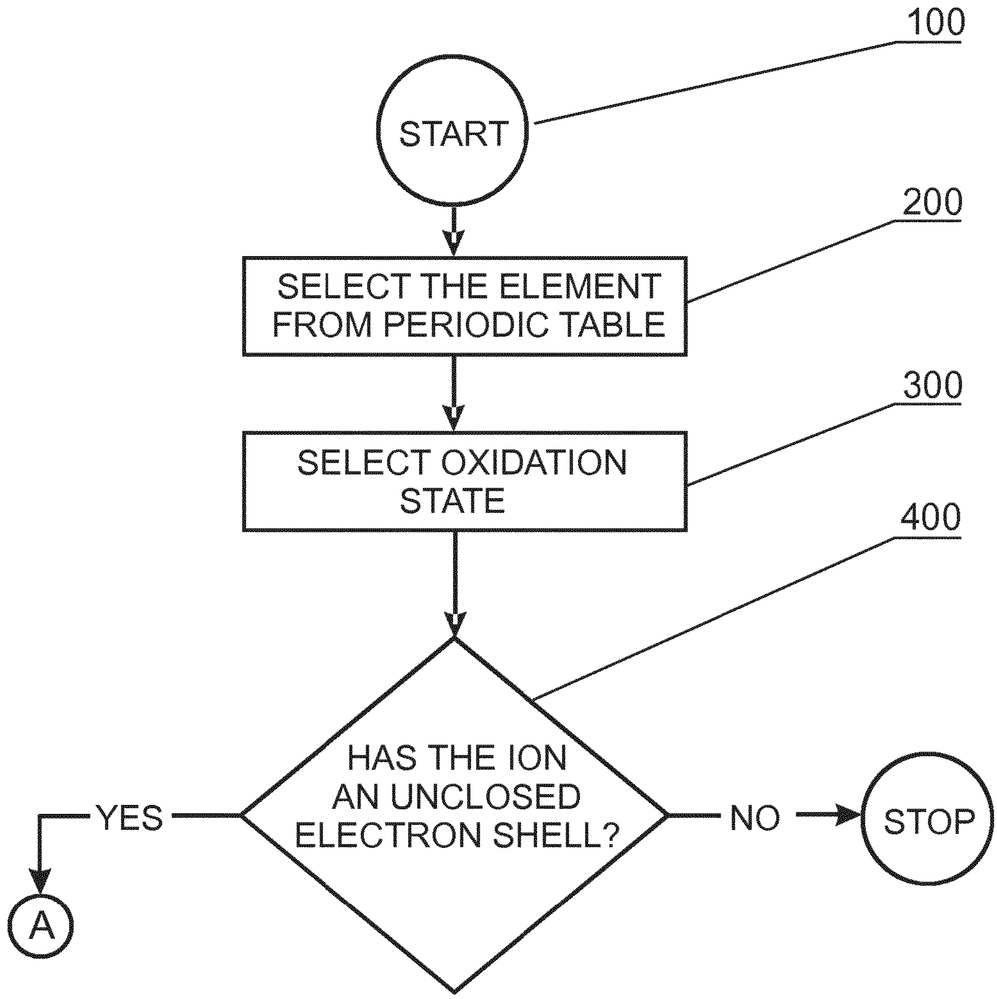
100
START
200
SELECT THE ELEMENT FROM PERIODIC TABLE
300
SELECT OXIDATION STATE
400
HAS THE ION AN UNCLOSED ELECTRON SHELL?
YES
A
NO
STOP

Fig. 4A

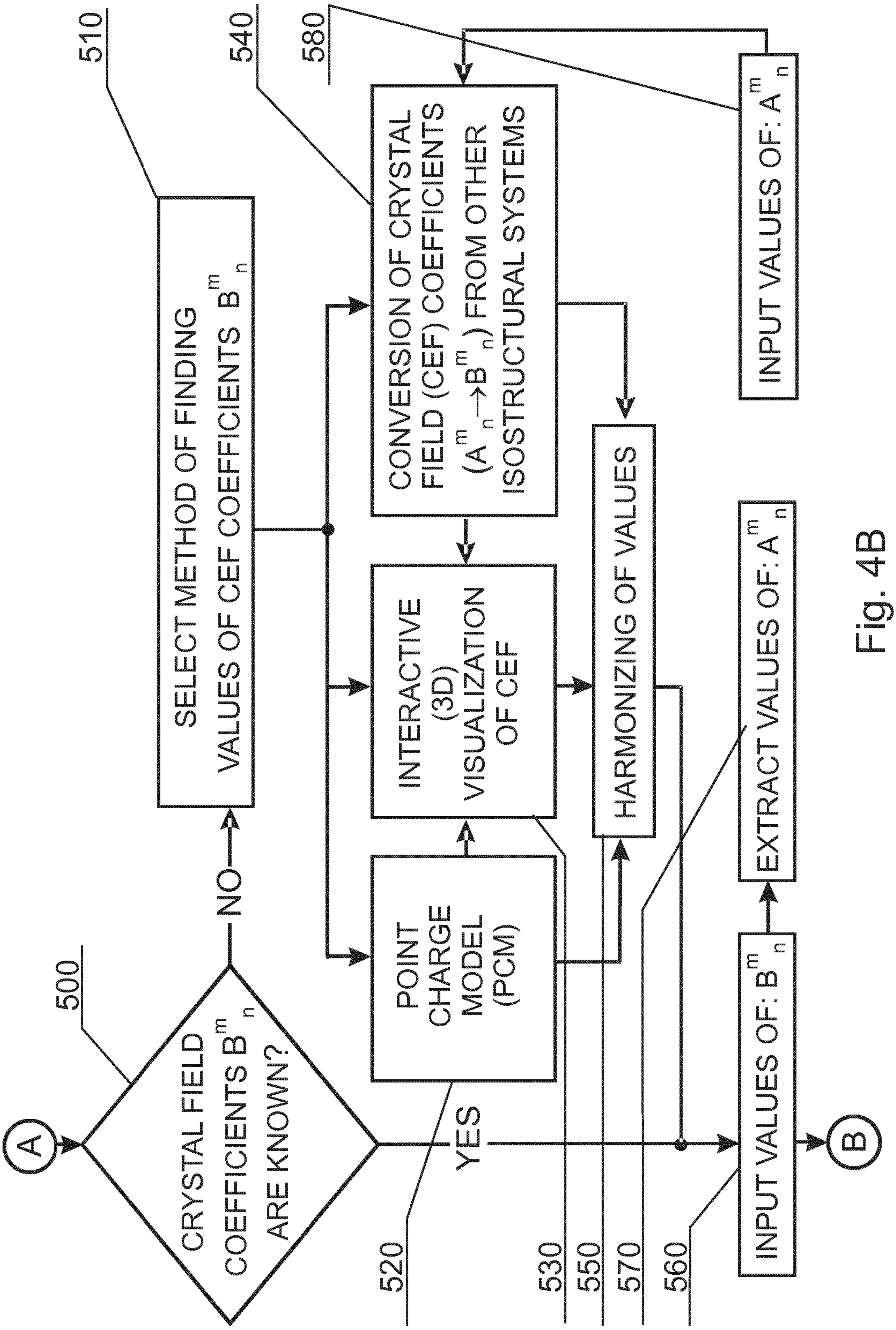
A
CRYSTAL FIELD COEFFICIENTS $B^m_n$ ARE KNOWN?
500
NO
YES
SELECT METHOD OF FINDING VALUES OF CEF COEFFICIENTS $B^m_n$
510
POINT CHARGE MODEL (PCM)
520
INTERACTIVE (3D) VISUALIZATION OF CEF
530
CONVERSION OF CRYSTAL FIELD (CEF) COEFFICIENTS ($A^m_n \rightarrow B^m_n$) FROM OTHER ISOSTRUCTURAL SYSTEMS
540
HARMONIZING OF VALUES
550
INPUT VALUES OF: $B^m_n$
560
EXTRACT VALUES OF: $A^m_n$
570
INPUT VALUES OF: $A^m_n$
580
B

Fig. 4B

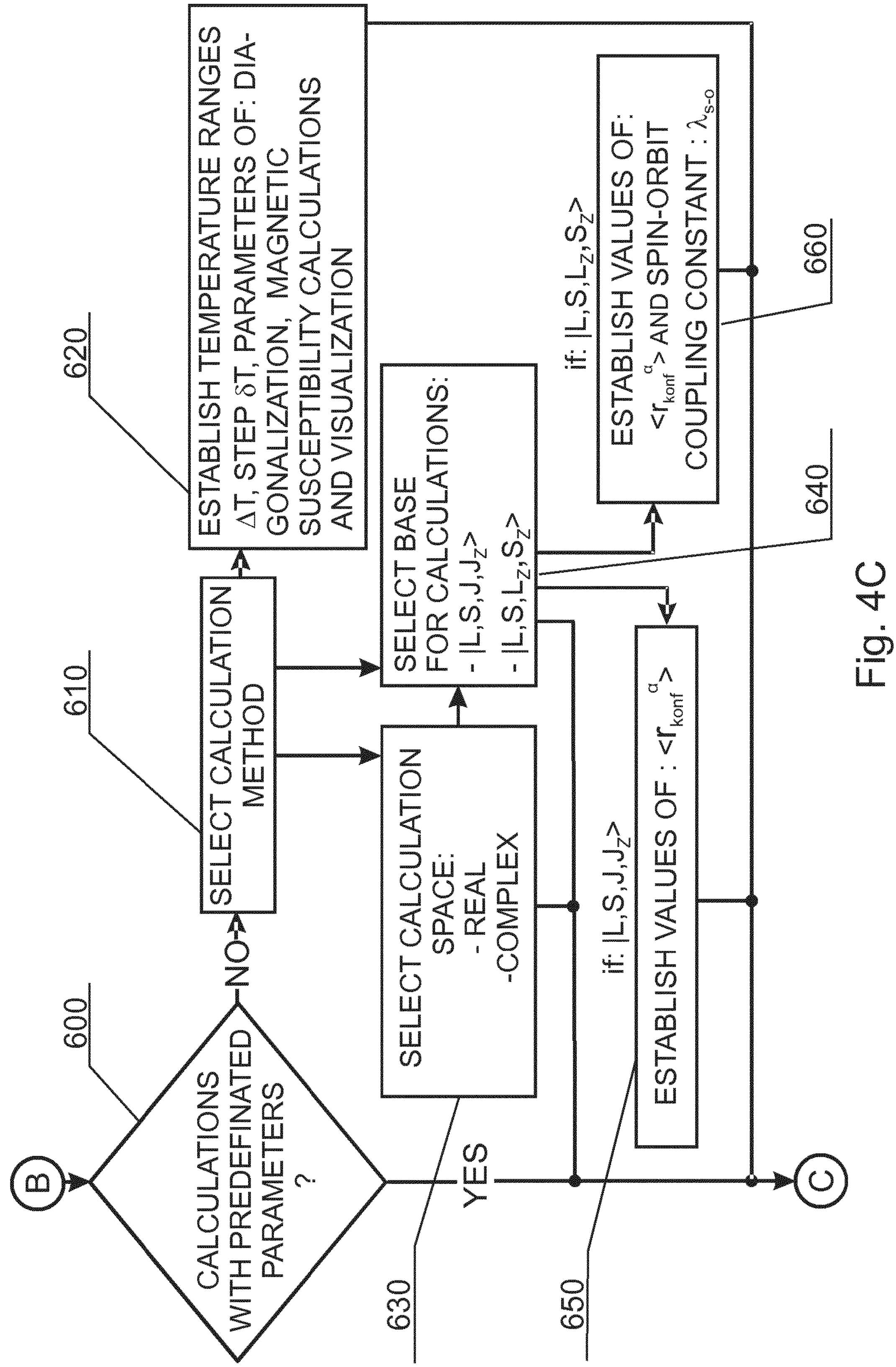
B
CALCULATIONS WITH PREDEFINATED PARAMETERS ?
600
NO
YES
SELECT CALCULATION METHOD
610
ESTABLISH TEMPERATURE RANGES ΔT, STEP δT, PARAMETERS OF: DIA-GONALIZATION, MAGNETIC SUSCEPTIBILITY CALCULATIONS AND VISUALIZATION
620
SELECT CALCULATION SPACE: - REAL -COMPLEX
630
SELECT BASE FOR CALCULATIONS: - |L,S,J,J_Z> - |L,S,L_Z,S_Z>
640
if: |L,S,J,J_Z>
ESTABLISH VALUES OF : <r_konf^α>
650
if: |L,S,L_Z,S_Z>
ESTABLISH VALUES OF: <r_konf^α> AND SPIN-ORBIT COUPLING CONSTANT : λ_s-o
660
C

Fig. 4C

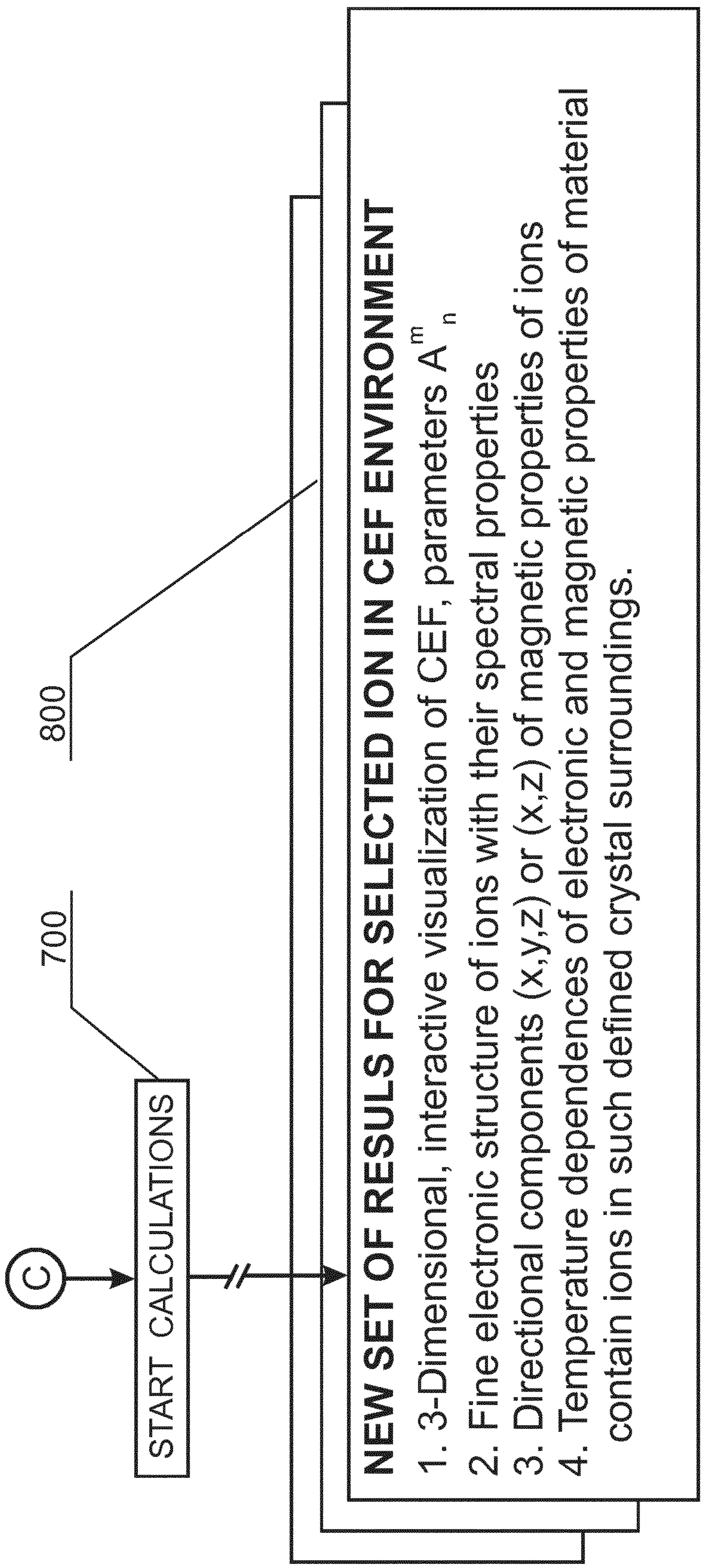
C
START CALCULATIONS
700
800
NEW SET OF RESULS FOR SELECTED ION IN CEF ENVIRONMENT
1. 3-Dimensional, interactive visualization of CEF, parameters $A^m_n$
2. Fine electronic structure of ions with their spectral properties
3. Directional components (x,y,z) or (x,z) of magnetic properties of ions
4. Temperature dependences of electronic and magnetic properties of material contain ions in such defined crystal surroundings.

Fig. 4D

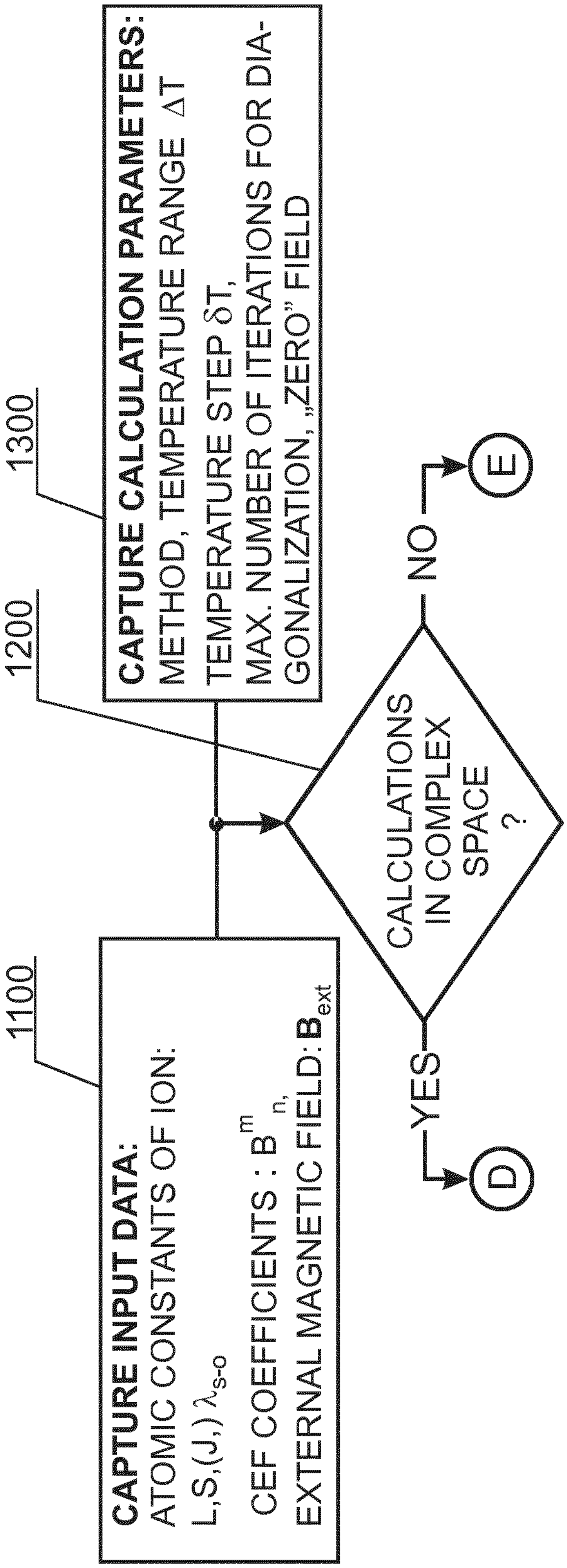
1100
CAPTURE INPUT DATA:
ATOMIC CONSTANTS OF ION:
L,S,(J,) $\lambda_{s-o}$
CEF COEFFICIENTS : $B^m_n$,
EXTERNAL MAGNETIC FIELD: $\mathbf{B}_{ext}$
1200
1300
CAPTURE CALCULATION PARAMETERS:
METHOD, TEMPERATURE RANGE $\Delta$T
TEMPERATURE STEP $\delta$T,
MAX. NUMBER OF ITERATIONS FOR DIAGONALIZATION, „ZERO" FIELD
CALCULATIONS IN COMPLEX SPACE ?
YES
NO
D
E

Fig. 5A

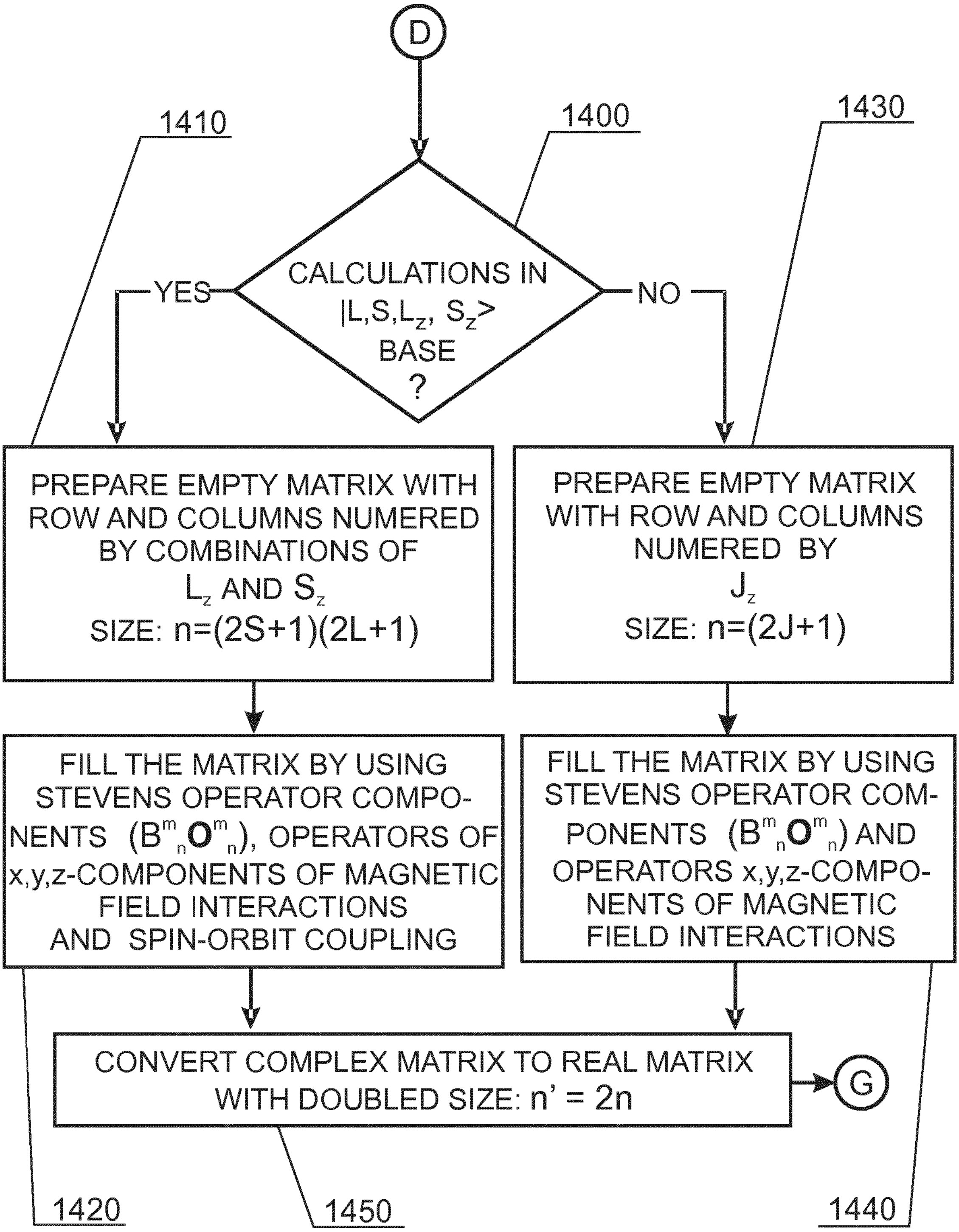
D
1410
1400
1430
CALCULATIONS IN |L,S,L_z, S_z> BASE ?
YES
NO
PREPARE EMPTY MATRIX WITH ROW AND COLUMNS NUMERED BY COMBINATIONS OF L_z AND S_z SIZE: n=(2S+1)(2L+1)
PREPARE EMPTY MATRIX WITH ROW AND COLUMNS NUMERED BY J_z SIZE: n=(2J+1)
FILL THE MATRIX BY USING STEVENS OPERATOR COMPONENTS (B^m_n O^m_n), OPERATORS OF x,y,z-COMPONENTS OF MAGNETIC FIELD INTERACTIONS AND SPIN-ORBIT COUPLING
FILL THE MATRIX BY USING STEVENS OPERATOR COMPONENTS (B^m_n O^m_n) AND OPERATORS x,y,z-COMPONENTS OF MAGNETIC FIELD INTERACTIONS
CONVERT COMPLEX MATRIX TO REAL MATRIX WITH DOUBLED SIZE: n' = 2n
G
1420
1450
1440

Fig. 5B

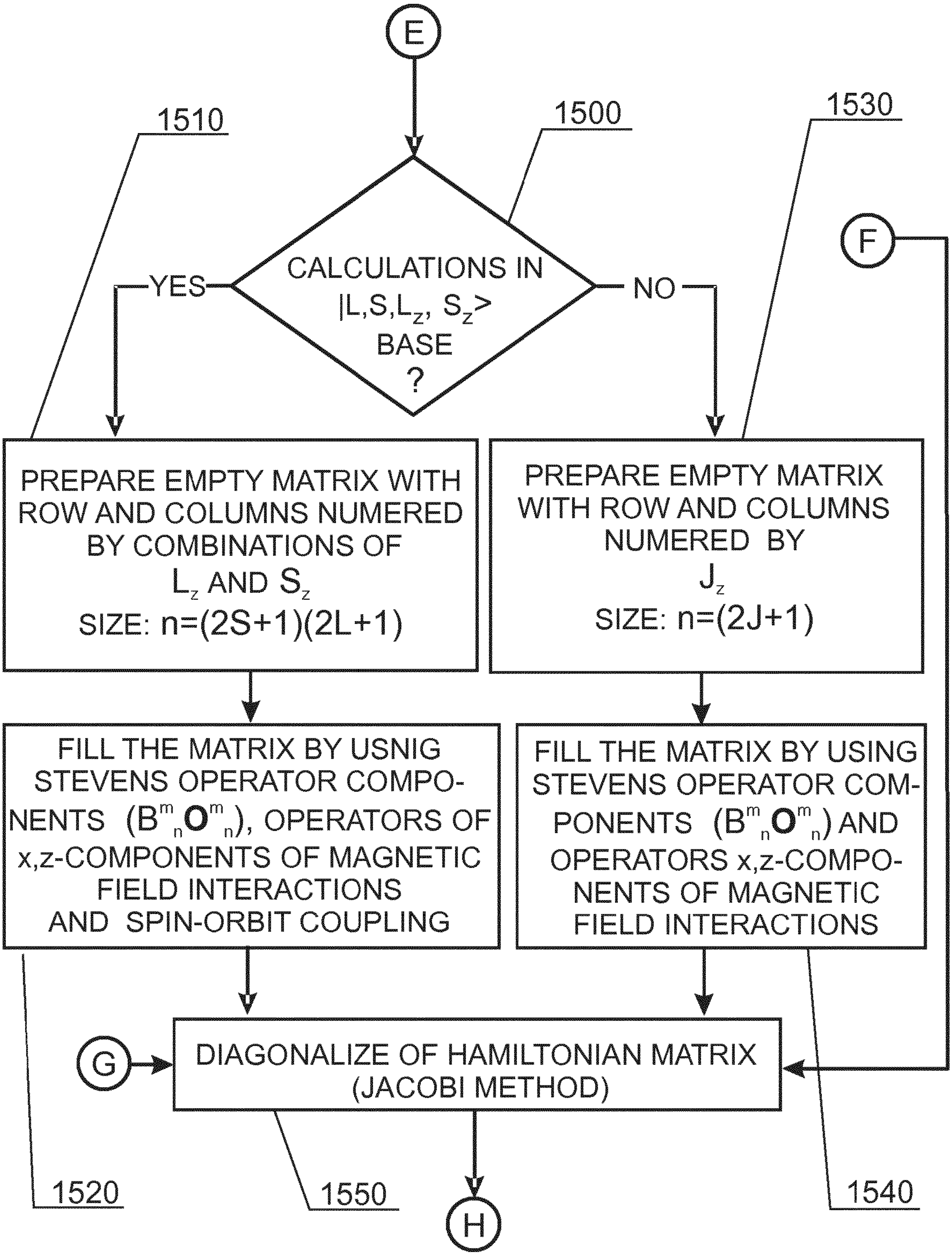
E
1510
1500
1530
CALCULATIONS IN
|L,S,Lz, Sz>
BASE
?
YES
NO
F
PREPARE EMPTY MATRIX WITH ROW AND COLUMNS NUMERED BY COMBINATIONS OF
Lz AND Sz
SIZE: n=(2S+1)(2L+1)
PREPARE EMPTY MATRIX WITH ROW AND COLUMNS NUMERED BY
Jz
SIZE: n=(2J+1)
FILL THE MATRIX BY USNIG STEVENS OPERATOR COMPONENTS (BmnOmn), OPERATORS OF x,z-COMPONENTS OF MAGNETIC FIELD INTERACTIONS AND SPIN-ORBIT COUPLING
FILL THE MATRIX BY USING STEVENS OPERATOR COMPONENTS (BmnOmn) AND OPERATORS x,z-COMPONENTS OF MAGNETIC FIELD INTERACTIONS
G
DIAGONALIZE OF HAMILTONIAN MATRIX (JACOBI METHOD)
1520
1550
1540
H

Fig. 5C

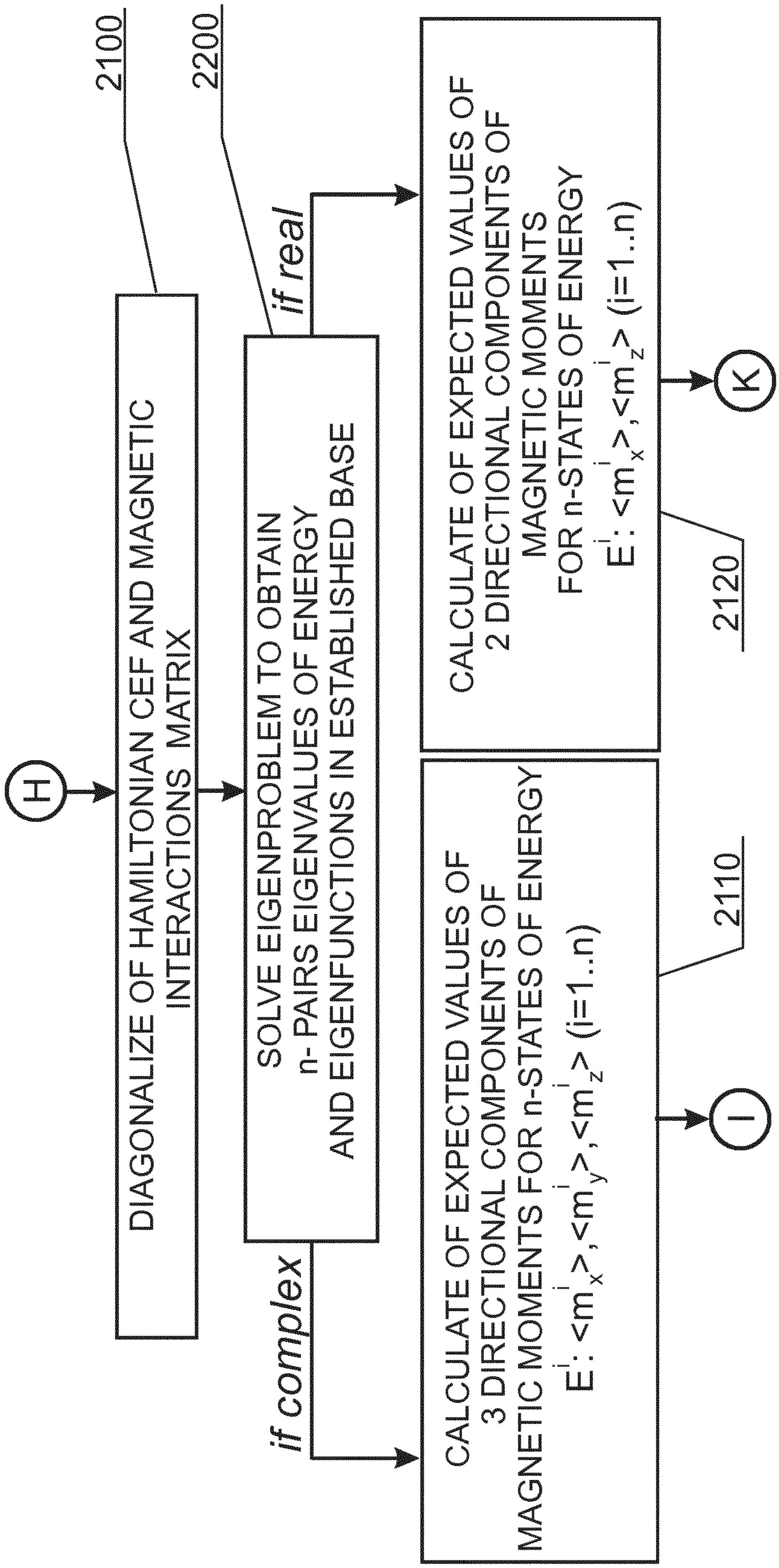
H
DIAGONALIZE OF HAMILTONIAN CEF AND MAGNETIC INTERACTIONS MATRIX
2100
SOLVE EIGENPROBLEM TO OBTAIN n- PAIRS EIGENVALUES OF ENERGY AND EIGENFUNCTIONS IN ESTABLISHED BASE
2200
if complex
if real
CALCULATE OF EXPECTED VALUES OF 3 DIRECTIONAL COMPONENTS OF MAGNETIC MOMENTS FOR n-STATES OF ENERGY
$E^i$: $<m^i_x>,<m^i_y>,<m^i_z>$ (i=1..n)
2110
CALCULATE OF EXPECTED VALUES OF 2 DIRECTIONAL COMPONENTS OF MAGNETIC MOMENTS FOR n-STATES OF ENERGY
$E^i$: $<m^i_x>,<m^i_z>$ (i=1..n)
2120
I
K

Fig. 5D

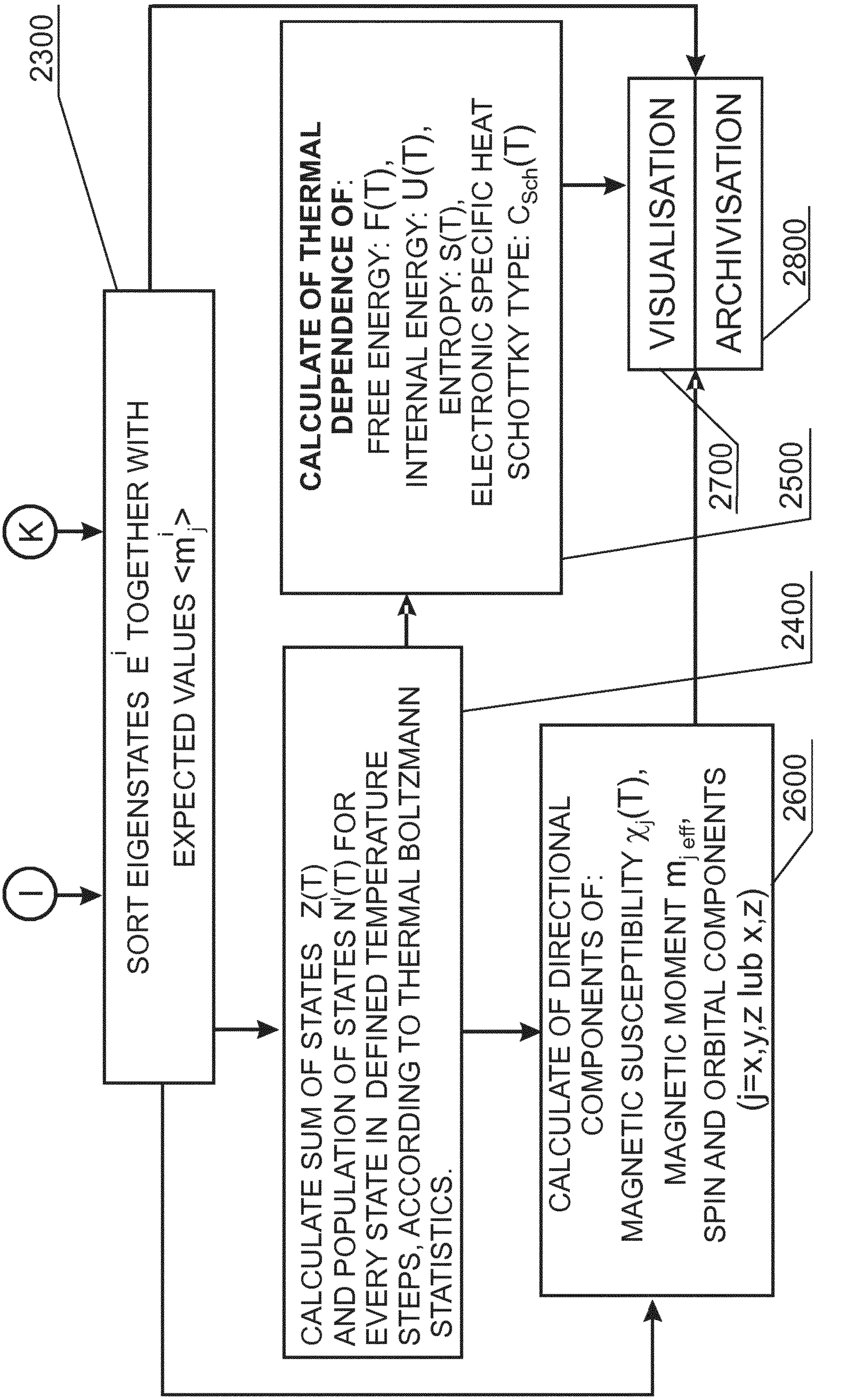
I
K
SORT EIGENSTATES $E^i$ TOGETHER WITH EXPECTED VALUES $<m^i_j>$
CALCULATE SUM OF STATES Z(T) AND POPULATION OF STATES $N^i(T)$ FOR EVERY STATE IN DEFINED TEMPERATURE STEPS, ACCORDING TO THERMAL BOLTZMANN STATISTICS.
CALCULATE OF THERMAL DEPENDENCE OF:
FREE ENERGY: F(T),
INTERNAL ENERGY: U(T),
ENTROPY: S(T),
ELECTRONIC SPECIFIC HEAT SCHOTTKY TYPE: $C_{Sch}(T)$
CALCULATE OF DIRECTIONAL COMPONENTS OF:
MAGNETIC SUSCEPTIBILITY $\chi_j(T)$,
MAGNETIC MOMENT $m_{j\,eff}$,
SPIN AND ORBITAL COMPONENTS
(j=x,y,z lub x,z)
VISUALISATION
ARCHIVISATION
2300
2400
2500
2600
2700
2800

Fig. 5E

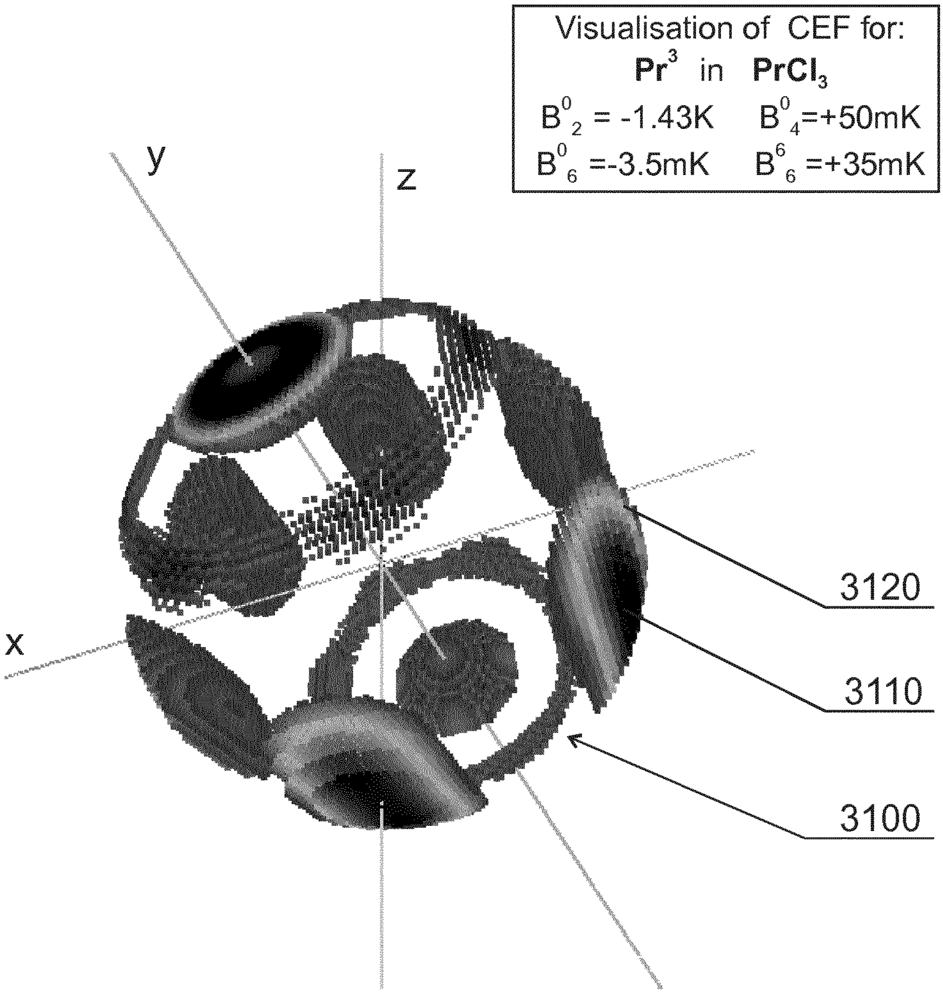
Visualisation of CEF for:
Pr3 in PrCl3
B02 = -1.43K B04=+50mK
B06 =-3.5mK B66 =+35mK
y
z
x
3120
3110
3100

Fig. 6

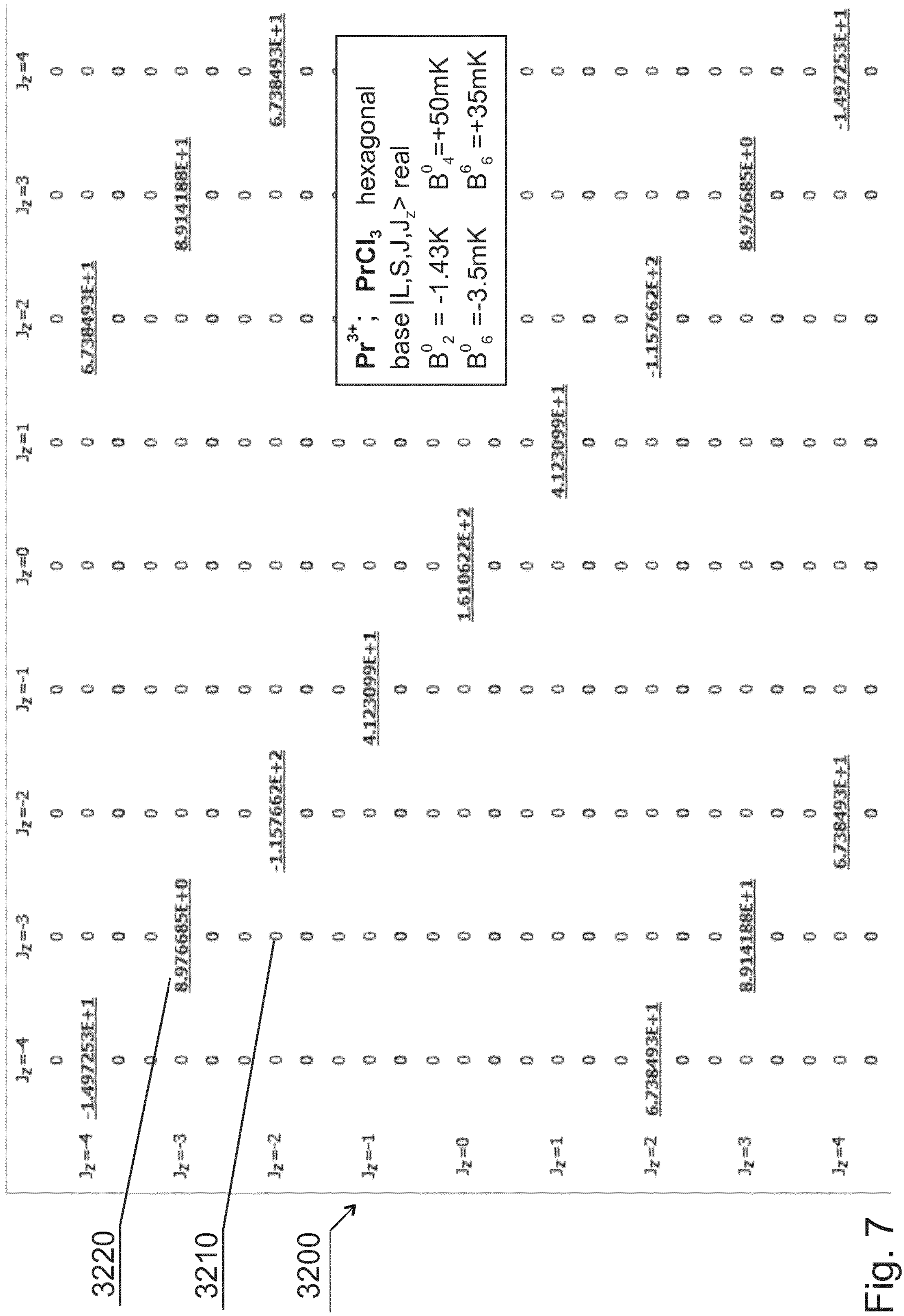
3220
3210
3200
$Pr^{3+}$; $PrCl_3$ hexagonal
base |L,S,J,Jz> real
$B^0_2$ = -1.43K $B^0_4$=+50mK
$B^0_6$ =-3.5mK $B^6_6$ =+35mK
Jz=-4 Jz=-3 Jz=-2 Jz=-1 Jz=0 Jz=1 Jz=2 Jz=3 Jz=4
Jz=-4, Jz=-4: -1.497253E+1
Jz=-4, Jz=2: 6.738493E+1
Jz=-3, Jz=-3: 8.976685E+0
Jz=-3, Jz=3: 8.914188E+1
Jz=-2, Jz=-2: -1.157662E+2
Jz=-2, Jz=4: 6.738493E+1
Jz=-1, Jz=-1: 4.123099E+1
Jz=0, Jz=0: 1.610622E+2
Jz=1, Jz=1: 4.123099E+1
Jz=2, Jz=-4: 6.738493E+1
Jz=2, Jz=2: -1.157662E+2
Jz=3, Jz=-3: 8.914188E+1
Jz=3, Jz=3: 8.976685E+0
Jz=4, Jz=-2: 6.738493E+1
Jz=4, Jz=4: -1.497253E+1

Fig. 7

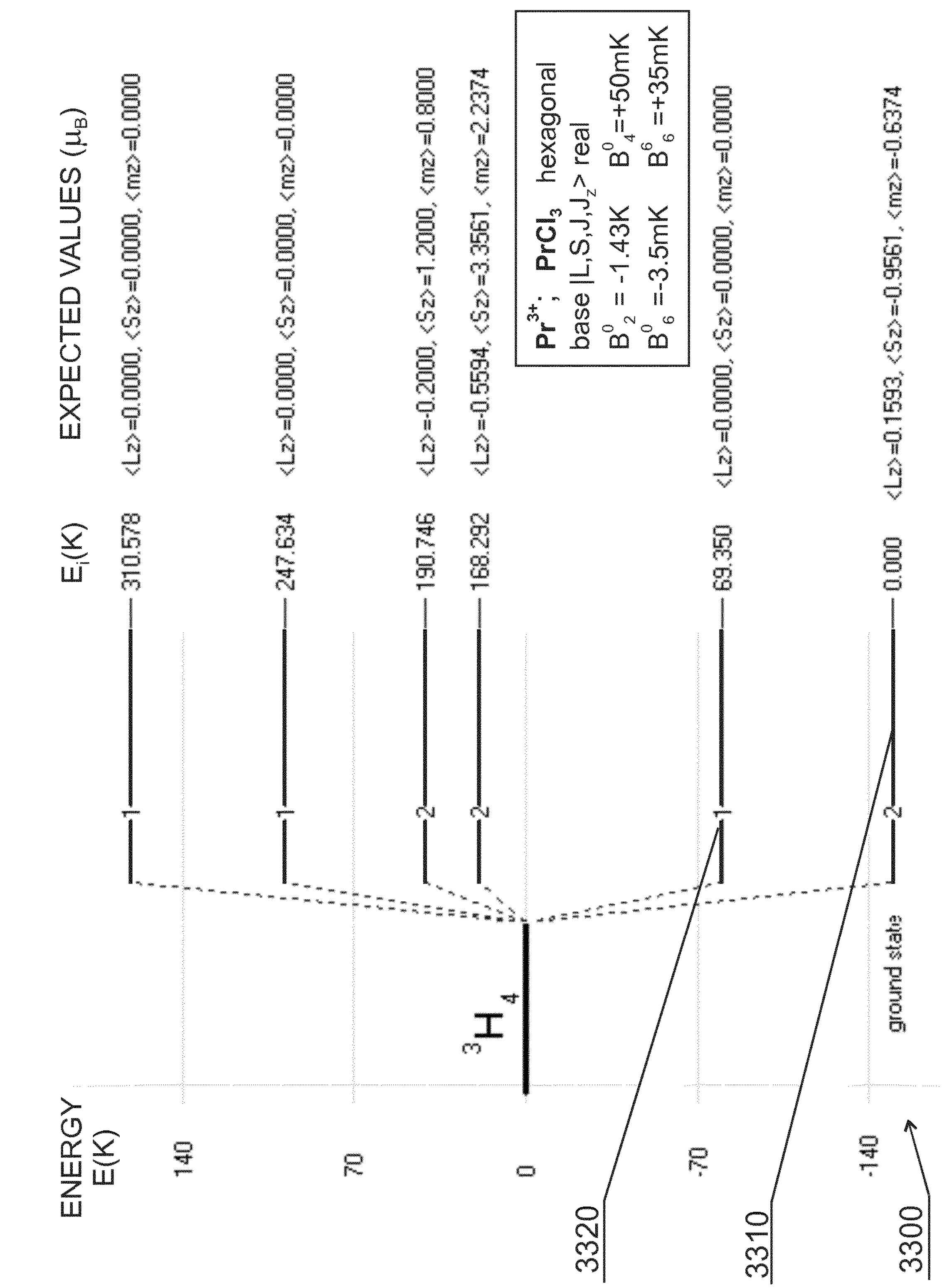
ENERGY E(K)
140
70
0
-70
-140
$^3H_4$
ground state
E_i(K)
EXPECTED VALUES ($\mu_B$)
1 — 310.578 <Lz>=0.0000, <Sz>=0.0000, <mz>=0.0000
1 — 247.634 <Lz>=0.0000, <Sz>=0.0000, <mz>=0.0000
2 — 190.746 <Lz>=-0.2000, <Sz>=-1.2000, <mz>=0.8000
2 — 168.292 <Lz>=-0.5594, <Sz>=-3.3561, <mz>=-2.2374
1 — 69.350 <Lz>=0.0000, <Sz>=0.0000, <mz>=0.0000
2 — 0.000 <Lz>=0.1593, <Sz>=-0.9561, <mz>=-0.6374
$Pr^{3+}$; $PrCl_3$ hexagonal
base $|L,S,J,J_z>$ real
$B^0_2$ = -1.43K $B^0_4$=+50mK
$B^0_6$ =-3.5mK $B^6_6$ =+35mK
3320
3310
3300

Fig. 8

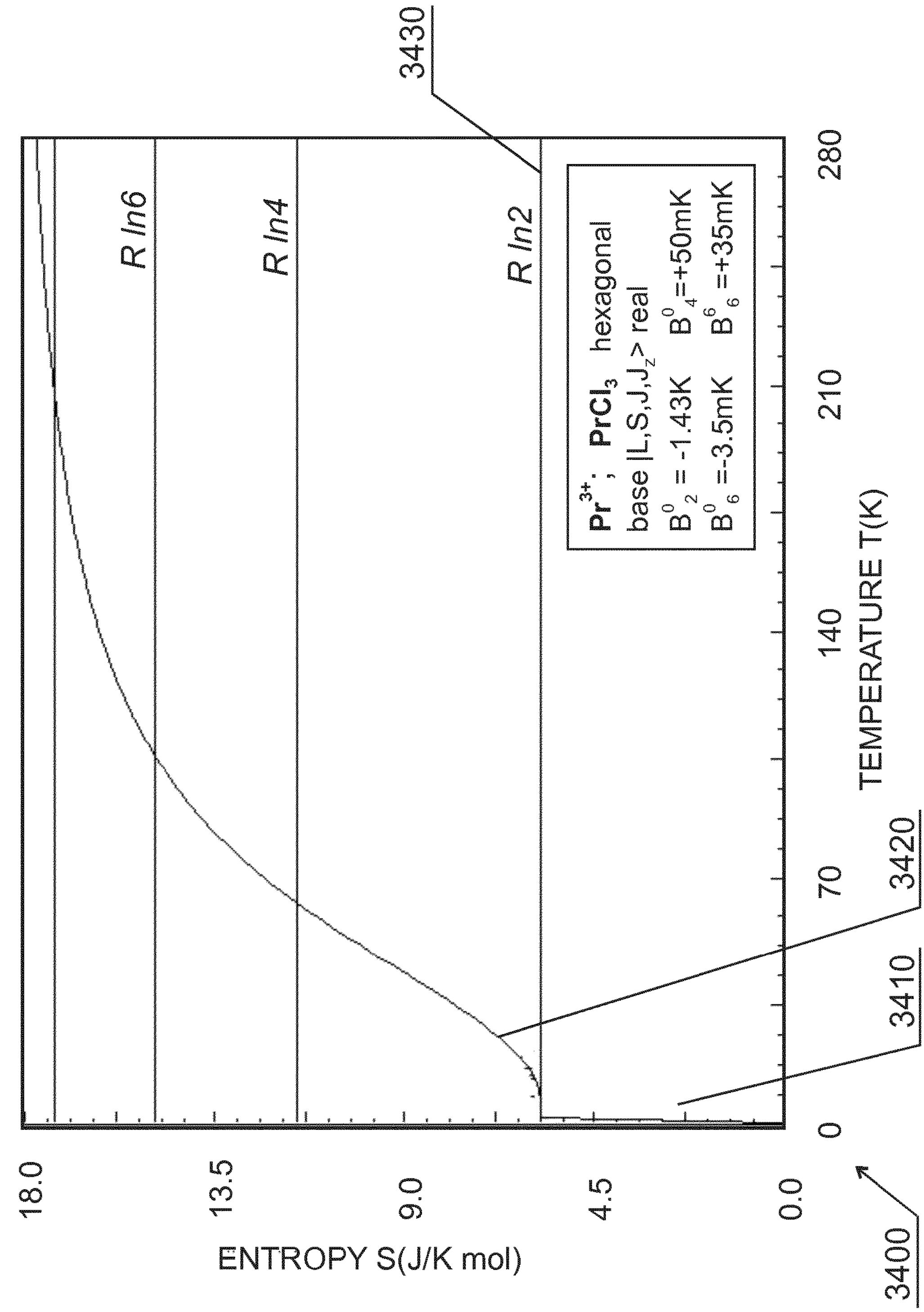
3430
R ln6
R ln4
R ln2
$Pr^{3+}$; $PrCl_3$ hexagonal
base $|L,S,J,J_z\rangle$ real
$B^0_2$ = -1.43K $B^0_4$=+50mK
$B^0_6$ =-3.5mK $B^6_6$ =+35mK
18.0
13.5
9.0
4.5
0.0
ENTROPY S(J/K mol)
0
70
140
210
280
TEMPERATURE T(K)
3400
3410
3420

Fig. 9

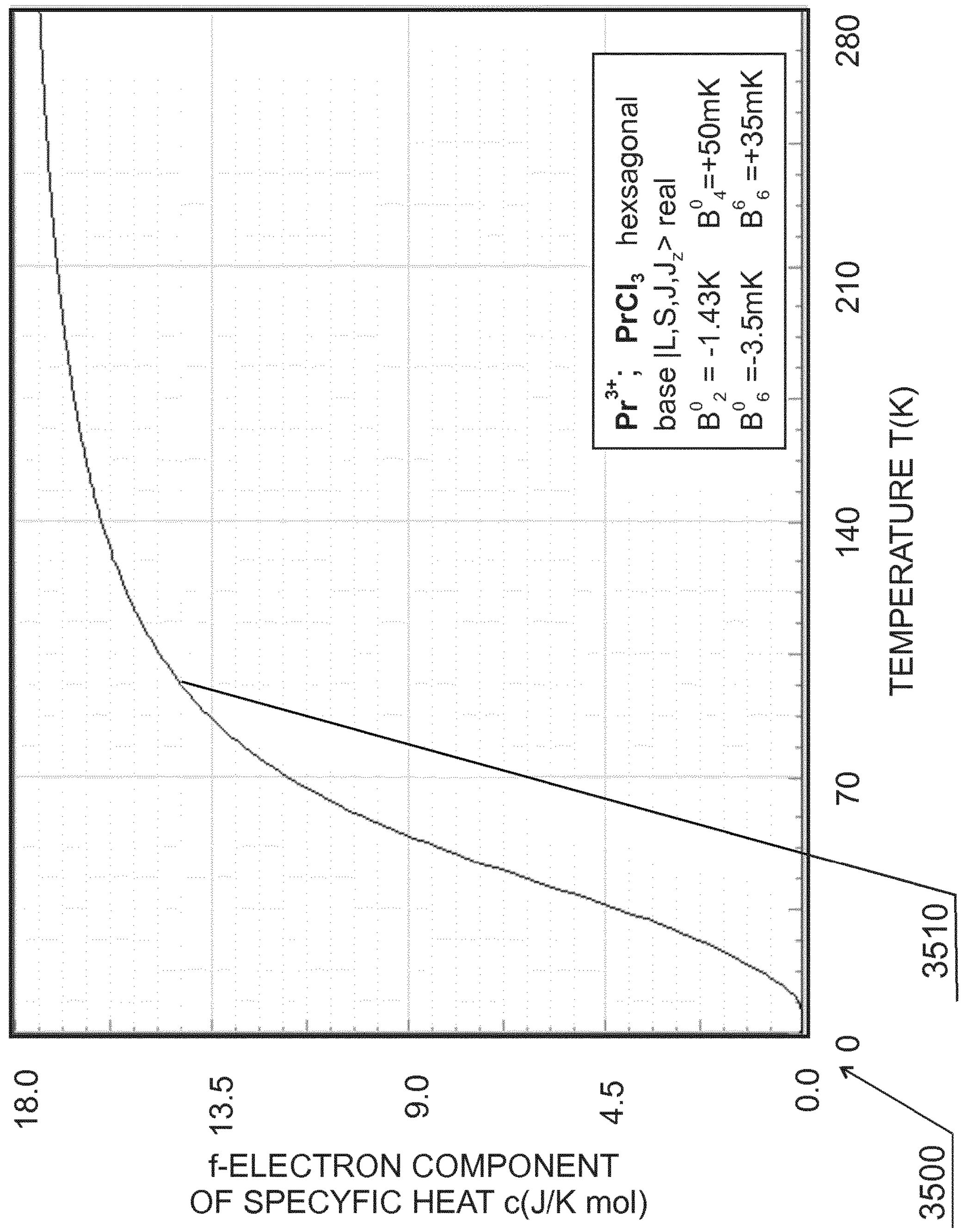
f-ELECTRON COMPONENT OF SPECYFIC HEAT c(J/K mol)
18.0
13.5
9.0
4.5
0.0
0
70
140
210
280
TEMPERATURE T(K)
Pr3+; PrCl3 hexsagonal
base |L,S,J,Jz> real
B02 = -1.43K B04=+50mK
B06 =-3.5mK B66 =+35mK
3510
3500

Fig. 10

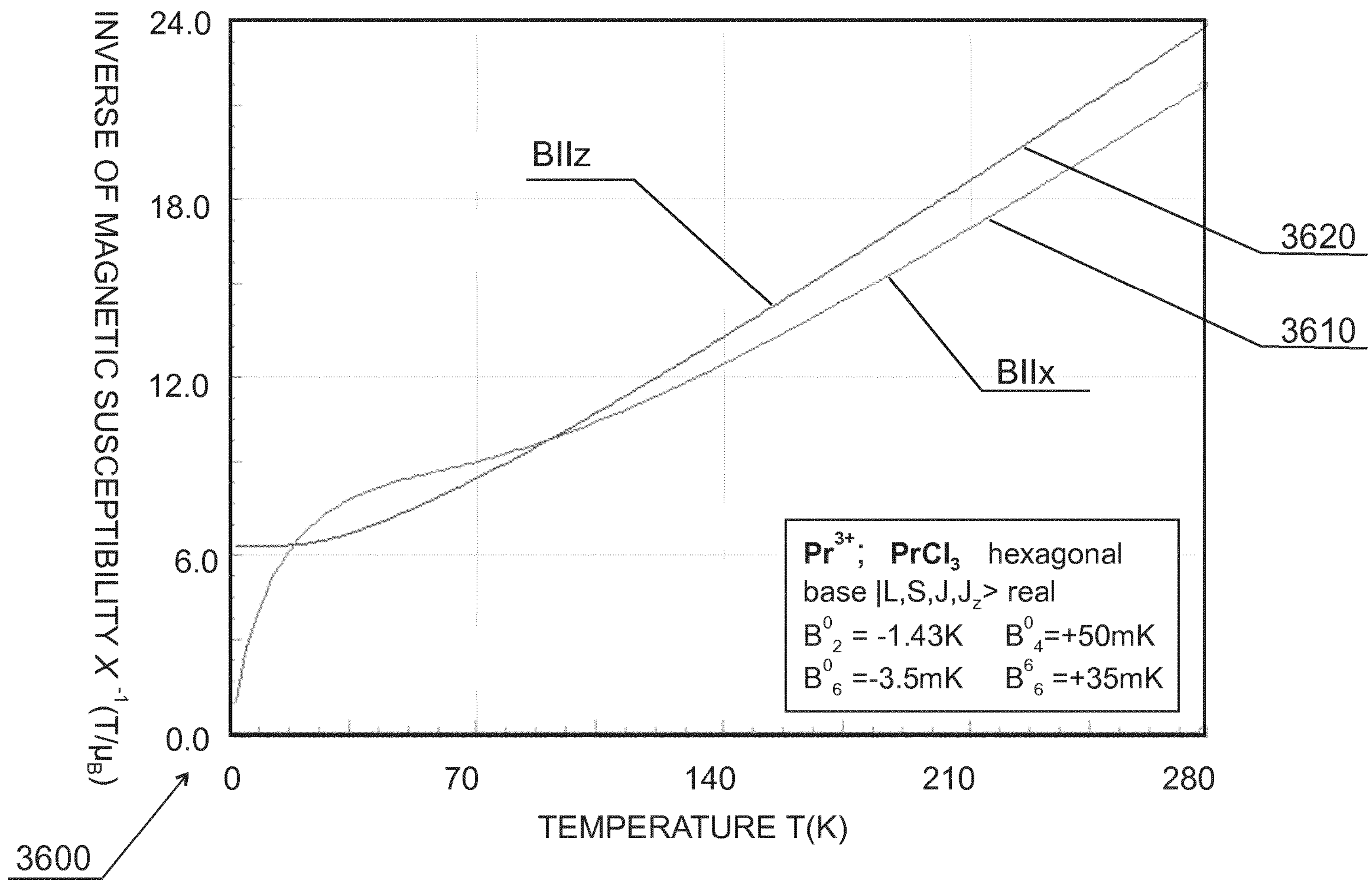
INVERSE OF MAGNETIC SUSCEPTIBILITY X -1 (T/μB)
24.0
18.0
12.0
6.0
0.0
0
70
140
210
280
TEMPERATURE T(K)
BIIz
BIIx
3620
3610
3600
Pr3+; PrCl3 hexagonal
base |L,S,J,Jz> real
B0 2 = -1.43K B0 4=+50mK
B0 6 =-3.5mK B6 6 =+35mK

Fig. 11

| | $L_Z=5 S_Z=1$ | $L_Z=5 S_Z=0$ | $L_Z=5 S_Z=-1$ | $L_Z=4 S_Z=1$ | $L_Z=4 S_Z=0$ | $L_Z=4 S_Z=-1$ | $L_Z=3 S_Z=1$ | $L_Z=3 S_Z=0$ | $L_Z=3 S_Z=-1$ | $L_Z=2 S_Z=1$ | L |
|---|---|---|---|---|---|---|---|---|---|---|---|
| $L_Z=5$ $S_Z=1$ | -2.75E+3<br>-3.997387E+1<br>4.703531E+0 | 0<br>0<br>9.5E-1-1i | 0<br>0<br>0 | 0<br>0<br>1E+0-1E+0i | -1.2298E+3<br>0<br>0 | 0<br>0<br>0 | 0<br>0<br>0 | 0<br>0<br>0 | 0<br>0<br>0 | 0<br>0<br>0 | |
| $L_Z=5$ $S_Z=0$ | 0<br>0<br>9.5E-1+1i | 0<br>-3.9974E+1<br>3.35855E+0 | 0<br>0<br>9.5E-1-1i | 0<br>0<br>0 | 0<br>0<br>1E+0-1E+0i | -1.2298E+3<br>0<br>0 | 0<br>0<br>0 | 0<br>0<br>0 | 0<br>0<br>0 | 0<br>0<br>0 | |
| $L_Z=5$ $S_Z=-1$ | 0<br>0<br>0 | 0<br>0<br>9.5E-1+1i | 2.75E+3<br>-3.997387E+1<br>2.013569E+0 | 0<br>0<br>0 | 0<br>0<br>0 | 0<br>0<br>1E+0-1E+0i | 0<br>0<br>0 | 0<br>0<br>0 | 0<br>0<br>0 | 0<br>0<br>0 | |
| $L_Z=4$ $S_Z=1$ | 0<br>0<br>1E+0+1E+0i | 0<br>0<br>0 | 0<br>0<br>0 | -2.2E+3<br>9.202388E+1<br>4.031821E+0 | 0<br>0<br>9.5E-1-1i | 0<br>0<br>0 | 0<br>0<br>1E+0-1E+0i | -1.65E+3<br>0<br>0 | 0<br>0<br>0 | 0<br>0<br>0 | |
| $L_Z=4$ $S_Z=0$ | -1.2298E+3<br>0<br>0 | 0<br>0<br>1E+0+1E+0i | 0<br>0<br>0 | 0<br>0<br>9.5E-1+1i | 0<br>9.20239E+1<br>2.68684E+0 | 0<br>0<br>9.5E-1-1i | 0<br>0<br>0 | 0<br>0<br>1E+0-1E+0i | -1.65E+3<br>0<br>0 | 0<br>0<br>0 | |
| $L_Z=4$ $S_Z=-1$ | 0<br>0<br>0 | -1.2298E+3<br>0<br>0 | 0<br>0<br>1E+0+1E+0i | 0<br>0<br>0 | 0<br>0<br>9.5E-1+1i | 2.2E+3<br>9.202388E+1<br>1.341859E+0 | 0<br>0<br>0 | 0<br>0<br>0 | 0<br>0<br>1E+0-1E+0i | 0<br>0<br>0 | |
| $L_Z=3$ $S_Z=1$ | 0<br>0<br>0 | 0<br>0<br>0 | 0<br>0<br>0 | 0<br>0<br>1E+0+1E+0i | 0<br>0<br>0 | 0<br>0<br>0 | -1.65E+3<br>-1.589052E+2<br>3.360111E+0 | 0<br>0<br>9.5E-1-1i | 0<br>0<br>0 | 0<br>0<br>2E+0-2E+0i | -1 |
| $L_Z=3$ $S_Z=0$ | 0 | 0 | 0 | -1.65E+3<br>0<br>0 | 0<br>0<br>1E+0+1E+0i | 0<br>0<br>0 | 0<br>0<br>9.5E-1+1i | 0<br>-1.5891E+2<br>2.01513E+0 | 0<br>0<br>9.5E-1-1i | 0<br>0<br>0 | 2E |
| $L_Z=3$ $S_Z=-1$ | | | | 0<br>0<br>0 | -1.65E+3<br>0<br>0 | 0<br>0<br>1E+0+1E+0i | 0<br>0<br>0 | 0<br>0<br>9.5E-1+1i | 1.65E+3<br>-1.589052E+2<br>6.701489E-1 | 0<br>0<br>0 | |

**$Pr^{3+}$; $PrCl_3$** hexagonal
base $|L,S,L_z,S_z>$, $\lambda_{s\text{-}o}$=-650K
$B^0_2$ = -1.43K, $B^0_4$=+50mK
$B^0_6$ =-3.5mK $B^6_6$ =+35mK

3700

Fig. 12

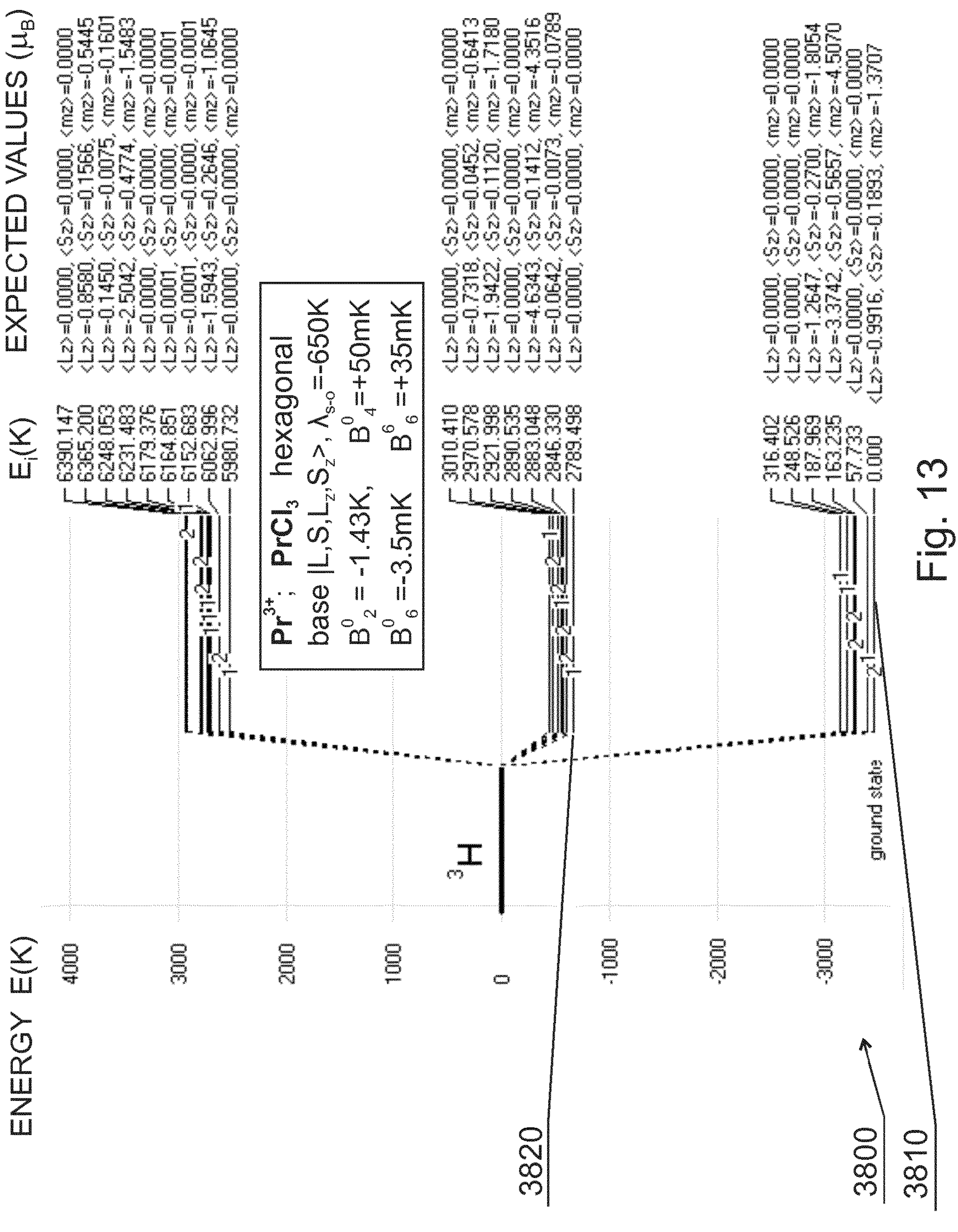
ENERGY E(K)
4000
3000
2000
1000
0
-1000
-2000
-3000
$^3H$
ground state
$E_i$(K)
EXPECTED VALUES ($\mu_B$)
6390.147 <Lz>=-0.0000, <Sz>=-0.0000, <mz>=0.0000
6365.200 <Lz>=-0.8580, <Sz>=-0.1566, <mz>=-0.5445
6248.053 <Lz>=-0.1450, <Sz>=-0.0075, <mz>=-0.1601
6231.483 <Lz>=-2.5042, <Sz>=-0.4774, <mz>=-1.5483
6179.376 <Lz>=-0.0000, <Sz>=-0.0000, <mz>=-0.0000
6164.851 <Lz>=0.0001, <Sz>=-0.0000, <mz>=0.0001
6152.683 <Lz>=-0.0001, <Sz>=-0.0000, <mz>=-0.0001
6062.996 <Lz>=-1.5943, <Sz>=-0.2646, <mz>=-1.0645
5980.732 <Lz>=-0.0000, <Sz>=-0.0000, <mz>=-0.0000
3010.410 <Lz>=-0.0000, <Sz>=-0.0000, <mz>=-0.0000
2970.578 <Lz>=-0.7318, <Sz>=-0.0452, <mz>=-0.6413
2921.998 <Lz>=-1.9422, <Sz>=-0.1120, <mz>=-1.7180
2890.535 <Lz>=-0.0000, <Sz>=-0.0000, <mz>=-0.0000
2883.048 <Lz>=-4.6343, <Sz>=-0.1412, <mz>=-4.3516
2846.330 <Lz>=-0.0642, <Sz>=-0.0073, <mz>=-0.0789
2789.498 <Lz>=-0.0000, <Sz>=-0.0000, <mz>=-0.0000
316.402 <Lz>=-0.0000, <Sz>=-0.0000, <mz>=-0.0000
248.526 <Lz>=-0.0000, <Sz>=-0.0000, <mz>=-0.0000
187.969 <Lz>=-1.2647, <Sz>=-0.2700, <mz>=-1.8054
163.235 <Lz>=-3.3742, <Sz>=-0.5657, <mz>=-4.5070
57.733 <Lz>=-0.0000, <Sz>=-0.0000, <mz>=-0.0000
0.000 <Lz>=-0.9916, <Sz>=-0.1893, <mz>=-1.3707
$Pr^{3+}$; $PrCl_3$ hexagonal
base $|L,S,L_z,S_z>$, $\lambda_{s-o}$=-650K
$B^0_2$ = -1.43K, $B^0_4$=+50mK
$B^0_6$ =-3.5mK $B^6_6$ =+35mK
3820
3800
3810

Fig. 13

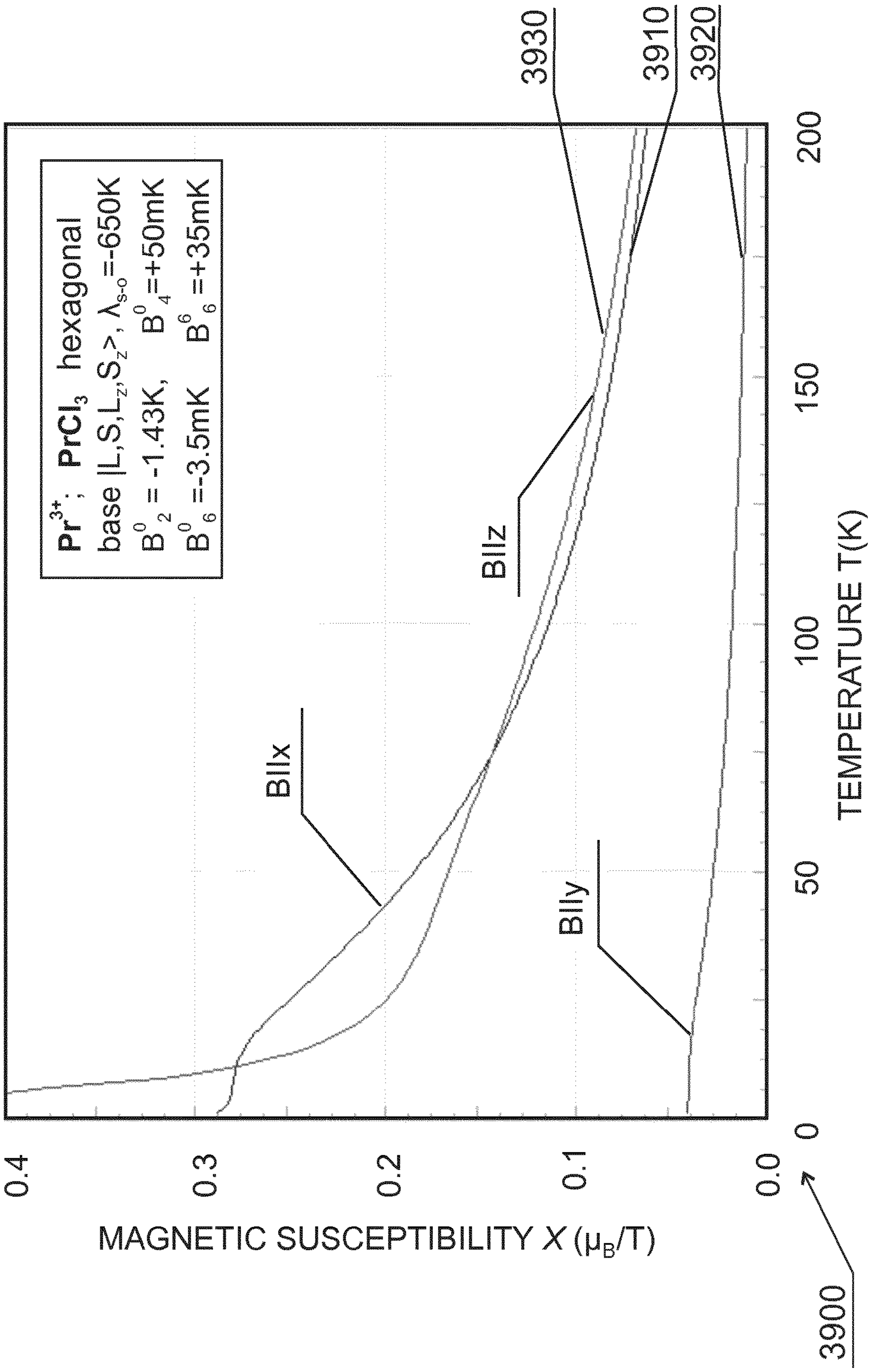
$Pr^{3+}$; $PrCl_3$ hexagonal
base $|L,S,L_z,S_z>$, $\lambda_{s-o}$=-650K
$B^0_2$ = -1.43K, $B^0_4$=+50mK
$B^0_6$ =-3.5mK $B^6_6$ =+35mK
BIIx
BIIz
BIIy
3930
3910
3920
3900
0.4
0.3
0.2
0.1
0.0
MAGNETIC SUSCEPTIBILITY $X$ ($\mu_B$/T)
0
50
100
150
200
TEMPERATURE T(K)

Fig. 14

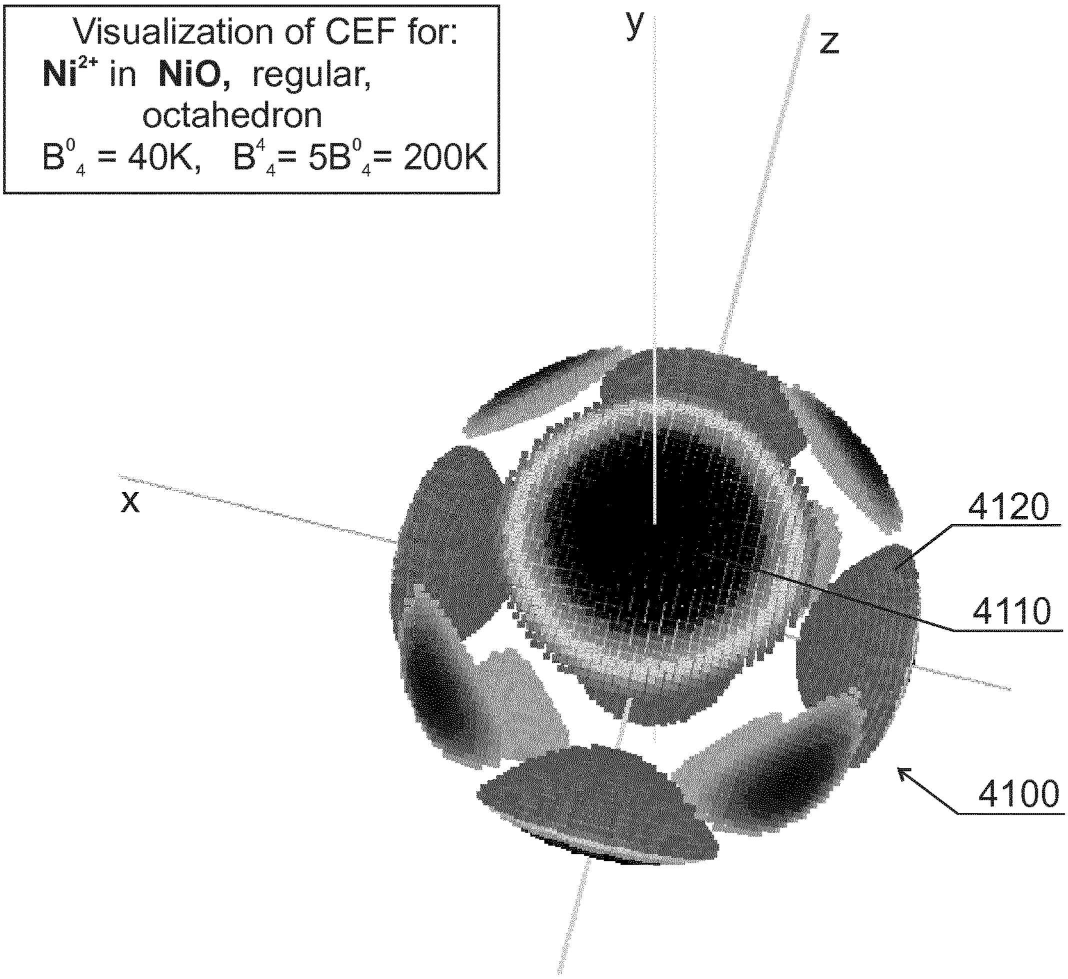
Visualization of CEF for:
Ni2+ in NiO, regular, octahedron
B04 = 40K, B44= 5B04= 200K
y
z
x
4120
4110
4100

Fig. 15

4200

| | $L_z$=3 $S_z$=1 | $L_z$=3 $S_z$=0 | $L_z$=3 $S_z$=-1 | $L_z$=2 $S_z$=1 | $L_z$=2 $S_z$=0 | $L_z$=2 $S_z$=-1 | $L_z$=1 $S_z$=1 | $L_z$=1 $S_z$=0 | $L_z$=1 $S_z$=-1 | $L_z$=0 $S_z$=1 | $L_z$=0 $S_z$=0 | $L_z$=0 $S_z$=-1 |
|---|---|---|---|---|---|---|---|---|---|---|---|---|
| $L_z$=3 $S_z$=1 | -1.44E+3<br>7.2E+3<br>3.36011E+0 | 0<br>0<br>9.5105E-3 | 0<br>0<br>0 | 0<br>0<br>8.2267E-3 | -8.314E+2<br>0<br>0 | 0<br>0<br>0 | 0<br>0<br>0 | 0<br>0<br>0 | 0<br>0 | 0<br>0 | 0<br>0 | 0<br>0 |
| $L_z$=3 $S_z$=0 | 0<br>0<br>9.5105E-3 | 0<br>7.2E+3<br>2.01513E+0 | 0<br>0<br>9.5105E-3 | 0<br>0<br>0 | 0<br>0<br>8.2267E-3 | -8.314E+2<br>0<br>0 | 0<br>0<br>0 | 0<br>0<br>0 | | | | |
| $L_z$=3 $S_z$=-1 | 0<br>0<br>0 | 0<br>0<br>9.5105E-3 | 1.44E+3<br>7.2E+3<br>6.70149E-1 | 0<br>0<br>0 | 0<br>0<br>0 | 0<br>0<br>8.2267E-3 | 0<br>0<br>0 | 0<br>0<br>0 | 0<br>0 | 0<br>0 | 0<br>0 | 0<br>0 |
| $L_z$=2 $S_z$=1 | 0<br>0<br>8.2267E-3 | 0<br>0<br>0 | 0<br>0<br>0 | -9.6E+2<br>-1.68E+4<br>2.6884E+0 | 0<br>0<br>9.5105E-3 | 0<br>0<br>0 | 0<br>0<br>1.0621E-2 | -1.073E+3<br>0<br>0 | 0<br>0<br>0 | 0<br>0<br>0 | 0<br>0<br>0 | 0<br>0<br>0 |
| $L_z$=2 $S_z$=0 | -8.314E+2<br>0<br>0 | 0<br>0<br>8.2267E-3 | 0<br>0<br>0 | 0<br>0<br>9.5105E-3 | 0<br>-1.68E+4<br>1.34342E+0 | 0<br>0<br>9.5105E-3 | 0<br>0<br>0 | 0<br>0<br>1.0621E-2 | -1.073E+3<br>0<br>0 | 0<br>0<br>0 | 0<br>0<br>0 | 0<br>0<br>0 |
| $L_z$=2 $S_z$=-1 | 0<br>0<br>0 | -8.314E+2<br>0<br>0 | 0<br>0<br>8.2267E-3 | 0<br>0<br>0 | 0<br>0<br>9.5105E-3 | 9.6E+2<br>-1.68E+4<br>-1.56105E-3 | 0<br>0<br>0 | 0<br>0<br>0 | 0<br>0<br>1.0621E-2 | 0<br>0<br>0 | 0<br>0<br>0 | 0<br>0<br>0 |
| $L_z$=1 $S_z$=1 | 0<br>0<br>0 | 0<br>0<br>0 | 0<br>0<br>0 | 0<br>0<br>1.0621E-2 | 0<br>0<br>0 | 0<br>0<br>0 | -4.8E+2<br>2.4E+3<br>2.01669E+0 | 0<br>0<br>9.5105E-3 | 0<br>0<br>0 | 0<br>0<br>1.1634E-2 | -1.176E+3<br>0<br>0 | 0<br>0<br>0 |
| $L_z$=1 $S_z$=0 | 0<br>0<br>0 | 0<br>0<br>0 | 0<br>0<br>0 | -1.073E+3<br>0<br>0 | 0<br>0<br>1.0621E-2 | 0<br>0<br>0 | 0<br>0<br>9.5105E-3 | 0<br>2.4E+3<br>6.7171E-1 | 0<br>0<br>9.5105E-3 | 0<br>0<br>0 | 0<br>0<br>1.1634E-2 | -1.176E+3<br>0<br>0 |
| $L_z$=1 $S_z$=-1 | 0<br>0<br>0 | 0<br>0<br>0 | 0<br>0<br>0 | 0<br>0<br>0 | -1.073E+3<br>0<br>0 | 0<br>0<br>1.0621E-2 | 0<br>0<br>0 | 0<br>0<br>9.5105E-3 | 4.8E+2<br>2.4E+3<br>-6.73271E-1 | 0<br>0<br>0 | 0<br>0<br>0 | 0<br>0<br>1.1634E-2 |

**$Ni^{2+}$; NiO,** regular, octahedron
base |L,S,$L_z$,$S_z$> real
$\lambda_{s-o}$=-480K
$B^0_4$ = 40K, $B^4_4$= $5B^0_4$= 200K

Fig. 16

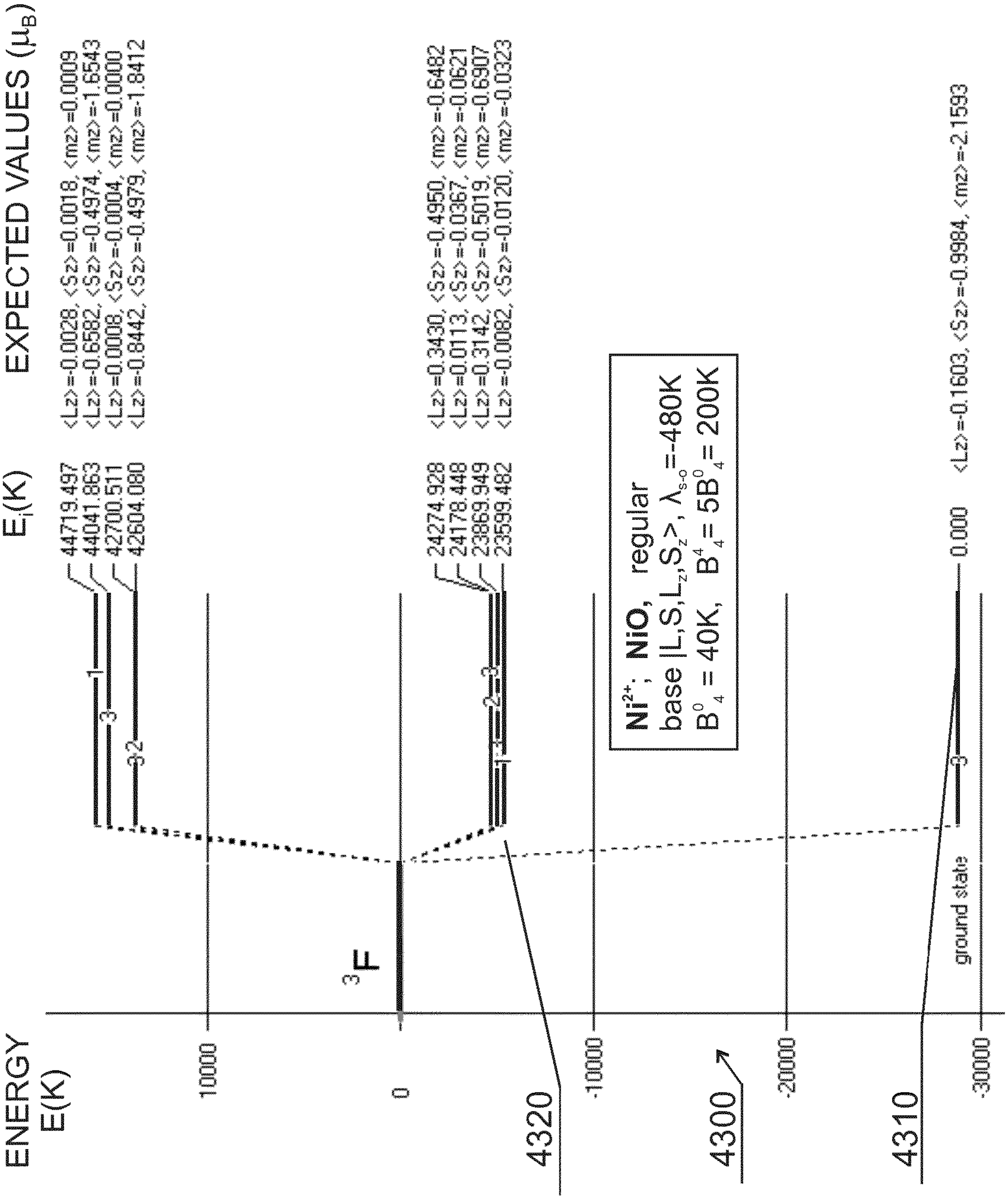
ENERGY
E(K)
10000
0
-10000
-20000
-30000
4320
4300
4310
$^3F$
ground state
$E_i$(K)
EXPECTED VALUES ($\mu_B$)
44719.497
44041.863
42700.511
42604.080
<Lz>=-0.0028, <Sz>=0.0018, <mz>=0.0009
<Lz>=-0.6582, <Sz>=-0.4974, <mz>=-1.6543
<Lz>=0.0008, <Sz>=-0.0004, <mz>=0.0000
<Lz>=-0.8442, <Sz>=-0.4979, <mz>=-1.8412
24274.928
24178.448
23869.949
23599.482
<Lz>=0.3430, <Sz>=-0.4950, <mz>=-0.6482
<Lz>=0.0113, <Sz>=-0.0367, <mz>=-0.0621
<Lz>=0.3142, <Sz>=-0.5019, <mz>=-0.6907
<Lz>=-0.0082, <Sz>=-0.0120, <mz>=-0.0323
0.000
<Lz>=-0.1603, <Sz>=-0.9984, <mz>=-2.1593
$Ni^{2+}$; NiO, regular
base $|L,S,L_z,S_z>$, $\lambda_{s-o}$=-480K
$B^0_4$ = 40K, $B^4_4$= $5B^0_4$= 200K

Fig. 17

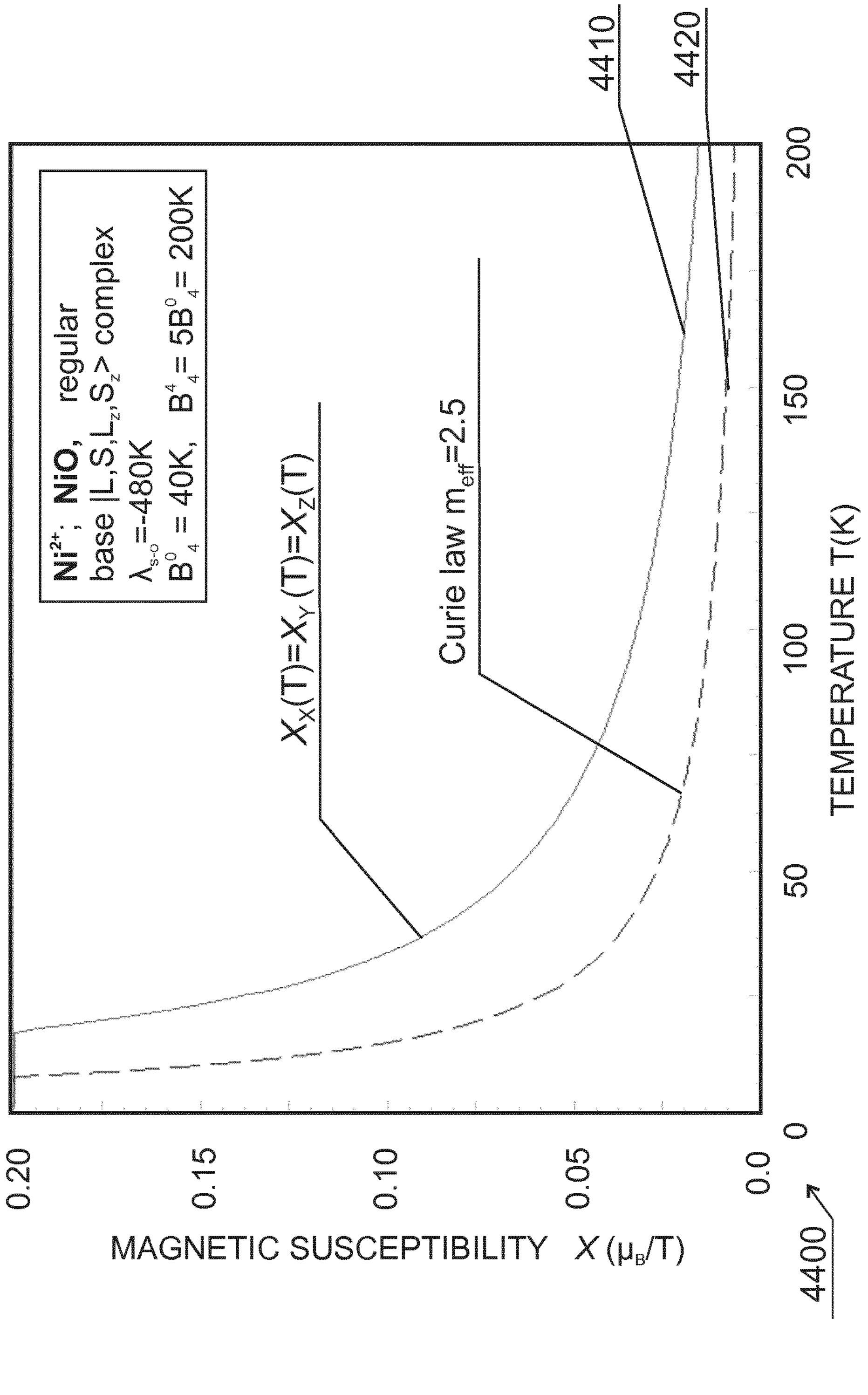
Ni2+; NiO, regular
base |L,S,Lz,Sz> complex
λs-o=-480K
B04 = 40K, B44 = 5B04 = 200K
XX(T)=XY(T)=XZ(T)
Curie law meff=2.5
4410
4420
4400
MAGNETIC SUSCEPTIBILITY X (μB/T)
0.20
0.15
0.10
0.05
0.0
0
50
100
150
200
TEMPERATURE T(K)

Fig. 18

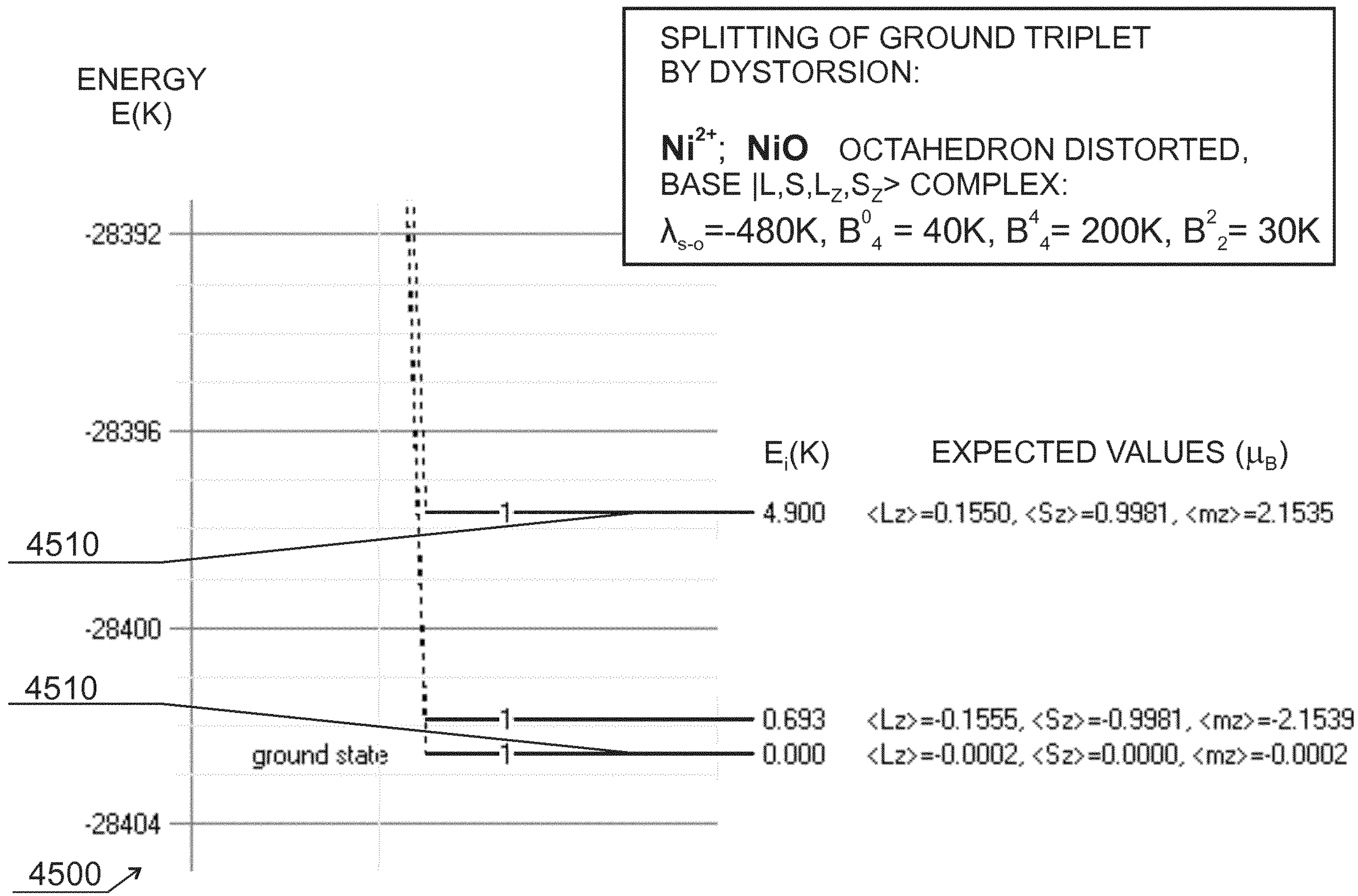
SPLITTING OF GROUND TRIPLET
BY DYSTORSION:
Ni2+; NiO OCTAHEDRON DISTORTED,
BASE |L,S,LZ,SZ> COMPLEX:
λs-o=-480K, B04 = 40K, B44= 200K, B22= 30K
ENERGY
E(K)
-28392
-28396
-28400
-28404
4510
4510
4500
ground state
1
1
1
Ei(K)
EXPECTED VALUES (μB)
4.900
0.693
0.000
<Lz>=0.1550, <Sz>=0.9981, <mz>=2.1535
<Lz>=-0.1555, <Sz>=-0.9981, <mz>=-2.1539
<Lz>=-0.0002, <Sz>=0.0000, <mz>=-0.0002

Fig. 19

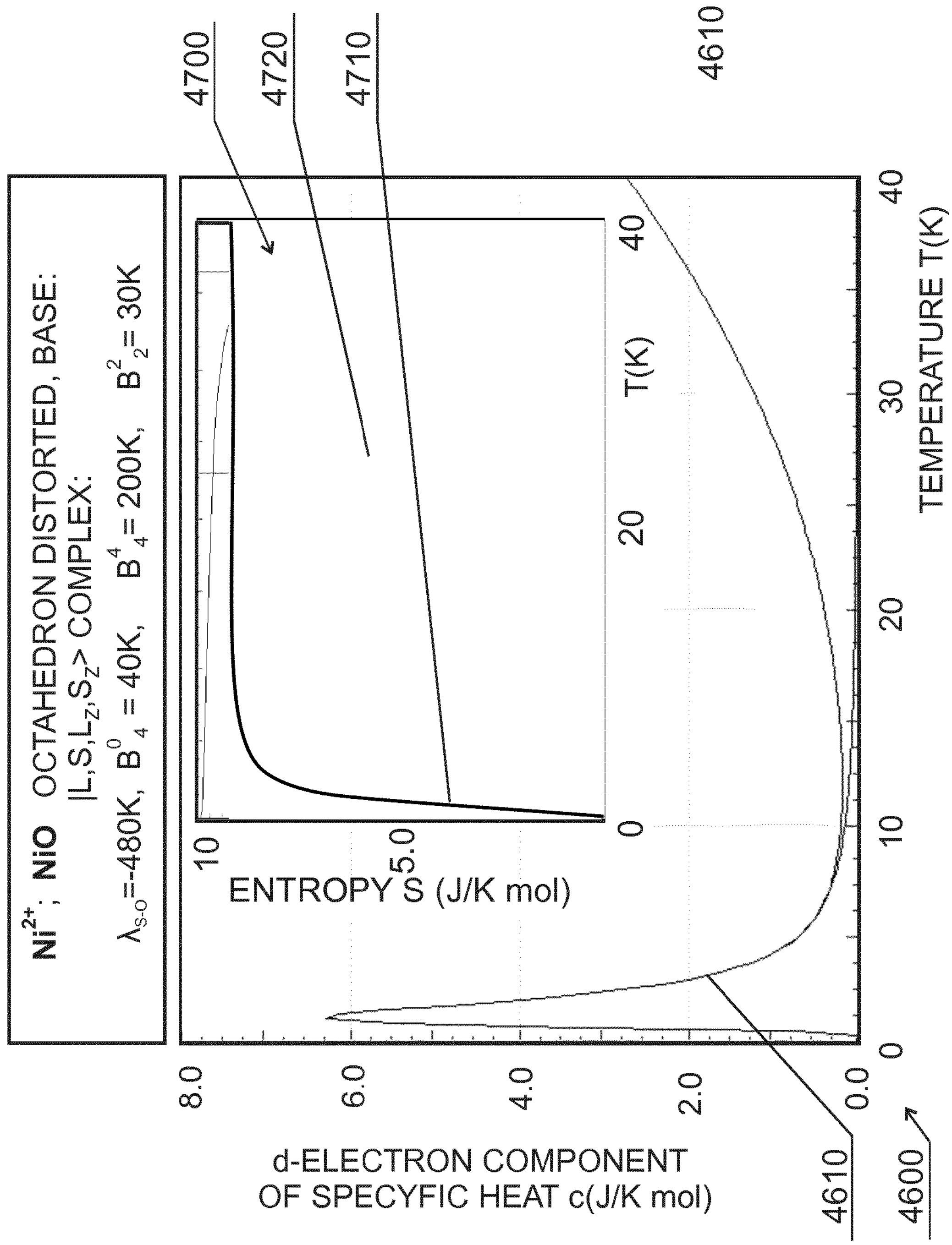
Ni2+; NiO OCTAHEDRON DISTORTED, BASE:
|L,S,LZ,SZ> COMPLEX:
λS-O=-480K, B04 = 40K, B44= 200K, B22= 30K
ENTROPY S (J/K mol)
10
5.0
0
20
40
T(K)
d-ELECTRON COMPONENT
OF SPECYFIC HEAT c(J/K mol)
8.0
6.0
4.0
2.0
0.0
0
10
20
30
40
TEMPERATURE T(K)
4700
4720
4710
4610
4610
4600

Fig. 20

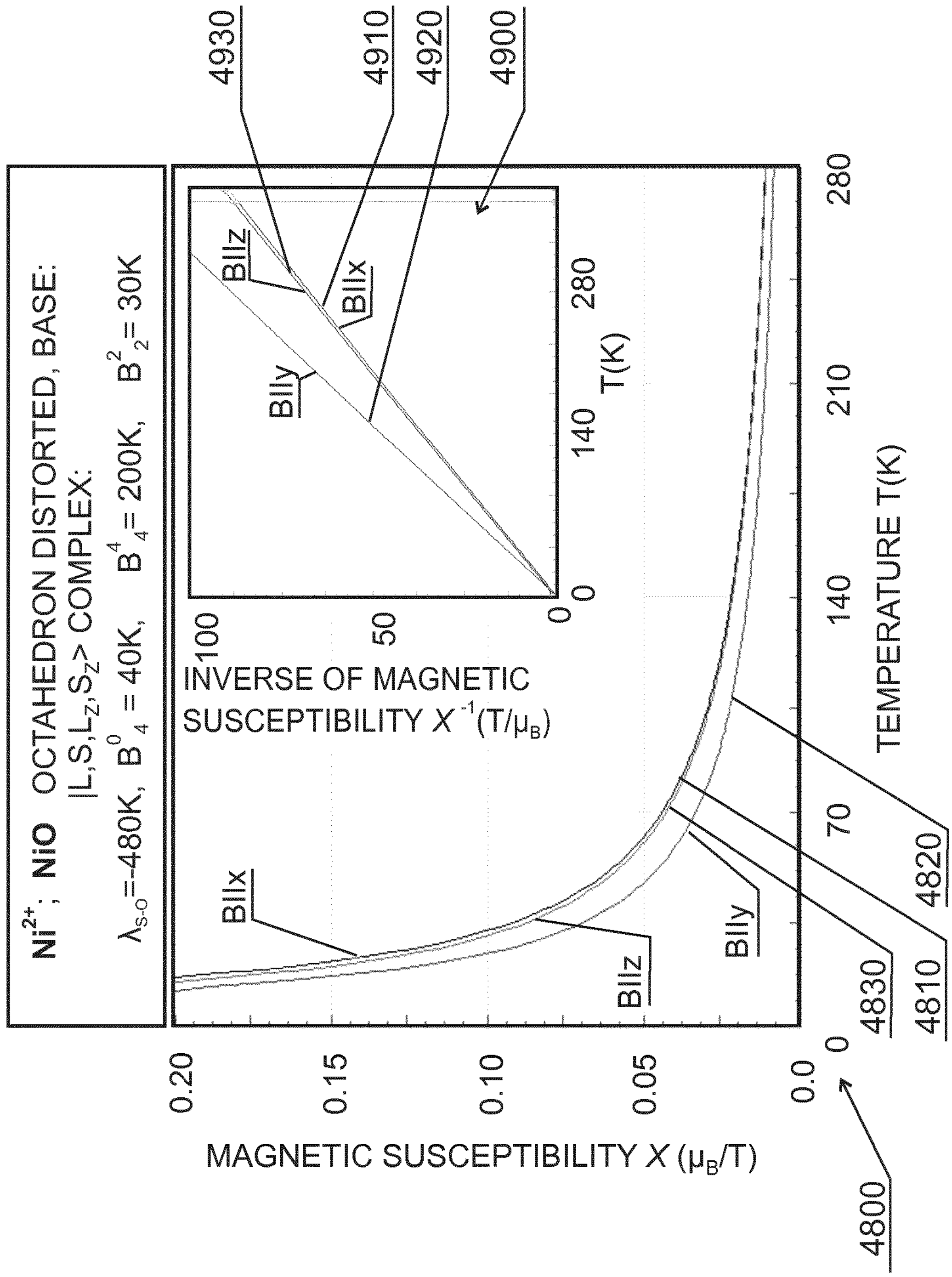
Ni2+; NiO OCTAHEDRON DISTORTED, BASE:
|L,S,LZ,SZ> COMPLEX:
λS-O=-480K, B04 = 40K, B44= 200K, B22= 30K
INVERSE OF MAGNETIC SUSCEPTIBILITY X-1(T/μB)
100
50
0
0
140
280
T(K)
BIIz
BIIx
BIIy
4930
4910
4920
4900
MAGNETIC SUSCEPTIBILITY X (μB/T)
0.20
0.15
0.10
0.05
0.0
0
70
140
210
280
TEMPERATURE T(K)
BIIx
BIIz
BIIy
4830
4810
4820
4800

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CA 2863843 A1
- WO 2014134655 A1
- WO 2014150916 A1


### Non-patent literature cited in the description


- **P.J. VON RANKE et al.** Influence of the crystalline electrical field on the magnetocaloric effect of DyAl2, ErAl2 and DyNi. *PHYSICAL REVIEW B,* 1998, vol. 58 (18), 12110-12116
- **C. ABADIA et al.** tudy of the crystal electric field interaction in RFe11Ti single crystals. *J.PHYS.: CONDENS. MATTER,* 1998, vol. 10 (2), 349-361
- **Y.J. WANG et al.** Perpendicular anisotropy in magnetic multilayer films. *Journal of Magnetism and Magnetic Materials,* 1992, vol. 115 (1), 9-19
- **G.G. HALL et al.** Point charge models for molecular properties. *CHEMICAL PHYSICS LETTERS,* 1973, vol. 20 (6), 501-503